# EUROPÄISCHE PATENTSCHRIFT

(11) **EP 3 341 363 B1**
(45) Veröffentlichungstag und Bekanntmachung des Hinweises auf die Patenterteilung: **24.07.2019**
(21) Anmeldenummer: 16753934.5
(22) Anmeldetag: 22.08.2016
(51) Int. Cl.: C07D 249/14, C07D 257/06, C07D 271/04, C07D 271/06, A01N 43/653, A01N 43/713, A01N 43/82

(54) **SUBSTITUIERTE KETOXIM-BENZOYLAMIDE**
SUBSTITUTED KETOXIME BENZOYLAMIDE
BENZAMIDE DE CETOXIME SUBSTITUE

(30) Priorität: 25.08.2015 EP 15182355
(43) Veröffentlichungstag der Anmeldung: 04.07.2018
(73) Patentinhaber: Bayer CropScience Aktiengesellschaft, 40789 Monheim am Rhein (DE)
(72) Erfinder: WALDRAFF, Christian, 61118 Bad Vilbel (DE); BRAUN, Ralf, 76857 Ramberg (DE); DIETRICH, Hansjörg, 65835 Liederbach (DE); GATZWEILER, Elmar, 61231 Bad Nauheim (DE); ROSINGER, Christopher Hugh, 65719 Hofheim (DE)
(74) Vertreter: BIP Patents
(86) Internationale Anmeldenummer: PCT/EP2016/069769
(87) Internationale Veröffentlichungsnummer: WO 2017/032728

(56) Entgegenhaltungen:
- EP-A1- 2 907 807
- WO-A1-2013/064459
- DE-A1- 19 700 096

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO 2013/064459 A1 sind Aldoxim-benzoylamide bekannt. WO 2015/052152 A1 und WO 2015/052153 A1 offenbaren Ketoxim-benzoylamide, die in 6-Position des Phenylrings jeweils substituiert sind.

Aufgabe der vorliegenden Erfindung war die Bereitstellung von weiteren herbizid wirksamen Verbindungen.

Es wurde nun gefunden, dass bestimmte substituierte Ketoxim-benzoylamide als Herbizide besonders gut geeignet sind.

Ein Gegenstand der vorliegenden Erfindung sind somit substituierte Ketoxim-benzoylamide der Formel (I) 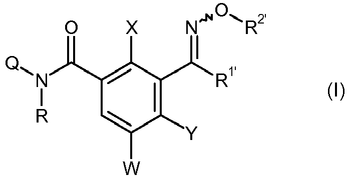 worin die Symbole und Indizes folgende Bedeutungen haben:
Q bedeutet einen Rest Q1, Q2, Q3 oder Q4, 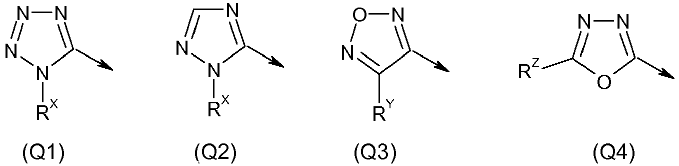
R bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, R¹(O)CO-(C₁-C₆)-Alkyl, R¹O(O)C-O-(C₁-C₆)-Alkyloxy, R²(O)²SO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C, R¹O(O)C, (R¹)₂N(O)C, R¹O, (R¹)₂N, R¹O(O)C(R¹)N, (R¹)₂N(O)C(R¹)N, R²(O)₂S oder jeweils durch s Reste aus der Gruppe bestehend aus Methyl, Ethyl, Methoxy, Nitro, Trifluormethyl und Halogen substituiertes Benzyl,
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl oder Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl oder Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R³ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁴ bedeutet (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet Wasserstoff oder (C₁-C₄)-Alkyl,
R⁶ bedeutet (C₁-C₄)-Alkyl,
R⁷ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, Methylsulfenyl, Methylsulfinyl, Methylsulfonyl, (C₃-C₆)-Cycloalkyl oder jeweils durch s Reste aus der Gruppe bestehend aus Methyl, Ethyl, Methoxy, Trifluormethyl und Halogen substituiertes Heteroaryl oder Heterocyclyl,
R⁸ bedeutet (C₁-C₆)-Alkyl, Halogen-(C₁-C₆)-alkyl, (C₁-C₆)-Alkyl-O-(C₁-C₆)-alkyl, (C₂-C₆)-Alkenyl oder (C₃-C₆)-Alkinyl,
R⁹ bedeutet (C₁-C₆)-Alkyl, Halogen-(C₁-C₆)-alkyl, (C₂-C₆)-Alkenyl oder (C₃-C₆)-Alkinyl,
R¹⁰ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹¹ bedeutet (C₁-C₆)-Alkyl, (C₂-C₆)-Alkenyl oder (C₂-C₆)-Alkinyl,
R^{1'} bedeutet Cyano, (C₁-C₆)-Alkyl, (C₃-C₆)-Cycloalkyl, Halogen-(C₁-C₆)-alkyl, OR⁸, SR⁸, NR⁸R⁹,
R^{2'} bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, wobei die letzten sechs Reste jeweils durch s Reste aus der Gruppe bestehend aus Cyano, Halogen, Nitro, Rhodano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind,
   oder R^{2'} bedeutet Phenyl, Phenyl-(C₁-C₆)-alkyl, Heteroaryl, (C₁-C₆)-Alkyl-heteroaryl, Heterocyclyl, (C₁-C₆)-Alkyl-heterocyclyl, (C₁-C₆)-Alkyl-O-heteroaryl, (C₁-C₆)-Alkyl-O-heterocyclyl, (C₁-C₆)-Alkyl-NR¹⁰-heteroaryl oder (C₁-C₆)-Alkyl-NR¹⁰-heterocyclyl, wobei die zehn letztgenannten Reste jeweils durch s Reste aus der Gruppe bestehend aus Cyano, Halogen, Nitro, Rhodano, (C₁-C₆)-Alkyl, Halogen-(C₁-C₆)-alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus Nitro, Cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, wobei die vier letztgenannten Reste selber wieder durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
   oder R^{X} bedeutet (C₃-C₇)-Cycloalkyl, Heteroaryl, Heterocyclyl oder Phenyl, wobei die vier vorstehend genannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl-S(O)ₙ, (C₁-C₆)-Alkoxy, Halogen-(C₁-C₆)-alkoxy und (C₁-C₆)-Alkoxy-(C₁-C₄)-alkyl substituiert sind,
R^{Y} bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, (C₁-C₆)-Alkoxy, Halogen-(C₁-C₆)-alkoxy, (C₂-C₆)-Alkenyloxy, (C₂-C₆)-Alkinyloxy, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl und wobei Heterocyclyl n Oxogruppen trägt,
R^{Z} bedeutet Wasserstoff, (C₁-C₆)-Alkyl, R¹O-(C₁-C₆)-Alkyl, R⁷CH₂, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, R¹O, R¹(H)N, Methoxycarbonyl, Ethoxycarbonyl, Methylcarbonyl, Dimethylamino, Trifluormethylcarbonyl, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl, wobei Heterocyclyl n Oxogruppen trägt,
W bedeutet Wasserstoff, Halogen, Nitro, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, Halogen-(C₃-C₇)-cycloalkyl, (C₁-C₆)-Alkoxy, Halogen-(C₁-C₆)-alkoxy, (C₁-C₆)-Alkyl-(O)ₙS-, (C₁-C₆)-Halogenalkyl-(O)ₙS-, (C₁-C₆)-Alkoxy-(C₁-C₄)-alkyl, (C₁-C₆)-Alkoxy-(C₁-C₄)-halogenalkyl, R¹(O)C, R¹(R¹ON=)C, R¹O(O)C, (R¹)₂N, R¹(O)C(R¹)N oder R²(O)₂S(R¹)N,
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R¹O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R¹O(O)CO, (R¹)₂N(O)CO, (R¹)₂N, R¹(O)C(R¹)N, R²(O)₂S(R¹)N, R¹O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R¹O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R¹O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R¹O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R¹O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R¹⁰(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R¹⁰(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R¹⁰(O)CO, (R¹)₂N(O)CO, (R¹)₂N, R¹(O)C(R¹)N, R²(O)₂S(R¹)N, R¹O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R¹O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R¹O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R¹O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R¹O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R¹O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
n bedeutet 0, 1 oder 2,
s bedeutet 0, 1, 2 oder 3.

Bevorzugt sind Verbindungen der allgemeinen Formel (I), worin die Symbole und Indices folgende Bedeutungen haben:
Q bedeutet einen Rest Q1, Q2, Q3 oder Q4, 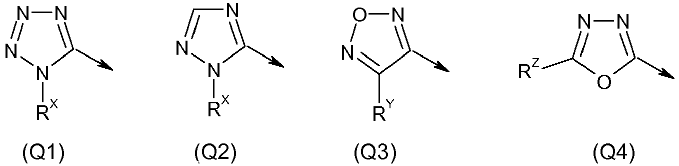
R bedeutet Wasserstoff, (C₁-C₆)-Alkyl, Halogen-(C₁-C₆)-alkyl, R¹(O)C-(C₁-C₆)-Alkyl, R¹O(O)C-(C₁-C₆)-Alkyl, R¹O(O)C-O-(C₁-C₆)-Alkyloxy, R¹O-(C₁-C₆)-Alkyl, R¹(O)C, R¹O(O)C, R¹O, R²(O)₂S oder jeweils durch s Reste aus der Gruppe bestehend aus Methyl, Ethyl, Methoxy, Nitro, Trifluormethyl und Halogen substituiertes Benzyl,
R¹ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkenyl, (C₁-C₆)-Alkyl-O-(C₁-C₆)-alkyl, Phenyl, Heteroaryl oder Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₁-C₆)-Alkyl-O-(C₁-C₆)-alkyl, Phenyl, Heteroaryl oder Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R³ bedeutet Wasserstoff, (C₁-C₆)-Alkyl, Halogen-(C₁-C₆)-alkyl, (C₃-C₆)-Cycloalkyl oder Phenyl,
R⁴ bedeutet (C₁-C₆)-Alkyl, Halogen-(C₁-C₆)-alkyl, (C₃-C₆)-Cycloalkyl oder Phenyl,
R⁵ bedeutet Wasserstoff oder (C₁-C₄)-Alkyl,
R⁶ bedeutet (C₁-C₄)-Alkyl,
R⁷ bedeutet Acetoxy, Benzoyloxy, Methoxycarbonyl, Ethoxycarbonyl, Methylcarbonyl, Trifluormethylcarbonyl, Methylsulfenyl, Methylsulfinyl, Methylsulfonyl, (C₃-C₆)-Cycloalkyl oder jeweils durch s Reste aus der Gruppe bestehend aus Methyl, Ethyl, Methoxy, Trifluormethyl und Halogen substituiertes Heteroaryl oder Heterocyclyl,
R⁸ bedeutet (C₁-C₆)-Alkyl, Halogen-(C₁-C₆)-alkyl, (C₁-C₆)-Alkyl-O-(C₁-C₆)-alkyl, (C₂-C₆)-Alkenyl oder (C₃-C₆)-Alkinyl,
R⁹ bedeutet (C₁-C₆)-Alkyl oder Halogen-(C₁-C₆)-alkyl,
R¹⁰ bedeutet Wasserstoff, (C₁-C₆)-Alkyl, Halogen-(C₁-C₆)-alkyl oder (C₃-C₆)-Cycloalkyl,
R¹¹ bedeutet (C₁-C₆)-Alkyl,
R^{1'} bedeutet (C₁-C₆)-Alkyl, (C₃-C₆)-Cycloalkyl, Halogen-(C₁-C₆)-alkyl, OR⁸, SR⁸ oder NR⁸R⁹,
R^{2'} bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, wobei die letzten sechs Reste jeweils durch s Reste aus der Gruppe bestehend aus Cyano, Halogen, OR¹⁰, S(O)ₙR¹¹, N(R¹⁰)₂, COR¹⁰, CO₂R¹⁰ und (C₁-C₄)-Alkoxy-(C₂-C₆)-alkoxycarbonyl substituiert sind,
   oder R^{2'} bedeutet Phenyl, Heteroaryl, Heterocyclyl, wobei die drei letztgenannten Reste jeweils durch s Reste aus der Gruppe bestehend aus Cyano, Halogen, Nitro, Rhodano, (C₁-C₆)-Alkyl, Halogen-(C₁-C₆)-alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, wobei die fünf vorstehend genannten Reste jeweils durch s Reste aus der Gruppe bestehend aus Cyano, (R⁶)₃Si, (R⁵O)₂(O)P, R²(O)ₙS, (R¹)₂N, R¹O, R¹(O)C, R¹O(O)C, R¹(O)CO, R²O(O)CO, (C₃-C₆)-Cycloalkyl, Heteroaryl, Heterocyclyl und Phenyl substituiert sind, wobei die vier letztgenannten Reste selber wieder durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
   oder R^{X} bedeutet (C₃-C₇)-Cycloalkyl, Heteroaryl, Heterocyclyl oder Phenyl, wobei die vier vorstehend genannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiert sind,
R^{Y} bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₇)-Cycloalkyl, (C₁-C₆)-Alkoxy, Cyano, Methylsulfenyl, Methylsulfinyl, Methylsulfonyl, Acetylamino, Methoxycarbonyl, Ethoxycarbonyl, Methoxycarbonylmethyl, Ethoxycarbonylmethyl, Benzoyl, Methylcarbonyl, Trifluormethylcarbonyl, Halogen, Amino, Methylaminocarbonyl, Dimethylaminocarbonyl, Methoxymethyl, oder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl und wobei Heterocyclyl n Oxogruppen trägt,
R^{Z} bedeutet Wasserstoff, (C₁-C₆)-Alkyl, R¹O-(C₁-C₆)-Alkyl, R⁷CH₂, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, R¹O, R¹(H)N, Methoxycarbonyl, Ethoxycarbonyl, Methylcarbonyl, Dimethylamino, Trifluormethylcarbonyl, Acetylamino, Methylsulfenyl, Methylsulfinyl, Methylsulfonyl oder jeweils durch s Reste aus der Gruppe bestehend aus Halogen, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl, wobei Heterocyclyl n Oxogruppen trägt,
W bedeutet Wasserstoff, Halogen, (C₁-C₆)-Alkyl, Halogen-(C₁-C₆)-alkyl, (C₂-C₆)-Alkenyl, (C₂-C₆)-Alkinyl, (C₃-C₇)-Cycloalkyl, (C₁-C₆)-Alkoxy, Halogen-(C₁-C₆)-alkoxy, (C₁-C₆)-Alkyl-(O)ₙS-, (C₁-C₆)-Alkoxy-(C₁-C₄)-alkyl, (C₁-C₆)-Alkoxy-(C₁-C₄)-halogenalkyl, R¹(O)C, (R¹)₂N, R¹(O)C(R¹)N oder R²(O)₂S(R¹)N,
X bedeutet Nitro, Halogen, Cyano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-Cycloalkyl-(C₁-C₆)-alkyl, R¹(O)C, R¹(R¹ON=)C, R¹O(O)C, (R¹)₂N(O)C, R¹O, R¹(O)CO, R¹O(O)CO, (R¹)₂N, R¹(O)C(R¹)N, R²(O)₂S(R¹)N, R¹O(O)C(R¹)N, R²(O)ₙS, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R¹O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R¹O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, (R⁵O)₂(O)P-(C₁-C₆)-Alkyl, Phenyl, Heteroaryl, Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, R¹(O)CO, R²(O)₂SO, R¹O(O)CO, (R¹)₂N(O)CO, (R¹)₂N, R¹(O)C(R¹)N, R²(O)₂S(R¹)N, R¹O(O)C(R¹)N, R²(O)ₙS, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R¹O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R¹O(O)C(R¹)N-(C₁-C₆)-Alkyl, (R¹)₂N(O)C(R¹)N-(C₁-C₆)-Alkyl, R¹⁰(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, (R⁵O)₂(O)P-(C₁-C₆)-Alkyl, Phenyl, Heteroaryl, Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
n bedeutet 0, 1 oder 2,
s bedeutet 0, 1, 2 oder 3.

Besonders bevorzugt sind Verbindungen der allgemeinen Formel (I), worin die Symbole und Indices folgende Bedeutungen haben:
Q bedeutet einen Rest Q1, Q2, Q3 oder Q4, 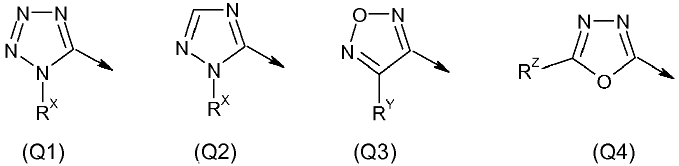
R bedeutet Wasserstoff, (C₁-C₆)-Alkyl, Halogen-(C₁-C₆)-alkyl, R¹(O)C-(C₁-C₆)-Alkyl, R¹O(O)C-O-(C₁-C₆)-Alkyloxy, R¹(O)C, R¹O(O)C oder R²(O)₂S,
R¹ bedeutet Wasserstoff, (C₁-C₆)-Alkyl, Halogen-(C₁-C₆)-alkyl, (C₃-C₆)-Cycloalkyl, (C₁-C₆)-Alkyl-O-(C₁-C₆)-alkyl, Phenyl, Heteroaryl oder Heterocyclyl, wobei die drei letztgenannten Reste jeweils durch s Reste aus der Gruppe bestehend aus Halogen, (C₁-C₆)-Alkyl, Halogen-(C₁-C₆)-alkyl und R³O(O)C substituiert sind, und wobei Heterocyclyl n Oxogruppen trägt,
R² bedeutet (C₁-C₆)-Alkyl, Halogen-(C₁-C₆)-alkyl, (C₃-C₆)-Cycloalkyl, (C₁-C₆)-Alkyl-O-(C₁-C₆)-alkyl, Phenyl, Heteroaryl oderHeterocyclyl, wobei die drei letztgenannten Reste jeweils durch s Reste aus der Gruppe bestehend aus Halogen, (C₁-C₆)-Alkyl, Halogen-(C₁-C₆)-alkyl, R³O(O)C, R³O, R⁴(O)ₙS und R³O-(C₁-C₆)-Alkyl substituiert sind, und wobei Heterocyclyl n Oxogruppen trägt,
R³ bedeutet Wasserstoff, (C₁-C₆)-Alkyl oder Halogen-(C₁-C₆)-alkyl,
R⁴ bedeutet (C₁-C₆)-Alkyl oder Halogen-(C₁-C₆)-alkyl,
R⁵ bedeutet Wasserstoff oder (C₁-C₄)-Alkyl,
R⁶ bedeutet (C₁-C₄)-Alkyl,
R⁷ bedeutet Acetoxy, Methylsulfenyl oder (C₃-C₆)-Cycloalkyl,
R⁸ bedeutet (C₁-C₆)-Alkyl oder Halogen-(C₁-C₆)-alkyl,
R⁹ bedeutet (C₁-C₆)-Alkyl,
R¹⁰ bedeutet Wasserstoff, (C₁-C₆)-Alkyl oder Halogen-(C₁-C₆)-alkyl,
R¹¹ bedeutet (C₁-C₆)-Alkyl,
R^{1'} bedeutet (C₁-C₆)-Alkyl, (C₃-C₆)-Cycloalkyl, Halogen-(C₁-C₆)-alkyl, OR⁸, SR⁸, NR⁸R⁹
R^{2'} bedeutet Wasserstoff, (C₁-C₆)-Alkyl, (C₃-C₆)-Cycloalkyl, (C₃-C₆)-Cycloalkyl-(C₁-C₆)-alkyl, wobei die letzten drei Reste jeweils durch s Reste aus der Gruppe bestehend aus Cyano, Halogen, OR¹⁰, S(O)ₙR¹¹, N(R¹⁰)₂, COR¹⁰, CO₂R¹⁰ und (C₁-C₄)-Alkoxy-(C₂-C₆)-alkoxycarbonyl substituiert sind,
   oder R^{2'} bedeutet Phenyl, Heteroaryl, Heterocyclyl, wobei die drei letztgenannten Reste jeweils durch s Reste aus der Gruppe bestehend aus Cyano, Halogen, (C₁-C₆)-Alkyl, Halogen-(C₁-C₆)-alkyl, OR¹⁰, S(O)ₙR¹¹, N(R¹⁰)₂, COR¹⁰ und CO₂R¹⁰ substituiert sind, und wobei Heterocyclyl n Oxogruppen trägt,
R^{X} bedeutet (C₁-C₆)-Alkyl, Halogen-(C₁-C₆)-alkyl, (C₂-C₆)-Alkenyl, (C₂-C₆)-Alkinyl, wobei die vier vorstehend genannten Reste jeweils durch s Reste aus der Gruppe bestehend aus Cyano, (R⁶)₃Si, (R⁵O)₂(O)P, R²(O)ₙS, (R¹)₂N, R¹O, R¹(O)C, R¹O(O)C, (C₃-C₆)-Cycloalkyl, Heteroaryl, Heterocyclyl und Phenyl substituiert sind, wobei die vier letztgenannten Reste selber wieder durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
   oder R^{X} bedeutet (C₃-C₇)-Cycloalkyl, Heteroaryl, Heterocyclyl oder Phenyl, wobei die vier vorstehend genannten Reste jeweils durch s Reste aus der Gruppe bestehend aus Halogen, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiert sind,
R^{Y} bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₇)-Cycloalkyl, (C₁-C₆)-Alkoxy, Methylsulfenyl, Methylsulfinyl, Methylsulfonyl, Acetylamino, Methoxycarbonyl, Ethoxycarbonyl, Methoxycarbonylmethyl, Ethoxycarbonylmethyl, Benzoyl, Methylcarbonyl, Trifluormethylcarbonyl, Halogen, Methoxymethyl, oder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Phenyl, R^{Z} bedeutet Wasserstoff, (C₁-C₆)-Alkyl, R¹O-(C₁-C₆)-Alkyl, R⁷CH₂, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, R¹O, R¹(H)N, Methoxycarbonyl, Ethoxycarbonyl, Methylcarbonyl, Dimethylamino, Trifluormethylcarbonyl, Acetylamino, Methylsulfenyl, Methylsulfinyl, Methylsulfonyl oder jeweils durch s Reste aus der Gruppe bestehend aus Halogen, Cyano, (C₁-C₆)-Alkyl, Halogen-(C₁-C₆)-alkyl, (C₃-C₆)-Cycloalkyl, (C₁-C₆)-Alkyl-S(O)ₙ, (C₁-C₆)-Alkoxy und Halogen-(C₁-C₆)-alkoxy substituiertes Phenyl,
W bedeutet Wasserstoff, Halogen, (C₁-C₆)-Alkyl, Halogen-(C₁-C₆)-alkyl, (C₃-C₇)-Cycloalkyl, (C₁-C₆)-Alkoxy, Halogen-(C₁-C₆)-alkoxy, (C₁-C₆)-Alkyl-S(O)ₙ oder (C₁-C₆)-Alkoxy-(C₁-C₄)-alkyl,
X bedeutet Halogen, (C₁-C₆)-Alkyl, Halogen-(C₁-C₆)-alkyl, (C₃-C₆)-Cycloalkyl, R¹(O)C, R¹O(O)C, (R¹)₂N(O)C, R¹O, (R¹)₂N, R²(O)ₙS, R¹O-(C₁-C₆)-Alkyl, (R¹)₂N-(C₁-C₆)-Alkyl, R²(O)ₙS-(C₁-C₆)-Alkyl, Phenyl, wobei der letztgenannte Rest jeweils durch s Reste aus der Gruppe bestehend aus Halogen, (C₁-C₆)-Alkyl, Halogen-(C₁-C₆)-alkyl, R¹O(O)C, R¹O, (R¹)₂N, R²(O)ₙS und R¹O-(C₁-C₆)-Alkyl substituiert ist,
Y bedeutet Wasserstoff, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, R¹(O)C, R¹O(O)C, R¹O, (R¹)₂N, R²(O)ₙS, (R⁵O)₂(O)P, R¹O-(C₁-C₆)-Alkyl, (R¹)₂N-(C₁-C₆)-Alkyl, R²(O)ₙS-(C₁-C₆)-Alkyl, Phenyl, wobei der letztgenannte Rest jeweils durch s Reste aus der Gruppe bestehend aus Halogen, (C₁-C₆)-Alkyl, Halogen-(C₁-C₆)-alkyl, R¹O(O)C, R¹O, (R¹)₂N, R²(O)ₙS und R¹O-(C₁-C₆)-Alkyl substituiert ist,
n bedeutet 0, 1 oder 2,
s bedeutet 0, 1, 2 oder 3.

Ganz besonders bevorzugt sind Verbindungen der allgemeinen Formel (I), worin die Symbole und Indices folgende Bedeutungen haben:
Q bedeutet einen Rest Q1, Q2, Q3 oder Q4, 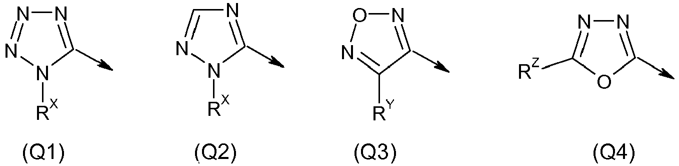
R bedeutet Wasserstoff,
R^{1'} bedeutet Methyl, Ethyl, Trifluormethyl oder cyclo-Propyl,
R^{2'} bedeutet Methyl, Ethyl, 2,2,2-Trifluorethyl, cyclo-Propylmethyl oder Trifluormethyl,
R^{x} bedeutet Methyl, Ethyl, Propyl, Methoxyethyl, 2-Methoxy-2-methyl-1-propyl, - oder Phenyl,
R^{y} bedeutet Chlor, Methyl oder Ethyl,
R^{z} bedeutet Chlor, Methyl oder Ethyl,
X bedeutet Fluor, Chlor, Brom, Methyl, Methoxy, Methylsulfonyl, Methoxymethyl, Methylsulfenyl, Trifluormethyl oder cyclo-Propyl,
Y bedeutet Chlor, Methyl, Ethyl, cyclo-Propyl, Allyl, Vinyl, Trifluormethyl, Difluormethyl, Chlordifluormethyl, Pentafluorethyl, Methoxy, Methylsulfenyl, Methylsulfinyl, Methylsulfonyl oder Ethylsulfonyl,
W bedeutet Wasserstoff, Fluor, Chlor, Methyl oder Trifluormethyl.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder lod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente.

Die Verbindungen der allgemeinen Formel (I) können je nach Art und Verknüpfung der Substituenten als Stereoisomere vorliegen. Sind beispielsweise ein oder mehrere asymmetrische Kohlenstoffatome vorhanden, so können Enantiomere und Diastereomere auftreten. Ebenso treten Stereoisomere auf, wenn n für 1 steht (Sulfoxide). Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind. Die erfindungsgemäßen Verbindungen können auf Grund der Oximether-Struktur auch als geometrische Isomere (E-/Z-Isomere) auftreten. Die Erfindung betrifft auch alle E-/Z-Isomere und deren Gemische, die von der allgemeinen Formel (I) umfasst, jedoch nicht spezifisch definiert sind. In der Regel fallen die E-Isomeren im Überschuss an.

Erfindungsgemäße Verbindungen, in denen Q für Q1 oder Q2 steht, können beispielsweise nach der in Schema 1 angegebenen Methode durch basenkatalysierte Umsetzung eines Benzoesäurechlorids (II) mit einem 5-Amino-1-H-1,2,4-triazol bzw. 5-Amino-1H-tetrazol (III) hergestellt werden:

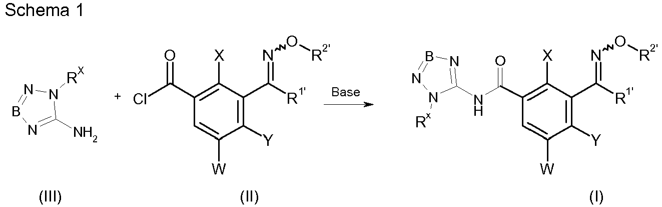

Darin steht B für CH oder N. Die Benzoesäurechloride der Formel (II) beziehungsweise die ihnen zugrunde liegenden Benzoesäuren sind grundsätzlich bekannt und können beispielsweise gemäß den in WO 98/29392 und WO 98/29384, bzw. in JP 11021274 und WO 98/45273 beschriebenen Methoden hergestellt werden. Bei der Synthese dieser Benzoesäurechloriden, bzw. den ihnen zugrundeliegenden Benzoesäuren kann je nach Syntheseführung oder Substitutionsmustern nur eins der beiden möglichen Isomeren (E- oder Z-Isomer) oder auch E/Z-Gemische entstehen. Für die Synthesen der erfindungsgemäßen Verbindungen (I) können solche E/Z-Gemische eingesetzt werden, wobei nach der Aufarbeitung, bzw. bei der Reinigung die Verbindungen des Typs I isomerenrein isoliert werden können. Alternativ werden die E/Z-Gemische beispielsweise der Benzoesäuren mittels präparativer HPLC getrennt, um dann die Isomere einzeln zu den erfindungsgemäßen Benzoylamiden (I) umzusetzen.

Erfindungsgemäße Verbindungen, in denen Q für Q1 oder Q2 steht, können auch nach der in Schema 2 angegebenen Methode durch Umsetzung einer Benzoesäure der Formel (IV) mit einem 5-Amino-1-H-1,2,4-triazol, bzw. 5-Amino-1H-tetrazol (III) hergestellt werden:

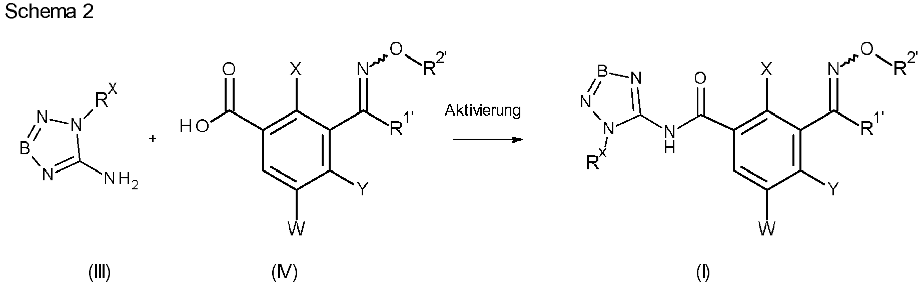

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Erfindungsgemäße Verbindungen, in denen Q für Q1 oder Q2 steht, können auch nach der in Schema 3 angegebenen Methode durch Umsetzung eines N-(1H-1,2,4-triazol-5-yl)benzamids oder eines N-(1H-tetrazol-5-yl)benzamids hergestellt werden:

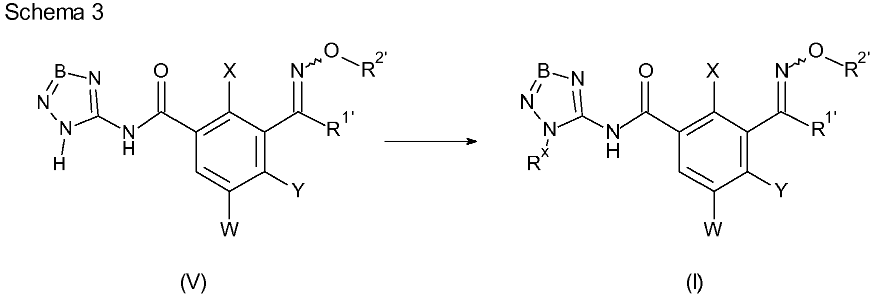

Für diese in Schema 3 genannte Reaktion können z. B. Alkylierungsmittel wie z. B. Alkylhalogenide, -sulfonate oder Dialkylsulfate in Gegenwart einer Base eingesetzt werden.

Die 5-Amino-1H-tetrazole der Formel (III) sind entweder käuflich erhältlich oder können analog zu literaturbekannten Methoden hergestellt werden. Beispielsweise können substituierte 5-Aminotetrazole nach der in Journal of the American Chemical Society (1954), 76, 923-924 beschriebenen Methode aus Amino-tetrazol hergestellt werden:

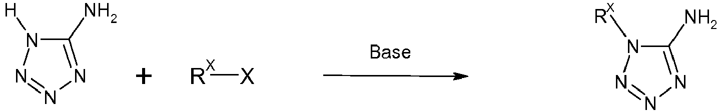

In der vorstehend genannten Reaktion bedeutet X eine Abgangsgruppe wie Iod. Substituierte 5-Aminotetrazole können zum Beispiel auch wie in Journal of the American Chemical Society (1954) 76, 88-89 beschrieben, synthetisiert werden:

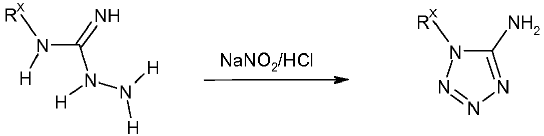

Die 5-Amino-1H-triazole der Formel (III) sind entweder käuflich erhältlich oder können analog zu literaturbekannten Methoden hergestellt werden. Beispielsweise können substituierte 5-Aminotriazole nach der in Zeitschrift für Chemie (1990), 30(12), 436 - 437 beschriebenen Methode aus Aminotriazol hergestellt werden:

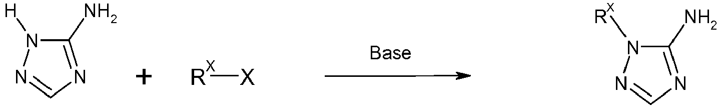

Substituierte 5-Aminotriazole können auch zum Beispiel wie in Chemische Berichte (1964), 97(2), 396-404 beschrieben synthetisiert werden:

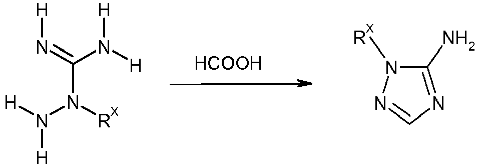

Substituierte 5-Aminotriazole können auch zum Beispiel wie in Angewandte Chemie (1963), 75, 918 beschrieben, synthetisiert werden:

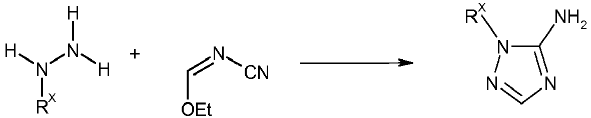

Erfindungsgemäße Verbindungen, in denen Q für Q3 steht, können beispielsweise nach der in Schema 4 angegebenen Methode durch basenkatalysierte Umsetzung eines Benzoesäurechlorids (II) mit einem 4-Amino-1,2,5-oxadiazol (VI) hergestellt werden:

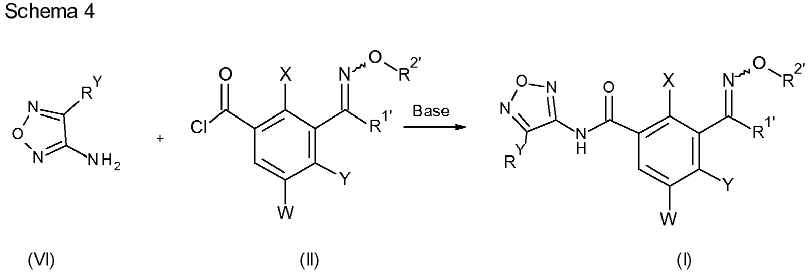

Erfindungsgemäße Verbindungen können auch nach der in Schema 5 angegebenen Methode durch Umsetzung einer Benzoesäure der Formel (IV) mit einem 4-Amino-1,2,5-oxadiazol (VI) hergestellt werden:

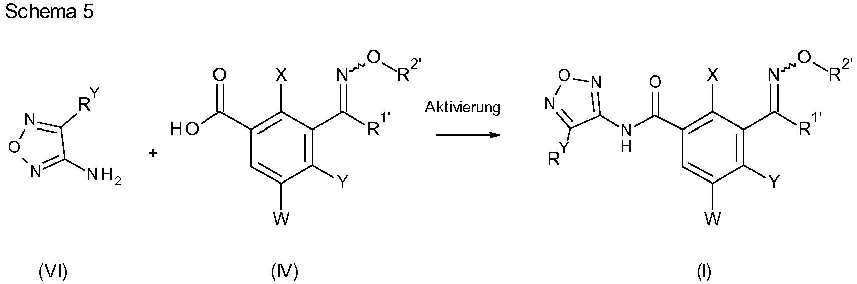

Für die Aktivierung können wasserentziehende Reagenzien die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc., eingesetzt werden.

Die 4-Amino-1,2,5-oxadiazole der Formel (VI) sind entweder käuflich erhältlich oder bekannt oder können analog zu literaturbekannten Methoden hergestellt werden. Beispielsweise können 3-Alkyl-4-amino-1,2,5-oxadiazole nach der in Russian Chemical Bulletin, Int. Ed., Vol. 54, No. 4, S. 1032-1037 (2005) beschriebenen Methode aus β-Ketoestern hergestellt werden:

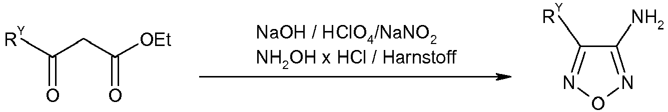

3-Aryl-4-amino-1,2,5-oxadiazole können zum Beispiel wie in Russian Chemical Bulletin, 54(4), 1057-1059, (2005) oder Indian Journal of Chemistry, Section B: Organic Chemistry Including Medicinal Chemistry, 26B(7), 690-2, (1987) beschrieben, synthetisiert werden:

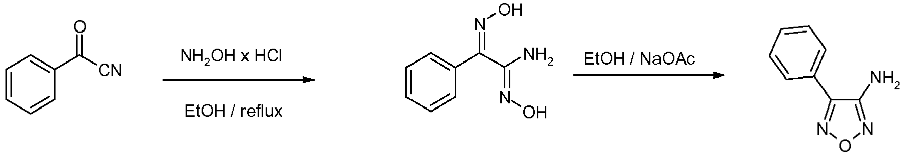

3-Amino-4-Halogen-1,2,5-oxadiazole können beispielsweise nach der in Heteroatom Chemistry 15(3), 199-207 (2004) beschrieben Methode aus dem käuflich erhältlichen 3,4-Diamino-1,2,5-oxadiazol durch eine Sandmeyer-Reaktion hergestellt werden:

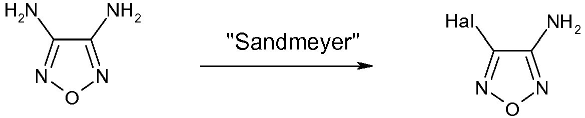

Nucleophile Reste R^{Y} können wie in Journal of Chemical Research, Synopses, (6), 190, 1985 oder in oder Izvestiya Akademii Nauk SSSR, Seriya Khimicheskaya, (9), 2086-8, 1986 oder in Russian Chemical Bulletin (Translation of Izvestiya Akademii Nauk, Seriya Khimicheskaya), 53(3), 596-614, 2004 beschrieben, durch Substitution der Austrittsgruppe L in 3-Amino-1,2,5-oxadiazolen eingeführt werden. L steht für eine Abgangsgruppe wie z. B. Chlor, Brom, Jod, Mesyloxy, Tosyloxy, Trifluorsulfonyloxy etc..

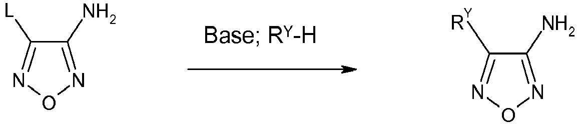

Erfindungsgemäße Verbindungen, in denen Q für Q4 steht, können beispielsweise nach der in Schema 6 angegebenen Methode durch basenkatalysierte Umsetzung eines Benzoesäurechlorids (II) mit einem 2-Amino-1,3,4-oxadiazol (VII) hergestellt werden:

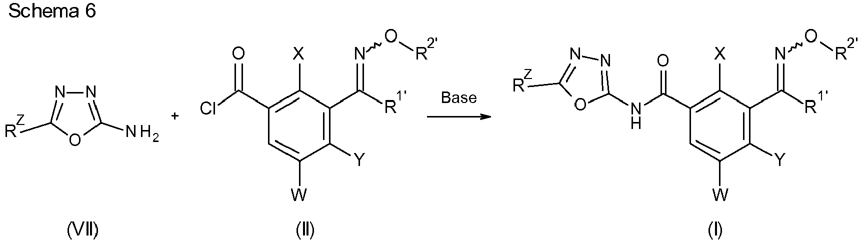

Erfindungsgemäße Verbindungen können auch nach der in Schema 7 angegebenen Methode durch Umsetzung einer Benzoesäure der Formel (IV) mit einem 2-Amino-1,3,4-oxadiazol (VII) hergestellt werden:

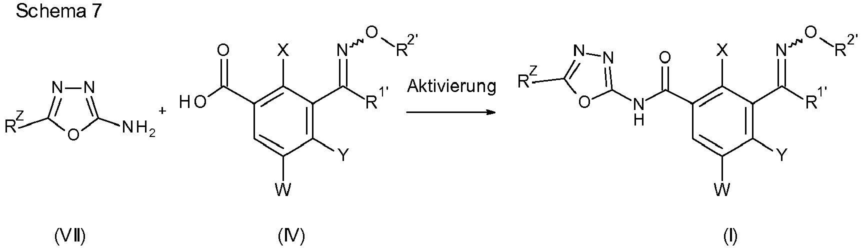

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Erfindungsgemäße Verbindungen können auch nach der in Schema 8 angegebenen Methode durch Cyclisierung einer Verbindung der Formel VIII hergestellt werden:

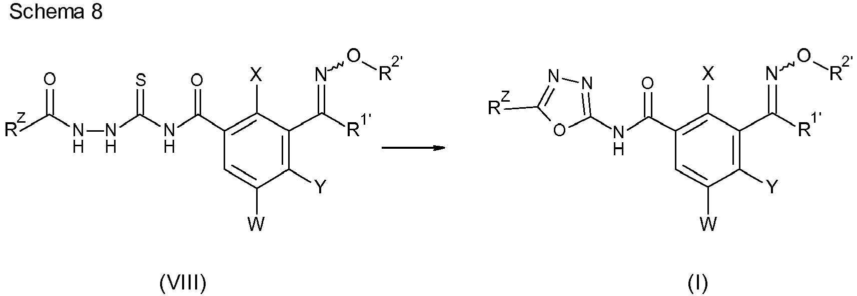

Die Cyclisierung kann gemäß der in Synth. Commun. 31 (12), 1907-1912 (2001) oder der in Indian J. Chem., Section B: Organic Chemistry Including Medicinal Chemistry; Vol. 43 (10), 2170-2174 (2004) beschriebenen Methoden durchgeführt werden.

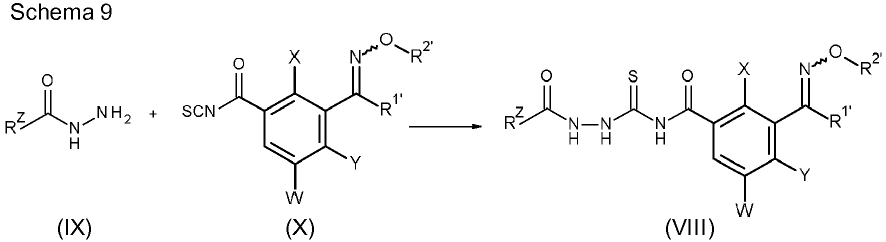

Die in Schema 8 eingesetzte Verbindung der Formel VIII kann durch Umsetzung eines Acylisothiocyanats der Formel X mit einem Hydrazid der Formel IX gemäß der von in Synth. Commun. 25(12), 1885-1892 (1995) beschriebenen Methode hergestellt werden.

Erfindungsgemäße Verbindungen, in denen der Substituent R nicht Wasserstoff bedeutet, können beispielsweise nach der in Schema 10 angegebenen Methode durch Umsetzung eines N-(1,2,5-Oxadiazol-3-yl)-, N-(1,3,4-Oxadiazol-2-yl), N-(Tetrazol-5-yl)- oder N-(Triazol-5-yl)- arylcarbonsäureamids (I) mit einer Verbindung der allgemeinen Formel (III), wobei L für eine Abgangsgruppe wie z. B. ein Chlor, Brom, Jod, Mesyloxy, Tosyloxy, Trifluorsulfonyloxy etc. steht, hergestellt werden:

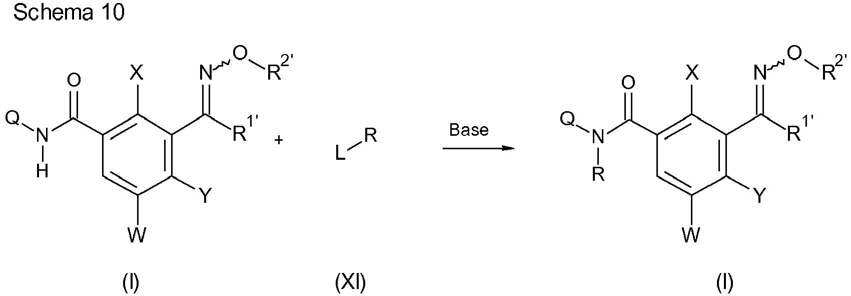

Die Verbindungen der Formel (XI), bei denen L eine Abgangsgruppe wie z. B. Chlor, Brom, Jod, Methylsulfonyloxy, Tosyloxy oder Trifluorsulfonyloxy bedeutet, sind entweder käuflich oder können nach bekannten in der Literatur beschriebenen Methoden hergestellt werden.

Erfindungsgemäße Verbindungen können auch nach der in Schema 11 angegebenen Methode durch Umsetzung eines Amins der Formel (XII) mit einem Säurechlorid (II), wie zum Beispiel in J. Het: Chem. (1972), 9 (1), 107-109) beschrieben, hergestellt werden:

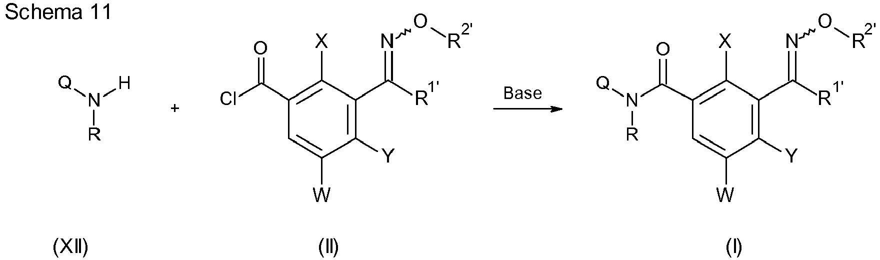

Erfindungsgemäße Verbindungen können auch nach der in Schema 12 angegebenen Methode durch Umsetzung eines Amins der Formel (XII) mit einer Säure der Formel (IV) hergestellt werden:

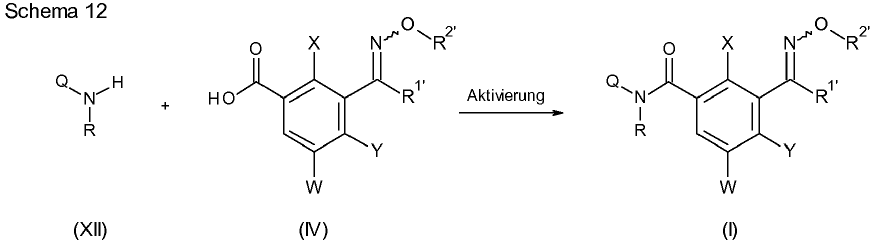

Für die Aktivierung können wasserentziehende Reagenzien, die üblicherweise für Amidierungsreaktionen, wie z. B. 1,1'-Carbonyldiimidazol (CDI), Dicyclohexylcarbodiimid (DCC), 2,4,6-Tripropyl-1,3,5,2,4,6-trioxatriphosphinane 2,4,6-trioxide (T3P) etc. eingesetzt werden.

Die Amine der Formel (XII) sind entweder käuflich oder in der Literatur bekannt oder können beispielsweise nach der in Schema 13 beschriebenen Methoden durch basenkatalysierte Alkylierung oder durch reduktive Alkylierung oder nach der in Schema 14 beschriebenen Methode durch nucleophile Substitution einer Abgangsgruppe L durch Amine R-NH₂ hergestellt werden.

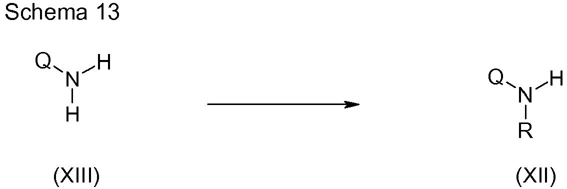

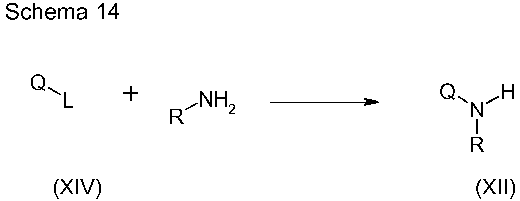

Die Amine der Formel (XII) können auch durch Cyclisierungsreaktionen wie zum Beispiel in J. Org. Chem. 73(10), 3738-3744 (2008) für Q = Q1 oder in Buletinul Institutului Politehnic din lasi (1974), 20(1-2), 95-99 oder in J. Org. Chem. 67(21), 7361-7364 (2002) für Q = Q4 beschrieben, hergestellt werden.

Es kann zweckmäßig sein, Reaktionsschritte in ihrer Reihenfolge zu ändern. So sind Benzoesäuren, die ein Sulfoxid tragen, nicht ohne weiteres in ihre Säurechloride zu überführen. Hier bietet sich an, zunächst auf Thioether-Stufe das Amid zu herzustellen und danach den Thioether zum Sulfoxid zu oxidieren.

Die Aufarbeitung der jeweiligen Reaktionsmischungen erfolgt in der Regel nach bekannten Verfahren, beispielsweise durch Kristallisation, wässrig-extraktive Aufarbeitung, durch chromatographische Methoden oder durch Kombination dieser Methoden.

Kollektionen aus Verbindungen der Formel (I), die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasenunterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) in Form von Substanzkollektionen, die Bibliotheken genannt werden. Gegenstand der vorliegenden Erfindung sind auch Bibliotheken, die mindestens zwei Verbindungen der Formel (I) enthalten.

Die erfindungsgemäßen Verbindungen der Formel (I), im Folgenden als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Bevorzugt ist die Anwendung der erfindungsgemäßen Verbindungen in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-ÖI-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia ofSurface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### 1. Synthese von 3-[(1E oder Z)-N-(Cyclopropylmethoxy)ethanimidoyl]-2-methyl-4-(methylsulfonyl)-N-(1-methyl-1H-1,2,4-triazol-5-yl)benzamid (Beispiel-Nr. 4-74)):

200 mg (0.62 mmol) 3-[(1E oder Z)-N-(Cyclopropylmethoxy)ethanimidoyl]-2-methyl-4-(methylsulfonyl)benzoesäure werden mit 83 mg (0.8 mmol) 1-Methyl-1H-1,2,4-triazol-5-amin in 10 ml Pyridin vorgelegt und bei Raumtemperatur mit 0.07 ml (0.8 mmol) Oxalylchlorid versetzt. Die Reaktionslösung wurde 12h bei Raumtemperatur gerührt, danach wurde bis ins Trockne eingedampft. Der Rückstand wurde in Acetonitril aufgenommen und säulenchromatographisch getrennt (HPLC, C18, Gradient: Acetonitril/Wasser +0.05%Trifluoressigsäure, 20:80→100:0 in 30 min). Man erhielt 92 mg 3-[(1E oder Z)-N-(Cyclopropylmethoxy)ethanimidoyl]-2-methyl-4-(methylsulfonyl)-N-(1-methyl-1H-1,2,4-triazol-5-yl)benzamid.

### 2. Synthese von 2,4-Dichlor-N-(4-chlor-1,2,5-oxadiazol-3-yl)-3-[(1E oder Z)-N-methoxyethanimidoyl]benzamid (Beispiel-Nr. 5-1)

200 mg (0.76 mmol) 2,4-Dichlor-3-[(1E oder Z)-N-methoxyethanimidoyl]benzoesäure werden mit 115.2 mg (0.92 mmol) 4-Chlor-1,2,5-oxadiazol-3-amin in 3 ml Pyridin vorgelegt und bei Raumtemperatur 0.1 ml (1.14 mmol) Oxalylchlorid versetzt. Die Reaktionslösung wurde 12h bei Raumtemperatur gerührt, danach wurden 10 ml Wasser zugegeben und 10 min gerührt. Daraufhin wurde die Mischung mit Dichlormethan extrahiert. Die organische Phase wurde mittels Phasenseperator abgetrennt und ins Trockne eingedampft. Der Rückstand wurde säulenchromatographisch getrennt (HPLC, C18, Gradient: Acetonitril/Wasser +0.05%Trifluoressigsäure, 20:80→100:0 in 30 min). Man erhielt 172 mg 2,4-Dichlor-N-(4-chlor-1,2,5-oxadiazol-3-yl)-3-[(1E oder Z)-N-methoxyethanimidoyl]benzamid.

Die in den nachfolgenden Tabellen aufgeführten Beispiele wurden analog oben genannten Methoden hergestellt beziehungsweise sind analog oben genannten Methoden erhältlich. Die in den nachfolgenden Tabellen aufgeführten Verbindungen sind ganz besonders bevorzugt. ¹H-NMR-Daten der in den Tabellen 1-14 mit einem (*) indizierten Verbindungen werden nach Tabelle 14 aufgeführt.

**Tabelle 1: Erfindungsgemäße Verbindungen der allgemeinen Formel (I), worin Q für Q1, R^{x} für eine Methylgruppe und R und W jeweils für Wasserstoff stehen (E-Isomer, ggf. Z-Isomer),**

| | | | | | |
|---|---|---|---|---|---|
| 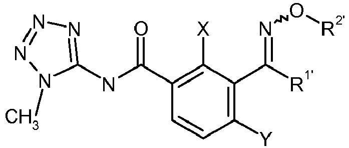 | | | | | |

| **Nr.** | **X** | **Y** | **R^{1'}** | **R^{2'}** | **Physikalische Daten** (¹H-NMR) |
|---|---|---|---|---|---|
| 1-1 | Cl | Cl | Me | Me | |
| 1-2 | Cl | Cl | Et | Me | |
| 1-3 | Cl | Cl | CF₃ | Me | |
| 1-4 | Cl | Cl | OMe | Me | |
| 1-5 | Cl | Cl | Me | Et | * |
| 1-6 | Cl | Cl | Me | Pr | |
| 1-7 | Cl | Cl | Me | c-Pr | |
| 1-8 | Cl | Cl | Me | CH₂-c-Pr | * |
| 1-9 | Cl | Cl | Me | CH₂-CF₃ | * |
| 1-10 | Cl | SMe | Me | Me | |
| 1-11 | Cl | SMe | Me | Et | |
| 1-12 | Cl | SMe | Me | CH₂-c-Pr | |
| 1-13 | Cl | SMe | Me | CH₂-CF₃ | |
| 1-14 | Cl | S(O)Me | Me | Me | |
| 1-15 | Cl | SO₂Me | Me | Me | * |
| 1-16 | Cl | SO₂Me | Et | Me | |
| 1-17 | Cl | SO₂Me | CF₃ | Me | |
| 1-18 | Cl | SO₂Me | OMe | Me | |
| 1-19 | Cl | SO₂Me | Me | Et | |
| 1-20 | Cl | SO₂Me | Me | Pr | |
| 1-21 | Cl | SO₂Me | Me | c-Pr | |
| 1-22 | Cl | SO₂Me | Me | CH₂-c-Pr | * |
| 1-23 | Cl | SO₂Me | Me | CH₂-CF₃ | |
| 1-24 | Cl | Me | Me | Me | |
| 1-25 | Cl | Me | Me | Et | |
| 1-26 | Cl | Me | Me | CH₂-c-Pr | |
| 1-27 | Cl | Me | Me | CH₂-CF₃ | |
| 1-28 | Cl | Et | Me | Me | |
| 1-29 | Cl | c-Pr | Me | Me | |
| 1-30 | Cl | CF₃ | Me | Me | |
| 1-31 | Cl | CF₃ | Et | Me | |
| 1-32 | Cl | CF₃ | CF₃ | Me | |
| 1-33 | Cl | CF₃ | OMe | Me | |
| 1-34 | Cl | CF₃ | Me | Et | |
| 1-35 | Cl | CF₃ | Me | Pr | |
| 1-36 | Cl | CF₃ | Me | c-Pr | |
| 1-37 | Cl | CF₃ | Me | CH₂-c-Pr | |
| 1-38 | Cl | CF₃ | Me | CH₂-CF₃ | |
| 1-39 | Cl | OMe | Me | Me | |
| 1-40 | Cl | OMe | Me | Et | |
| 1-41 | Cl | OMe | Me | CH₂-c-Pr | |
| 1-42 | Cl | OMe | Me | CH₂-CF₃ | |
| 1-43 | Cl | SO₂Et | Me | Me | |
| 1-44 | Cl | SO₂Et | Me | Et | |
| 1-45 | Cl | SO₂Et | Me | CH₂-c-Pr | |
| 1-46 | Cl | SO₂Et | Me | CH₂-CF₃ | |
| 1-47 | Cl | C₂F₅ | Me | Me | |
| 1-48 | Cl | Vinyl | Me | Me | |
| 1-49 | Cl | 1-Me-vinyl | Me | Me | |
| 1-50 | Br | SO₂Me | Me | Me | |
| 1-51 | Br | SO₂Me | Et | Me | |
| 1-52 | Br | SO₂Me | CF₃ | Me | |
| 1-53 | Br | SO₂Me | OMe | Me | |
| 1-54 | Br | SO₂Me | Me | Et | |
| 1-55 | Br | SO₂Me | Me | Pr | |
| 1-56 | Br | SO₂Me | Me | c-Pr | |
| 1-57 | Br | SO₂Me | Me | CH₂-c-Pr | |
| 1-58 | Br | SO₂Me | Me | CH₂-CF₃ | |
| 1-59 | Br | Me | Me | Me | |
| 1-60 | Br | Et | Me | Me | |
| 1-61 | Br | CF₃ | Me | Me | |
| 1-62 | Br | CF₃ | Me | Et | |
| 1-63 | Br | CF₃ | Me | CH₂-c-Pr | |
| 1-64 | Br | CF₃ | Me | CH₂-CF₃ | |
| 1-65 | Me | SMe | Me | Me | |
| 1-66 | Me | S(O)Me | Me | Me | |
| 1-67 | Me | SO₂Me | Me | Me | * |
| 1-68 | Me | SO₂Me | Et | Me | |
| 1-69 | Me | SO₂Me | CF₃ | Me | |
| 1-70 | Me | SO₂Me | OMe | Me | |
| 1-71 | Me | SO₂Me | Me | Et | * |
| 1-72 | Me | SO₂Me | Me | Pr | |
| 1-73 | Me | SO₂Me | Me | c-Pr | |
| 1-74 | Me | SO₂Me | Me | CH₂-c-Pr | * |
| 1-75 | Me | SO₂Me | Me | CH₂-CF₃ | |
| 1-76 | Me | Me | Me | Me | |
| 1-77 | Me | Me | Et | Me | |
| 1-78 | Me | Me | CF₃ | Me | |
| 1-79 | Me | Me | OMe | Me | |
| 1-80 | Me | Me | Me | Et | |
| 1-81 | Me | Me | Me | Pr | |
| 1-82 | Me | Me | Me | c-Pr | |
| 1-83 | Me | Me | Me | CH₂-c-Pr | |
| 1-84 | Me | Me | Me | CH₂-CF₃ | |
| 1-85 | Me | Et | Me | Me | |
| 1-86 | Me | CF₃ | Me | Me | |
| 1-87 | Me | CF₃ | Et | Me | |
| 1-88 | Me | CF₃ | CF₃ | Me | |
| 1-89 | Me | CF₃ | OMe | Me | |
| 1-90 | Me | CF₃ | Me | Et | |
| 1-91 | Me | CF₃ | Me | Pr | |
| 1-92 | Me | CF₃ | Me | c-Pr | |
| 1-93 | Me | CF₃ | Me | CH₂-c-Pr | |
| 1-94 | Me | CF₃ | Me | CH₂-CF₃ | |
| 1-95 | Me | SO₂Et | Me | Me | |
| 1-96 | Me | SO₂Et | Me | Et | |
| 1-97 | Me | SO₂Et | Me | CH₂-c-Pr | |
| 1-98 | Me | SO₂Et | Me | CH₂-CF₃ | |
| 1-99 | Me | C₂F₅ | Me | Me | |
| 1-100 | Me | c-Pr | Me | Me | |
| 1-101 | OMe | Cl | Me | Me | |
| 1-102 | OMe | CF₃ | Me | Me | |
| 1-103 | OMe | CF₃ | Me | Et | |
| 1-104 | OMe | CF₃ | Me | CH₂-c-Pr | |
| 1-105 | OMe | CF₃ | Me | CH₂-CF₃ | |
| 1-106 | OMe | CHF₂ | Me | Me | |
| 1-107 | OMe | CHF₂ | Et | Me | |
| 1-108 | OMe | CHF₂ | CF₃ | Me | |
| 1-109 | OMe | CHF₂ | OMe | Me | |
| 1-110 | OMe | CHF₂ | Me | Et | |
| 1-111 | OMe | CHF₂ | Me | Pr | |
| 1-112 | OMe | CHF₂ | Me | c-Pr | |
| 1-113 | OMe | CHF₂ | Me | CH₂-c-Pr | |
| 1-114 | OMe | CHF₂ | Me | CH₂-CF₃ | |
| 1-115 | OMe | CF₂Cl | Me | Me | |
| 1-116 | OMe | SO₂Me | Me | Me | |
| 1-117 | SO₂Me | CF₃ | Me | Me | |
| 1-118 | SO₂Me | CF₃ | Et | Me | |
| 1-119 | SO₂Me | CF₃ | CF₃ | Me | |
| 1-120 | SO₂Me | CF₃ | OMe | Me | |
| 1-121 | SO₂Me | CF₃ | Me | Et | |
| 1-122 | SO₂Me | CF₃ | Me | Pr | |
| 1-123 | SO₂Me | CF₃ | Me | c-Pr | |
| 1-124 | SO₂Me | CF₃ | Me | CH₂-c-Pr | |
| 1-125 | CH₂OMe | CF₃ | Me | CH₂-CF₃ | |
| 1-126 | SMe | CF₃ | Me | Me | |
| 1-127 | SMe | CF₃ | Me | Et | |
| 1-128 | SMe | CF₃ | Me | CH₂-c-Pr | |
| 1-129 | SMe | CF₃ | Me | CH₂-CF₃ | |
| 1-130 | SMe | SO₂Me | Me | Me | |
| 1-131 | CF₃ | CF₃ | Me | Me | |
| 1-132 | CF₃ | CF₃ | Me | Et | |
| 1-133 | CF₃ | CF₃ | Me | CH₂-c-Pr | |
| 1-134 | CF₃ | CF₃ | Me | CH₂-CF₃ | |
| 1-135 | F | CF₃ | Me | Me | |
| 1-136 | Me | SO₂Me | Me | i-Bu | |
| 1-137 | | | | | |

**Tabelle 2: Erfindungsgemäße Verbindungen der allgemeinen Formel (I), worin Q für Q1, R^{x} für eine Ethylgruppe und R und W jeweils für Wasserstoff stehen (E-Isomer, ggf. Z-Isomer),**

| | | | | | |
|---|---|---|---|---|---|
| 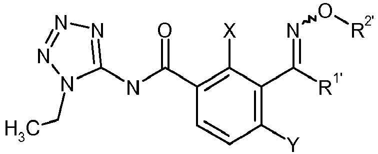 | | | | | |

| **Nr.** | **X** | **Y** | **R^{1'}** | **R^{2'}** | **Physikalische Daten** (¹H-NMR) |
|---|---|---|---|---|---|
| 2-1 | Cl | Cl | Me | Me | * |
| 2-2 | Cl | Cl | Et | Me | |
| 2-3 | Cl | Cl | CF₃ | Me | |
| 2-4 | Cl | Cl | OMe | Me | |
| 2-5 | Cl | Cl | Me | Et | |
| 2-6 | Cl | Cl | Me | Pr | |
| 2-7 | Cl | Cl | Me | c-Pr | |
| 2-8 | Cl | Cl | Me | CH₂-c-Pr | |
| 2-9 | Cl | Cl | Me | CH₂-CF₃ | |
| 2-10 | Cl | SMe | Me | Me | |
| 2-11 | Cl | SMe | Me | Et | |
| 2-12 | Cl | SMe | Me | CH₂-c-Pr | |
| 2-13 | Cl | SMe | Me | CH₂-CF₃ | |
| 2-14 | Cl | S(O)Me | Me | Me | |
| 2-15 | Cl | SO₂Me | Me | Me | * |
| 2-16 | Cl | SO₂Me | Et | Me | |
| 2-17 | Cl | SO₂Me | CF₃ | Me | |
| 2-18 | Cl | SO₂Me | OMe | Me | |
| 2-19 | Cl | SO₂Me | Me | Et | * |
| 2-20 | Cl | SO₂Me | Me | Pr | |
| 2-21 | Cl | SO₂Me | Me | c-Pr | |
| 2-22 | Cl | SO₂Me | Me | CH₂-c-Pr | * |
| 2-23 | Cl | SO₂Me | Me | CH₂-CF₃ | |
| 2-24 | Cl | Me | Me | Me | |
| 2-25 | Cl | Me | Me | Et | |
| 2-26 | Cl | Me | Me | CH₂-c-Pr | |
| 2-27 | Cl | Me | Me | CH₂-CF₃ | |
| 2-28 | Cl | Et | Me | Me | |
| | Cl | c-Pr | Me | Me | |
| 2-29 | Cl | CF₃ | Me | Me | |
| 2-30 | Cl | CF₃ | Et | Me | |
| 2-31 | Cl | CF₃ | CF₃ | Me | |
| 2-32 | Cl | CF₃ | OMe | Me | |
| 2-33 | Cl | CF₃ | Me | Et | |
| 2-34 | Cl | CF₃ | Me | Pr | |
| 2-35 | Cl | CF₃ | Me | c-Pr | |
| 2-36 | Cl | CF₃ | Me | CH₂-c-Pr | |
| 2-37 | Cl | CF₃ | Me | CH₂-CF₃ | |
| 2-38 | Cl | OMe | Me | Me | |
| 2-39 | Cl | OMe | Me | Et | |
| 2-40 | Cl | OMe | Me | CH₂-c-Pr | |
| 2-41 | Cl | OMe | Me | CH₂-CF₃ | |
| 2-42 | Cl | SO₂Et | Me | Me | |
| 2-43 | Cl | SO₂Et | Me | Et | |
| 2-44 | Cl | SO₂Et | Me | CH₂-c-Pr | |
| 2-45 | Cl | SO₂Et | Me | CH₂-CF₃ | |
| 2-46 | Cl | C₂F₅ | Me | Me | |
| 2-47 | Cl | Vinyl | Me | Me | |
| 2-48 | Cl | 1-Me-vinyl | Me | Me | |
| 2-49 | Br | SO₂Me | Me | Me | |
| 2-50 | Br | SO₂Me | Et | Me | |
| 2-51 | Br | SO₂Me | CF₃ | Me | |
| 2-52 | Br | SO₂Me | OMe | Me | |
| 2-53 | Br | SO₂Me | Me | Et | |
| 2-54 | Br | SO₂Me | Me | Pr | |
| 2-55 | Br | SO₂Me | Me | c-Pr | |
| 2-56 | Br | SO₂Me | Me | CH₂-c-Pr | |
| 2-57 | Br | SO₂Me | Me | CH₂-CF₃ | |
| 2-58 | Br | Me | Me | Me | |
| 2-59 | Br | Et | Me | Me | |
| 2-60 | Br | CF₃ | Me | Me | |
| 2-61 | Br | CF₃ | Me | Et | |
| 2-62 | Br | CF₃ | Me | CH₂-c-Pr | |
| 2-63 | Br | CF₃ | Me | CH₂-CF₃ | |
| 2-64 | Me | SMe | Me | Me | |
| 2-65 | Me | S(O)Me | Me | Me | |
| 2-66 | Me | SO₂Me | Me | Me | * |
| 2-67 | Me | SO₂Me | Et | Me | |
| 2-68 | Me | SO₂Me | CF₃ | Me | |
| 2-69 | Me | SO₂Me | OMe | Me | |
| 2-70 | Me | SO₂Me | Me | Et | * |
| 2-71 | Me | SO₂Me | Me | Pr | |
| 2-72 | Me | SO₂Me | Me | c-Pr | |
| 2-73 | Me | SO₂Me | Me | CH₂-c-Pr | * |
| 2-74 | Me | SO₂Me | Me | CH₂-CF₃ | |
| 2-75 | Me | Me | Me | Me | |
| 2-76 | Me | Me | Et | Me | |
| 2-77 | Me | Me | CF₃ | Me | |
| 2-78 | Me | Me | OMe | Me | |
| 2-79 | Me | Me | Me | Et | |
| 2-80 | Me | Me | Me | Pr | |
| 2-81 | Me | Me | Me | c-Pr | |
| 2-82 | Me | Me | Me | CH₂-c-Pr | |
| 2-83 | Me | Me | Me | CH₂-CF₃ | |
| 2-84 | Me | Et | Me | Me | |
| 2-85 | Me | CF₃ | Me | Me | |
| 2-86 | Me | CF₃ | Et | Me | |
| 2-87 | Me | CF₃ | CF₃ | Me | |
| 2-88 | Me | CF₃ | OMe | Me | |
| 2-89 | Me | CF₃ | Me | Et | |
| 2-90 | Me | CF₃ | Me | Pr | |
| 2-91 | Me | CF₃ | Me | c-Pr | |
| 2-92 | Me | CF₃ | Me | CH₂-c-Pr | |
| 2-93 | Me | CF₃ | Me | CH₂-CF₃ | |
| 2-94 | Me | SO₂Et | Me | Me | |
| 2-95 | Me | SO₂Et | Me | Et | |
| 2-96 | Me | SO₂Et | Me | CH₂-c-Pr | |
| 2-97 | Me | SO₂Et | Me | CH₂-CF₃ | |
| 2-98 | Me | C₂F₅ | Me | Me | |
| 2-99 | Me | c-Pr | Me | Me | |
| 2-100 | OMe | Cl | Me | Me | |
| 2-101 | OMe | CF₃ | Me | Me | |
| 2-102 | OMe | CF₃ | Me | Et | |
| 2-103 | OMe | CF₃ | Me | CH₂-c-Pr | |
| 2-104 | OMe | CF₃ | Me | CH₂-CF₃ | |
| 2-105 | OMe | CHF₂ | Me | Me | |
| 2-106 | OMe | CHF₂ | Et | Me | |
| 2-107 | OMe | CHF₂ | CF₃ | Me | |
| 2-108 | OMe | CHF₂ | OMe | Me | |
| 2-109 | OMe | CHF₂ | Me | Et | |
| 2-110 | OMe | CHF₂ | Me | Pr | |
| 2-111 | OMe | CHF₂ | Me | c-Pr | |
| 2-112 | OMe | CHF₂ | Me | CH₂-c-Pr | |
| 2-113 | OMe | CHF₂ | Me | CH₂-CF₃ | |
| 2-114 | OMe | CF₂Cl | Me | Me | |
| 2-115 | OMe | SO₂Me | Me | Me | |
| 2-116 | SO₂Me | CF₃ | Me | Me | |
| 2-117 | SO₂Me | CF₃ | Et | Me | |
| 2-118 | SO₂Me | CF₃ | CF₃ | Me | |
| 2-119 | SO₂Me | CF₃ | OMe | Me | |
| 2-120 | SO₂Me | CF₃ | Me | Et | |
| 2-121 | SO₂Me | CF₃ | Me | Pr | |
| 2-122 | SO₂Me | CF₃ | Me | c-Pr | |
| 2-123 | SO₂Me | CF₃ | Me | CH₂-c-Pr | |
| 2-124 | CH₂OMe | CF₃ | Me | CH₂-CF₃ | |
| 2-125 | SMe | CF₃ | Me | Me | |
| 2-126 | SMe | CF₃ | Me | Et | |
| 2-127 | SMe | CF₃ | Me | CH₂-c-Pr | |
| 2-128 | SMe | CF₃ | Me | CH₂-CF₃ | |
| 2-129 | SMe | SO₂Me | Me | Me | |
| 2-130 | CF₃ | CF₃ | Me | Me | |
| 2-131 | CF₃ | CF₃ | Me | Et | |
| 2-132 | CF₃ | CF₃ | Me | CH₂-c-Pr | |
| 2-133 | CF₃ | CF₃ | Me | CH₂-CF₃ | |
| 2-134 | F | CF₃ | Me | Me | |
| 2-135 | Me | SO₂Me | Me | i-Bu | * |
| 2-136 | | | | | |

**Tabelle 3: Erfindungsgemäße Verbindungen der allgemeinen Formel (I), worin Q für Q1, R^{x} für eine Propylgruppe und R und W jeweils für Wasserstoff stehen (E-Isomer, ggf. Z-Isomer),**

| | | | | | |
|---|---|---|---|---|---|
| 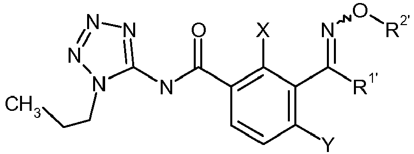 | | | | | |

| **Nr.** | **X** | **Y** | **R^{1'}** | **R^{2'}** | **Physikalische Daten** (¹H-NMR) |
|---|---|---|---|---|---|
| 3-1 | Cl | Cl | Me | Me | * |
| 3-2 | Cl | Cl | Et | Me | |
| 3-3 | Cl | Cl | CF₃ | Me | |
| 3-4 | Cl | Cl | OMe | Me | |
| 3-5 | Cl | Cl | Me | Et | * |
| 3-6 | Cl | Cl | Me | Pr | |
| 3-7 | Cl | Cl | Me | c-Pr | |
| 3-8 | Cl | Cl | Me | CH₂-c-Pr | * |
| 3-9 | Cl | Cl | Me | CH₂-CF₃ | * |
| 3-10 | Cl | SMe | Me | Me | |
| 3-11 | Cl | SMe | Me | Et | |
| 3-12 | Cl | SMe | Me | CH₂-c-Pr | |
| 3-13 | Cl | SMe | Me | CH₂-CF₃ | |
| 3-14 | Cl | S(O)Me | Me | Me | |
| 3-15 | Cl | SO₂Me | Me | Me | * |
| 3-16 | Cl | SO₂Me | Et | Me | |
| 3-17 | Cl | SO₂Me | CF₃ | Me | |
| 3-18 | Cl | SO₂Me | OMe | Me | |
| 3-19 | Cl | SO₂Me | Me | Et | * |
| 3-20 | Cl | SO₂Me | Me | Pr | |
| 3-21 | Cl | SO₂Me | Me | c-Pr | |
| 3-22 | Cl | SO₂Me | Me | CH₂-c-Pr | * |
| 3-23 | Cl | SO₂Me | Me | CH₂-CF₃ | |
| 3-24 | Cl | Me | Me | Me | |
| 3-25 | Cl | Me | Me | Et | |
| 3-26 | Cl | Me | Me | CH₂-c-Pr | |
| 3-27 | Cl | Me | Me | CH₂-CF₃ | |
| 3-28 | Cl | Et | Me | Me | |
| 3-29 | Cl | c-Pr | Me | Me | |
| 3-30 | Cl | CF₃ | Me | Me | |
| 3-31 | Cl | CF₃ | Et | Me | |
| 3-32 | Cl | CF₃ | CF₃ | Me | |
| 3-33 | Cl | CF₃ | OMe | Me | |
| 3-34 | Cl | CF₃ | Me | Et | |
| 3-35 | Cl | CF₃ | Me | Pr | |
| 3-36 | Cl | CF₃ | Me | c-Pr | |
| 3-37 | Cl | CF₃ | Me | CH₂-c-Pr | |
| 3-38 | Cl | CF₃ | Me | CH₂-CF₃ | |
| 3-39 | Cl | OMe | Me | Me | |
| 3-40 | Cl | OMe | Me | Et | |
| 3-41 | Cl | OMe | Me | CH₂-c-Pr | |
| 3-42 | Cl | OMe | Me | CH₂-CF₃ | |
| 3-43 | Cl | SO₂Et | Me | Me | |
| 3-44 | Cl | SO₂Et | Me | Et | |
| 3-45 | Cl | SO₂Et | Me | CH₂-c-Pr | |
| 3-46 | Cl | SO₂Et | Me | CH₂-CF₃ | |
| 3-47 | Cl | C₂F₅ | Me | Me | |
| 3-48 | Cl | Vinyl | Me | Me | |
| 3-49 | Cl | 1-Me-vinyl | Me | Me | |
| 3-50 | Br | SO₂Me | Me | Me | |
| 3-51 | Br | SO₂Me | Et | Me | |
| 3-52 | Br | SO₂Me | CF₃ | Me | |
| 3-53 | Br | SO₂Me | OMe | Me | |
| 3-54 | Br | SO₂Me | Me | Et | |
| 3-55 | Br | SO₂Me | Me | Pr | |
| 3-56 | Br | SO₂Me | Me | c-Pr | |
| 3-57 | Br | SO₂Me | Me | CH₂-c-Pr | |
| 3-58 | Br | SO₂Me | Me | CH₂-CF₃ | |
| 3-59 | Br | Me | Me | Me | |
| 3-60 | Br | Et | Me | Me | |
| 3-61 | Br | CF₃ | Me | Me | |
| 3-62 | Br | CF₃ | Me | Et | |
| 3-63 | Br | CF₃ | Me | CH₂-c-Pr | |
| 3-64 | Br | CF₃ | Me | CH₂-CF₃ | |
| 3-65 | Me | SMe | Me | Me | |
| 3-66 | Me | S(O)Me | Me | Me | |
| 3-67 | Me | SO₂Me | Me | Me | |
| 3-68 | Me | SO₂Me | Et | Me | |
| 3-69 | Me | SO₂Me | CF₃ | Me | |
| 3-70 | Me | SO₂Me | OMe | Me | |
| 3-71 | Me | SO₂Me | Me | Et | |
| 3-72 | Me | SO₂Me | Me | Pr | |
| 3-73 | Me | SO₂Me | Me | c-Pr | |
| 3-74 | Me | SO₂Me | Me | CH₂-c-Pr | |
| 3-75 | Me | SO₂Me | Me | CH₂-CF₃ | |
| 3-76 | Me | Me | Me | Me | |
| 3-77 | Me | Me | Et | Me | |
| 3-78 | Me | Me | CF₃ | Me | |
| 3-79 | Me | Me | OMe | Me | |
| 3-80 | Me | Me | Me | Et | |
| 3-81 | Me | Me | Me | Pr | |
| 3-82 | Me | Me | Me | c-Pr | |
| 3-83 | Me | Me | Me | CH₂-c-Pr | |
| 3-84 | Me | Me | Me | CH₂-CF₃ | |
| 3-85 | Me | Et | Me | Me | |
| 3-86 | Me | CF₃ | Me | Me | |
| 3-87 | Me | CF₃ | Et | Me | |
| 3-88 | Me | CF₃ | CF₃ | Me | |
| 3-89 | Me | CF₃ | OMe | Me | |
| 3-90 | Me | CF₃ | Me | Et | |
| 3-91 | Me | CF₃ | Me | Pr | |
| 3-92 | Me | CF₃ | Me | c-Pr | |
| 3-93 | Me | CF₃ | Me | CH₂-c-Pr | |
| 3-94 | Me | CF₃ | Me | CH₂-CF₃ | |
| 3-95 | Me | SO₂Et | Me | Me | |
| 3-96 | Me | SO₂Et | Me | Et | |
| 3-97 | Me | SO₂Et | Me | CH₂-c-Pr | |
| 3-98 | Me | SO₂Et | Me | CH₂-CF₃ | |
| 3-99 | Me | C₂F₅ | Me | Me | |
| 3-100 | Me | c-Pr | Me | Me | |
| 3-101 | OMe | Cl | Me | Me | |
| 3-102 | OMe | CF₃ | Me | Me | |
| 3-103 | OMe | CF₃ | Me | Et | |
| 3-104 | OMe | CF₃ | Me | CH₂-c-Pr | |
| 3-105 | OMe | CF₃ | Me | CH₂-CF₃ | |
| 3-106 | OMe | CHF₂ | Me | Me | |
| 3-107 | OMe | CHF₂ | Et | Me | |
| 3-108 | OMe | CHF₂ | CF₃ | Me | |
| 3-109 | OMe | CHF₂ | OMe | Me | |
| 3-110 | OMe | CHF₂ | Me | Et | |
| 3-111 | OMe | CHF₂ | Me | Pr | |
| 3-112 | OMe | CHF₂ | Me | c-Pr | |
| 3-113 | OMe | CHF₂ | Me | CH₂-c-Pr | |
| 3-114 | OMe | CHF₂ | Me | CH₂-CF₃ | |
| 3-115 | OMe | CF₂Cl | Me | Me | |
| 3-116 | OMe | SO₂Me | Me | Me | |
| 3-117 | SO₂Me | CF₃ | Me | Me | |
| 3-118 | SO₂Me | CF₃ | Et | Me | |
| 3-119 | SO₂Me | CF₃ | CF₃ | Me | |
| 3-120 | SO₂Me | CF₃ | OMe | Me | |
| 3-121 | SO₂Me | CF₃ | Me | Et | |
| 3-122 | SO₂Me | CF₃ | Me | Pr | |
| 3-123 | SO₂Me | CF₃ | Me | c-Pr | |
| 3-124 | SO₂Me | CF₃ | Me | CH₂-c-Pr | |
| 3-125 | CH₂OMe | CF₃ | Me | CH₂-CF₃ | |
| 3-126 | SMe | CF₃ | Me | Me | |
| 3-127 | SMe | CF₃ | Me | Et | |
| 3-128 | SMe | CF₃ | Me | CH₂-c-Pr | |
| 3-129 | SMe | CF₃ | Me | CH₂-CF₃ | |
| 3-130 | SMe | SO₂Me | Me | Me | |
| 3-131 | CF₃ | CF₃ | Me | Me | |
| 3-132 | CF₃ | CF₃ | Me | Et | |
| 3-133 | CF₃ | CF₃ | Me | CH₂-c-Pr | |
| 3-134 | CF₃ | CF₃ | Me | CH₂-CF₃ | |
| 3-135 | F | CF₃ | Me | Me | |
| 3-136 | Me | SO₂Me | Me | i-Bu | |

**Tabelle 4: Erfindungsgemäße Verbindungen der allgemeinen Formel (I), worin Q für Q2, R^{x} für eine Methylgruppe und R und W jeweils für Wasserstoff stehen (E-Isomer, ggf. Z-Isomer),**

| | | | | | |
|---|---|---|---|---|---|
| 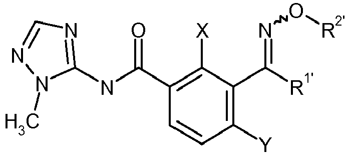 | | | | | |

| **Nr.** | **X** | **Y** | **R^{1'}** | **R^{2'}** | **Physikalische Daten** (¹H-NMR) |
|---|---|---|---|---|---|
| 4-1 | Cl | Cl | Me | Me | * |
| 4-2 | Cl | Cl | Et | Me | |
| 4-3 | Cl | Cl | CF₃ | Me | |
| 4-4 | Cl | Cl | OMe | Me | |
| 4-5 | Cl | Cl | Me | Et | * |
| 4-6 | Cl | Cl | Me | Pr | |
| 4-7 | Cl | Cl | Me | c-Pr | |
| 4-8 | Cl | Cl | Me | CH₂-c-Pr | * |
| 4-9 | Cl | Cl | Me | CH₂-CF₃ | * |
| 4-10 | Cl | SMe | Me | Me | |
| 4-11 | Cl | SMe | Me | Et | |
| 4-12 | Cl | SMe | Me | CH₂-c-Pr | |
| 4-13 | Cl | SMe | Me | CH₂-CF₃ | |
| 4-14 | Cl | S(O)Me | Me | Me | |
| 4-15 | Cl | SO₂Me | Me | Me | * |
| 4-16 | Cl | SO₂Me | Et | Me | |
| 4-17 | Cl | SO₂Me | CF₃ | Me | |
| 4-18 | Cl | SO₂Me | OMe | Me | |
| 4-19 | Cl | SO₂Me | Me | Et | * |
| 4-20 | Cl | SO₂Me | Me | Pr | |
| 4-21 | Cl | SO₂Me | Me | c-Pr | |
| 4-22 | Cl | SO₂Me | Me | CH₂-c-Pr | * |
| 4-23 | Cl | SO₂Me | Me | CH₂-CF₃ | |
| 4-24 | Cl | Me | Me | Me | |
| 4-25 | Cl | Me | Me | Et | |
| 4-26 | Cl | Me | Me | CH₂-c-Pr | |
| 4-27 | Cl | Me | Me | CH₂-CF₃ | |
| 4-28 | Cl | Et | Me | Me | |
| 4-29 | Cl | c-Pr | Me | Me | |
| 4-30 | Cl | CF₃ | Me | Me | |
| 4-31 | Cl | CF₃ | Et | Me | |
| 4-32 | Cl | CF₃ | CF₃ | Me | |
| 4-33 | Cl | CF₃ | OMe | Me | |
| 4-34 | Cl | CF₃ | Me | Et | |
| 4-35 | Cl | CF₃ | Me | Pr | |
| 4-36 | Cl | CF₃ | Me | c-Pr | |
| 4-37 | Cl | CF₃ | Me | CH₂-c-Pr | |
| 4-38 | Cl | CF₃ | Me | CH₂-CF₃ | |
| 4-39 | Cl | OMe | Me | Me | |
| 4-40 | Cl | OMe | Me | Et | |
| 4-41 | Cl | OMe | Me | CH₂-c-Pr | |
| 4-42 | Cl | OMe | Me | CH₂-CF₃ | |
| 4-43 | Cl | SO₂Et | Me | Me | |
| 4-44 | Cl | SO₂Et | Me | Et | |
| 4-45 | Cl | SO₂Et | Me | CH₂-c-Pr | |
| 4-46 | Cl | SO₂Et | Me | CH₂-CF₃ | |
| 4-47 | Cl | C₂F₅ | Me | Me | |
| 4-48 | Cl | Vinyl | Me | Me | |
| 4-49 | Cl | 1-Me-vinyl | Me | Me | |
| 4-50 | Br | SO₂Me | Me | Me | |
| 4-51 | Br | SO₂Me | Et | Me | |
| 4-52 | Br | SO₂Me | CF₃ | Me | |
| 4-53 | Br | SO₂Me | OMe | Me | |
| 4-54 | Br | SO₂Me | Me | Et | |
| 4-55 | Br | SO₂Me | Me | Pr | |
| 4-56 | Br | SO₂Me | Me | c-Pr | |
| 4-57 | Br | SO₂Me | Me | CH₂-c-Pr | |
| 4-58 | Br | SO₂Me | Me | CH₂-CF₃ | |
| 4-59 | Br | Me | Me | Me | |
| 4-60 | Br | Et | Me | Me | |
| 4-61 | Br | CF₃ | Me | Me | |
| 4-62 | Br | CF₃ | Me | Et | |
| 4-63 | Br | CF₃ | Me | CH₂-c-Pr | |
| 4-64 | Br | CF₃ | Me | CH₂-CF₃ | |
| 4-65 | Me | SMe | Me | Me | |
| 4-66 | Me | S(O)Me | Me | Me | |
| 4-67 | Me | SO₂Me | Me | Me | |
| 4-68 | Me | SO₂Me | Et | Me | |
| 4-69 | Me | SO₂Me | CF₃ | Me | |
| 4-70 | Me | SO₂Me | OMe | Me | |
| 4-71 | Me | SO₂Me | Me | Et | |
| 4-72 | Me | SO₂Me | Me | Pr | |
| 4-73 | Me | SO₂Me | Me | c-Pr | |
| 4-74 | Me | SO₂Me | Me | CH₂-c-Pr | * |
| 4-75 | Me | SO₂Me | Me | CH₂-CF₃ | |
| 4-76 | Me | Me | Me | Me | |
| 4-77 | Me | Me | Et | Me | |
| 4-78 | Me | Me | CF₃ | Me | |
| 4-79 | Me | Me | OMe | Me | |
| 4-80 | Me | Me | Me | Et | |
| 4-81 | Me | Me | Me | Pr | |
| 4-82 | Me | Me | Me | c-Pr | |
| 4-83 | Me | Me | Me | CH₂-c-Pr | |
| 4-84 | Me | Me | Me | CH₂-CF₃ | |
| 4-85 | Me | Et | Me | Me | |
| 4-86 | Me | CF₃ | Me | Me | |
| 4-87 | Me | CF₃ | Et | Me | |
| 4-88 | Me | CF₃ | CF₃ | Me | |
| 4-89 | Me | CF₃ | OMe | Me | |
| 4-90 | Me | CF₃ | Me | Et | |
| 4-91 | Me | CF₃ | Me | Pr | |
| 4-92 | Me | CF₃ | Me | c-Pr | |
| 4-93 | Me | CF₃ | Me | CH₂-c-Pr | |
| 4-94 | Me | CF₃ | Me | CH₂-CF₃ | |
| 4-95 | Me | SO₂Et | Me | Me | |
| 4-96 | Me | SO₂Et | Me | Et | |
| 4-97 | Me | SO₂Et | Me | CH₂-c-Pr | |
| 4-98 | Me | SO₂Et | Me | CH₂-CF₃ | |
| 4-99 | Me | C₂F₅ | Me | Me | |
| 4-100 | Me | c-Pr | Me | Me | |
| 4-101 | OMe | Cl | Me | Me | |
| 4-102 | OMe | CF₃ | Me | Me | |
| 4-103 | OMe | CF₃ | Me | Et | |
| 4-104 | OMe | CF₃ | Me | CH₂-c-Pr | |
| 4-105 | OMe | CF₃ | Me | CH₂-CF₃ | |
| 4-106 | OMe | CHF₂ | Me | Me | |
| 4-107 | OMe | CHF₂ | Et | Me | |
| 4-108 | OMe | CHF₂ | CF₃ | Me | |
| 4-109 | OMe | CHF₂ | OMe | Me | |
| 4-110 | OMe | CHF₂ | Me | Et | |
| 4-111 | OMe | CHF₂ | Me | Pr | |
| 4-112 | OMe | CHF₂ | Me | c-Pr | |
| 4-113 | OMe | CHF₂ | Me | CH₂-c-Pr | |
| 4-114 | OMe | CHF₂ | Me | CH₂-CF₃ | |
| 4-115 | OMe | CF₂Cl | Me | Me | |
| 4-116 | OMe | SO₂Me | Me | Me | |
| 4-117 | SO₂Me | CF₃ | Me | Me | |
| 4-118 | SO₂Me | CF₃ | Et | Me | |
| 4-119 | SO₂Me | CF₃ | CF₃ | Me | |
| 4-120 | SO₂Me | CF₃ | OMe | Me | |
| 4-121 | SO₂Me | CF₃ | Me | Et | |
| 4-122 | SO₂Me | CF₃ | Me | Pr | |
| 4-123 | SO₂Me | CF₃ | Me | c-Pr | |
| 4-124 | SO₂Me | CF₃ | Me | CH₂-c-Pr | |
| 4-125 | CH₂OMe | CF₃ | Me | CH₂-CF₃ | |
| 4-126 | SMe | CF₃ | Me | Me | |
| 4-127 | SMe | CF₃ | Me | Et | |
| 4-128 | SMe | CF₃ | Me | CH₂-c-Pr | |
| 4-129 | SMe | CF₃ | Me | CH₂-CF₃ | |
| 4-130 | SMe | SO₂Me | Me | Me | |
| 4-131 | CF₃ | CF₃ | Me | Me | |
| 4-132 | CF₃ | CF₃ | Me | Et | |
| 4-133 | CF₃ | CF₃ | Me | CH₂-c-Pr | |
| 4-134 | CF₃ | CF₃ | Me | CH₂-CF₃ | |
| 4-135 | F | CF₃ | Me | Me | |
| 4-136 | Me | SO₂Me | Me | i-Bu | * |

**Tabelle 5: Erfindungsgemäße Verbindungen der allgemeinen Formel (I), worin Q für Q3, R^{y} für Chlor und R und W jeweils für Wasserstoff stehen (E-Isomer, ggf. Z-Isomer),**

| | | | | | |
|---|---|---|---|---|---|
| 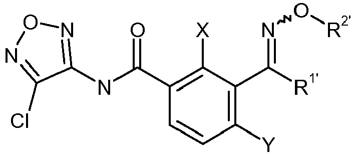 | | | | | |

| **Nr.** | **X** | **Y** | **R^{1'}** | **R^{2'}** | **Physikalische Daten** (¹H-NMR) |
|---|---|---|---|---|---|
| 5-1 | Cl | Cl | Me | Me | * |
| 5-2 | Cl | Cl | Et | Me | |
| 5-3 | Cl | Cl | CF₃ | Me | |
| 5-4 | Cl | Cl | OMe | Me | |
| 5-5 | Cl | Cl | Me | Et | * |
| 5-6 | Cl | Cl | Me | Pr | |
| 5-7 | Cl | Cl | Me | c-Pr | |
| 5-8 | Cl | Cl | Me | CH₂-c-Pr | * |
| 5-9 | Cl | Cl | Me | CH₂-CF₃ | * |
| 5-10 | Cl | SMe | Me | Me | |
| 5-11 | Cl | SMe | Me | Et | |
| 5-12 | Cl | SMe | Me | CH₂-c-Pr | |
| 5-13 | Cl | SMe | Me | CH₂-CF₃ | |
| 5-14 | Cl | S(O)Me | Me | Me | |
| 5-15 | Cl | SO₂Me | Me | Me | * |
| 5-16 | Cl | SO₂Me | Et | Me | |
| 5-17 | Cl | SO₂Me | CF₃ | Me | |
| 5-18 | Cl | SO₂Me | OMe | Me | |
| 5-19 | Cl | SO₂Me | Me | Et | |
| 5-20 | Cl | SO₂Me | Me | Pr | |
| 5-21 | Cl | SO₂Me | Me | c-Pr | |
| 5-22 | Cl | SO₂Me | Me | CH₂-c-Pr | * |
| 5-23 | Cl | SO₂Me | Me | CH₂-CF₃ | |
| 5-24 | Cl | Me | Me | Me | |
| 5-25 | Cl | Me | Me | Et | |
| 5-26 | Cl | Me | Me | CH₂-c-Pr | |
| 5-27 | Cl | Me | Me | CH₂-CF₃ | |
| 5-28 | Cl | Et | Me | Me | |
| 5-29 | Cl | c-Pr | Me | Me | |
| 5-30 | Cl | CF₃ | Me | Me | |
| 5-31 | Cl | CF₃ | Et | Me | |
| 5-32 | Cl | CF₃ | CF₃ | Me | |
| 5-33 | Cl | CF₃ | OMe | Me | |
| 5-34 | Cl | CF₃ | Me | Et | |
| 5-35 | Cl | CF₃ | Me | Pr | |
| 5-36 | Cl | CF₃ | Me | c-Pr | |
| 5-37 | Cl | CF₃ | Me | CH₂-c-Pr | |
| 5-38 | Cl | CF₃ | Me | CH₂-CF₃ | |
| 5-39 | Cl | OMe | Me | Me | |
| 5-40 | Cl | OMe | Me | Et | |
| 5-41 | Cl | OMe | Me | CH₂-c-Pr | |
| 5-42 | Cl | OMe | Me | CH₂-CF₃ | |
| 5-43 | Cl | SO₂Et | Me | Me | |
| 5-44 | Cl | SO₂Et | Me | Et | |
| 5-45 | Cl | SO₂Et | Me | CH₂-c-Pr | |
| 5-46 | Cl | SO₂Et | Me | CH₂-CF₃ | |
| 5-47 | Cl | C₂F₅ | Me | Me | |
| 5-48 | Cl | Vinyl | Me | Me | |
| 5-49 | Cl | 1-Me-vinyl | Me | Me | |
| 5-50 | Br | SO₂Me | Me | Me | |
| 5-51 | Br | SO₂Me | Et | Me | |
| 5-52 | Br | SO₂Me | CF₃ | Me | |
| 5-53 | Br | SO₂Me | OMe | Me | |
| 5-54 | Br | SO₂Me | Me | Et | |
| 5-55 | Br | SO₂Me | Me | Pr | |
| 5-56 | Br | SO₂Me | Me | c-Pr | |
| 5-57 | Br | SO₂Me | Me | CH₂-c-Pr | |
| 5-58 | Br | SO₂Me | Me | CH₂-CF₃ | |
| 5-59 | Br | Me | Me | Me | |
| 5-60 | Br | Et | Me | Me | |
| 5-61 | Br | CF₃ | Me | Me | |
| 5-62 | Br | CF₃ | Me | Et | |
| 5-63 | Br | CF₃ | Me | CH₂-c-Pr | |
| 5-64 | Br | CF₃ | Me | CH₂-CF₃ | |
| 5-65 | Me | SMe | Me | Me | |
| 5-66 | Me | S(O)Me | Me | Me | |
| 5-67 | Me | SO₂Me | Me | Me | * |
| 5-68 | Me | SO₂Me | Et | Me | |
| 5-69 | Me | SO₂Me | CF₃ | Me | |
| 5-70 | Me | SO₂Me | OMe | Me | |
| 5-71 | Me | SO₂Me | Me | Et | * |
| 5-72 | Me | SO₂Me | Me | Pr | |
| 5-73 | Me | SO₂Me | Me | c-Pr | |
| 5-74 | Me | SO₂Me | Me | CH₂-c-Pr | * |
| 5-75 | Me | SO₂Me | Me | CH₂-CF₃ | |
| 5-76 | Me | Me | Me | Me | |
| 5-77 | Me | Me | Et | Me | |
| 5-78 | Me | Me | CF₃ | Me | |
| 5-79 | Me | Me | OMe | Me | |
| 5-80 | Me | Me | Me | Et | |
| 5-81 | Me | Me | Me | Pr | |
| 5-82 | Me | Me | Me | c-Pr | |
| 5-83 | Me | Me | Me | CH₂-c-Pr | |
| 5-84 | Me | Me | Me | CH₂-CF₃ | |
| 5-85 | Me | Et | Me | Me | |
| 5-86 | Me | CF₃ | Me | Me | |
| 5-87 | Me | CF₃ | Et | Me | |
| 5-88 | Me | CF₃ | CF₃ | Me | |
| 5-89 | Me | CF₃ | OMe | Me | |
| 5-90 | Me | CF₃ | Me | Et | |
| 5-91 | Me | CF₃ | Me | Pr | |
| 5-92 | Me | CF₃ | Me | c-Pr | |
| 5-93 | Me | CF₃ | Me | CH₂-c-Pr | |
| 5-94 | Me | CF₃ | Me | CH₂-CF₃ | |
| 5-95 | Me | SO₂Et | Me | Me | |
| 5-96 | Me | SO₂Et | Me | Et | |
| 5-97 | Me | SO₂Et | Me | CH₂-c-Pr | |
| 5-98 | Me | SO₂Et | Me | CH₂-CF₃ | |
| 5-99 | Me | C₂F₅ | Me | Me | |
| 5-100 | Me | c-Pr | Me | Me | |
| 5-101 | OMe | Cl | Me | Me | |
| 5-102 | OMe | CF₃ | Me | Me | |
| 5-103 | OMe | CF₃ | Me | Et | |
| 5-104 | OMe | CF₃ | Me | CH₂-c-Pr | |
| 5-105 | OMe | CF₃ | Me | CH₂-CF₃ | |
| 5-106 | OMe | CHF₂ | Me | Me | |
| 5-107 | OMe | CHF₂ | Et | Me | |
| 5-108 | OMe | CHF₂ | CF₃ | Me | |
| 5-109 | OMe | CHF₂ | OMe | Me | |
| 5-110 | OMe | CHF₂ | Me | Et | |
| 5-111 | OMe | CHF₂ | Me | Pr | |
| 5-112 | OMe | CHF₂ | Me | c-Pr | |
| 5-113 | OMe | CHF₂ | Me | CH₂-c-Pr | |
| 5-114 | OMe | CHF₂ | Me | CH₂-CF₃ | |
| 5-115 | OMe | CF₂Cl | Me | Me | |
| 5-116 | OMe | SO₂Me | Me | Me | |
| 5-117 | SO₂Me | CF₃ | Me | Me | |
| 5-118 | SO₂Me | CF₃ | Et | Me | |
| 5-119 | SO₂Me | CF₃ | CF₃ | Me | |
| 5-120 | SO₂Me | CF₃ | OMe | Me | |
| 5-121 | SO₂Me | CF₃ | Me | Et | |
| 5-122 | SO₂Me | CF₃ | Me | Pr | |
| 5-123 | SO₂Me | CF₃ | Me | c-Pr | |
| 5-124 | SO₂Me | CF₃ | Me | CH₂-c-Pr | |
| 5-125 | CH₂OMe | CF₃ | Me | CH₂-CF₃ | |
| 5-126 | SMe | CF₃ | Me | Me | |
| 5-127 | SMe | CF₃ | Me | Et | |
| 5-128 | SMe | CF₃ | Me | CH₂-c-Pr | |
| 5-129 | SMe | CF₃ | Me | CH₂-CF₃ | |
| 5-130 | SMe | SO₂Me | Me | Me | |
| 5-131 | CF₃ | CF₃ | Me | Me | |
| 5-132 | CF₃ | CF₃ | Me | Et | |
| 5-133 | CF₃ | CF₃ | Me | CH₂-c-Pr | |
| 5-134 | CF₃ | CF₃ | Me | CH₂-CF₃ | |
| 5-135 | F | CF₃ | Me | Me | |
| 5-136 | Me | SO₂Me | Me | i-Bu | |

**Tabelle 6: Erfindungsgemäße Verbindungen der allgemeinen Formel (I), worin Q für Q3, R^{y} für eine Methylgruppe und R und W jeweils für Wasserstoff stehen (E-Isomer, ggf. Z-Isomer),**

| | | | | | |
|---|---|---|---|---|---|
| 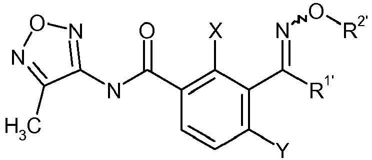 | | | | | |

| **Nr.** | **X** | **Y** | **R^{1'}** | **R^{2'}** | **Physikalische Daten** (¹H-NMR) |
|---|---|---|---|---|---|
| 6-1 | Cl | Cl | Me | Me | * |
| 6-2 | Cl | Cl | Et | Me | |
| 6-3 | Cl | Cl | CF₃ | Me | |
| 6-4 | Cl | Cl | OMe | Me | |
| 6-5 | Cl | Cl | Me | Et | * |
| 6-6 | Cl | Cl | Me | Pr | |
| 6-7 | Cl | Cl | Me | c-Pr | |
| 6-8 | Cl | Cl | Me | CH₂-c-Pr | * |
| 6-9 | Cl | Cl | Me | CH₂-CF₃ | * |
| 6-10 | Cl | SMe | Me | Me | |
| 6-11 | Cl | SMe | Me | Et | |
| 6-12 | Cl | SMe | Me | CH₂-c-Pr | |
| 6-13 | Cl | SMe | Me | CH₂-CF₃ | |
| 6-14 | Cl | S(O)Me | Me | Me | |
| 6-15 | Cl | SO₂Me | Me | Me | * |
| 6-16 | Cl | SO₂Me | Et | Me | |
| 6-17 | Cl | SO₂Me | CF₃ | Me | |
| 6-18 | Cl | SO₂Me | OMe | Me | |
| 6-19 | Cl | SO₂Me | Me | Et | * |
| 6-20 | Cl | SO₂Me | Me | Pr | |
| 6-21 | Cl | SO₂Me | Me | c-Pr | |
| 6-22 | Cl | SO₂Me | Me | CH₂-c-Pr | * |
| 6-23 | Cl | SO₂Me | Me | CH₂-CF₃ | |
| 6-24 | Cl | Me | Me | Me | |
| 6-25 | Cl | Me | Me | Et | |
| 6-26 | Cl | Me | Me | CH₂-c-Pr | |
| 6-27 | Cl | Me | Me | CH₂-CF₃ | |
| 6-28 | Cl | Et | Me | Me | |
| 6-29 | Cl | c-Pr | Me | Me | |
| 6-30 | Cl | CF₃ | Me | Me | |
| 6-31 | Cl | CF₃ | Et | Me | |
| 6-32 | Cl | CF₃ | CF₃ | Me | |
| 6-33 | Cl | CF₃ | OMe | Me | |
| 6-34 | Cl | CF₃ | Me | Et | |
| 6-35 | Cl | CF₃ | Me | Pr | |
| 6-36 | Cl | CF₃ | Me | c-Pr | |
| 6-37 | Cl | CF₃ | Me | CH₂-c-Pr | |
| 6-38 | Cl | CF₃ | Me | CH₂-CF₃ | |
| 6-39 | Cl | OMe | Me | Me | |
| 6-40 | Cl | OMe | Me | Et | |
| 6-41 | Cl | OMe | Me | CH₂-c-Pr | |
| 6-42 | Cl | OMe | Me | CH₂-CF₃ | |
| 6-43 | Cl | SO₂Et | Me | Me | |
| 6-44 | Cl | SO₂Et | Me | Et | |
| 6-45 | Cl | SO₂Et | Me | CH₂-c-Pr | |
| 6-46 | Cl | SO₂Et | Me | CH₂-CF₃ | |
| 6-47 | Cl | C₂F₅ | Me | Me | |
| 6-48 | Cl | Vinyl | Me | Me | |
| 6-49 | Cl | 1-Me-vinyl | Me | Me | |
| 6-50 | Br | SO₂Me | Me | Me | |
| 6-51 | Br | SO₂Me | Et | Me | |
| 6-52 | Br | SO₂Me | CF₃ | Me | |
| 6-53 | Br | SO₂Me | OMe | Me | |
| 6-54 | Br | SO₂Me | Me | Et | |
| 6-55 | Br | SO₂Me | Me | Pr | |
| 6-56 | Br | SO₂Me | Me | c-Pr | |
| 6-57 | Br | SO₂Me | Me | CH₂-c-Pr | |
| 6-58 | Br | SO₂Me | Me | CH₂-CF₃ | |
| 6-59 | Br | Me | Me | Me | |
| 6-60 | Br | Et | Me | Me | |
| 6-61 | Br | CF₃ | Me | Me | |
| 6-62 | Br | CF₃ | Me | Et | |
| 6-63 | Br | CF₃ | Me | CH₂-c-Pr | |
| 6-64 | Br | CF₃ | Me | CH₂-CF₃ | |
| 6-65 | Me | SMe | Me | Me | |
| 6-66 | Me | S(O)Me | Me | Me | |
| 6-67 | Me | SO₂Me | Me | Me | * |
| 6-68 | Me | SO₂Me | Et | Me | |
| 6-69 | Me | SO₂Me | CF₃ | Me | |
| 6-70 | Me | SO₂Me | OMe | Me | |
| 6-71 | Me | SO₂Me | Me | Et | * |
| 6-72 | Me | SO₂Me | Me | Pr | |
| 6-73 | Me | SO₂Me | Me | c-Pr | |
| 6-74 | Me | SO₂Me | Me | CH₂-c-Pr | * |
| 6-75 | Me | SO₂Me | Me | CH₂-CF₃ | |
| 6-76 | Me | Me | Me | Me | |
| 6-77 | Me | Me | Et | Me | |
| 6-78 | Me | Me | CF₃ | Me | |
| 6-79 | Me | Me | OMe | Me | |
| 6-80 | Me | Me | Me | Et | |
| 6-81 | Me | Me | Me | Pr | |
| 6-82 | Me | Me | Me | c-Pr | |
| 6-83 | Me | Me | Me | CH₂-c-Pr | |
| 6-84 | Me | Me | Me | CH₂-CF₃ | |
| 6-85 | Me | Et | Me | Me | |
| 6-86 | Me | CF₃ | Me | Me | |
| 6-87 | Me | CF₃ | Et | Me | |
| 6-88 | Me | CF₃ | CF₃ | Me | |
| 6-89 | Me | CF₃ | OMe | Me | |
| 6-90 | Me | CF₃ | Me | Et | |
| 6-91 | Me | CF₃ | Me | Pr | |
| 6-92 | Me | CF₃ | Me | c-Pr | |
| 6-93 | Me | CF₃ | Me | CH₂-c-Pr | |
| 6-94 | Me | CF₃ | Me | CH₂-CF₃ | |
| 6-95 | Me | SO₂Et | Me | Me | |
| 6-96 | Me | SO₂Et | Me | Et | |
| 6-97 | Me | SO₂Et | Me | CH₂-c-Pr | |
| 6-98 | Me | SO₂Et | Me | CH₂-CF₃ | |
| 6-99 | Me | C₂F₅ | Me | Me | |
| 6-100 | Me | c-Pr | Me | Me | |
| 6-101 | OMe | Cl | Me | Me | |
| 6-102 | OMe | CF₃ | Me | Me | |
| 6-103 | OMe | CF₃ | Me | Et | |
| 6-104 | OMe | CF₃ | Me | CH₂-c-Pr | |
| 6-105 | OMe | CF₃ | Me | CH₂-CF₃ | |
| 6-106 | OMe | CHF₂ | Me | Me | |
| 6-107 | OMe | CHF₂ | Et | Me | |
| 6-108 | OMe | CHF₂ | CF₃ | Me | |
| 6-109 | OMe | CHF₂ | OMe | Me | |
| 6-110 | OMe | CHF₂ | Me | Et | |
| 6-111 | OMe | CHF₂ | Me | Pr | |
| 6-112 | OMe | CHF₂ | Me | c-Pr | |
| 6-113 | OMe | CHF₂ | Me | CH₂-c-Pr | |
| 6-114 | OMe | CHF₂ | Me | CH₂-CF₃ | |
| 6-115 | OMe | CF₂Cl | Me | Me | |
| 6-116 | OMe | SO₂Me | Me | Me | |
| 6-117 | SO₂Me | CF₃ | Me | Me | |
| 6-118 | SO₂Me | CF₃ | Et | Me | |
| 6-119 | SO₂Me | CF₃ | CF₃ | Me | |
| 6-120 | SO₂Me | CF₃ | OMe | Me | |
| 6-121 | SO₂Me | CF₃ | Me | Et | |
| 6-122 | SO₂Me | CF₃ | Me | Pr | |
| 6-123 | SO₂Me | CF₃ | Me | c-Pr | |
| 6-124 | SO₂Me | CF₃ | Me | CH₂-c-Pr | |
| 6-125 | CH₂OMe | CF₃ | Me | CH₂-CF₃ | |
| 6-126 | SMe | CF₃ | Me | Me | |
| 6-127 | SMe | CF₃ | Me | Et | |
| 6-128 | SMe | CF₃ | Me | CH₂-c-Pr | |
| 6-129 | SMe | CF₃ | Me | CH₂-CF₃ | |
| 6-130 | SMe | SO₂Me | Me | Me | |
| 6-131 | CF₃ | CF₃ | Me | Me | |
| 6-132 | CF₃ | CF₃ | Me | Et | |
| 6-133 | CF₃ | CF₃ | Me | CH₂-c-Pr | |
| 6-134 | CF₃ | CF₃ | Me | CH₂-CF₃ | |
| 6-135 | F | CF₃ | Me | Me | |
| 6-136 | Me | SO₂Me | Me | i-Bu | |

**Tabelle 7: Erfindungsgemäße Verbindungen der allgemeinen Formel (I), worin Q für Q4, R^{z} für eine Methylgruppe und R und W jeweils für Wasserstoff stehen (E-Isomer, ggf. Z-Isomer),**

| | | | | | |
|---|---|---|---|---|---|
| 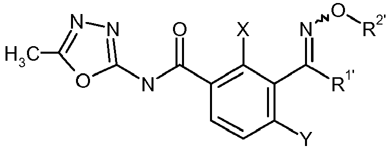 | | | | | |

| **Nr.** | **X** | **Y** | **R^{1'}** | **R^{2'}** | **Physikalische Daten** (¹H-NMR) |
|---|---|---|---|---|---|
| 7-1 | Cl | Cl | Me | Me | * |
| 7-2 | Cl | Cl | Et | Me | |
| 7-3 | Cl | Cl | CF₃ | Me | |
| 7-4 | Cl | Cl | OMe | Me | |
| 7-5 | Cl | Cl | Me | Et | * |
| 7-6 | Cl | Cl | Me | Pr | |
| 7-7 | Cl | Cl | Me | c-Pr | |
| 7-8 | Cl | Cl | Me | CH₂-c-Pr | * |
| 7-9 | Cl | Cl | Me | CH₂-CF₃ | * |
| 7-10 | Cl | SMe | Me | Me | |
| 7-11 | Cl | SMe | Me | Et | |
| 7-12 | Cl | SMe | Me | CH₂-c-Pr | |
| 7-13 | Cl | SMe | Me | CH₂-CF₃ | |
| 7-14 | Cl | S(O)Me | Me | Me | |
| 7-15 | Cl | SO₂Me | Me | Me | * |
| 7-16 | Cl | SO₂Me | Et | Me | |
| 7-17 | Cl | SO₂Me | CF₃ | Me | |
| 7-18 | Cl | SO₂Me | OMe | Me | |
| 7-19 | Cl | SO₂Me | Me | Et | * |
| 7-20 | Cl | SO₂Me | Me | Pr | |
| 7-21 | Cl | SO₂Me | Me | c-Pr | |
| 7-22 | Cl | SO₂Me | Me | CH₂-c-Pr | * |
| 7-23 | Cl | SO₂Me | Me | CH₂-CF₃ | |
| 7-24 | Cl | Me | Me | Me | |
| 7-25 | Cl | Me | Me | Et | |
| 7-26 | Cl | Me | Me | CH₂-c-Pr | |
| 7-27 | Cl | Me | Me | CH₂-CF₃ | |
| 7-28 | Cl | Et | Me | Me | |
| 7-29 | Cl | c-Pr | Me | Me | |
| 7-30 | Cl | CF₃ | Me | Me | |
| 7-31 | Cl | CF₃ | Et | Me | |
| 7-32 | Cl | CF₃ | CF₃ | Me | |
| 7-33 | Cl | CF₃ | OMe | Me | |
| 7-34 | Cl | CF₃ | Me | Et | |
| 7-35 | Cl | CF₃ | Me | Pr | |
| 7-36 | Cl | CF₃ | Me | c-Pr | |
| 7-37 | Cl | CF₃ | Me | CH₂-c-Pr | |
| 7-38 | Cl | CF₃ | Me | CH₂-CF₃ | |
| 7-39 | Cl | OMe | Me | Me | |
| 7-40 | Cl | OMe | Me | Et | |
| 7-41 | Cl | OMe | Me | CH₂-c-Pr | |
| 7-42 | Cl | OMe | Me | CH₂-CF₃ | |
| 7-43 | Cl | SO₂Et | Me | Me | |
| 7-44 | Cl | SO₂Et | Me | Et | |
| 7-45 | Cl | SO₂Et | Me | CH₂-c-Pr | |
| 7-46 | Cl | SO₂Et | Me | CH₂-CF₃ | |
| 7-47 | Cl | C₂F₅ | Me | Me | |
| 7-48 | Cl | Vinyl | Me | Me | |
| 7-49 | Cl | 1-Me-vinyl | Me | Me | |
| 7-50 | Br | SO₂Me | Me | Me | |
| 7-51 | Br | SO₂Me | Et | Me | |
| 7-52 | Br | SO₂Me | CF₃ | Me | |
| 7-53 | Br | SO₂Me | OMe | Me | |
| 7-54 | Br | SO₂Me | Me | Et | |
| 7-55 | Br | SO₂Me | Me | Pr | |
| 7-56 | Br | SO₂Me | Me | c-Pr | |
| 7-57 | Br | SO₂Me | Me | CH₂-c-Pr | |
| 7-58 | Br | SO₂Me | Me | CH₂-CF₃ | |
| 7-59 | Br | Me | Me | Me | |
| 7-60 | Br | Et | Me | Me | |
| 7-61 | Br | CF₃ | Me | Me | |
| 7-62 | Br | CF₃ | Me | Et | |
| 7-63 | Br | CF₃ | Me | CH₂-c-Pr | |
| 7-64 | Br | CF₃ | Me | CH₂-CF₃ | |
| 7-65 | Me | SMe | Me | Me | |
| 7-66 | Me | S(O)Me | Me | Me | |
| 7-67 | Me | SO₂Me | Me | Me | |
| 7-68 | Me | SO₂Me | Et | Me | |
| 7-69 | Me | SO₂Me | CF₃ | Me | |
| 7-70 | Me | SO₂Me | OMe | Me | |
| 7-71 | Me | SO₂Me | Me | Et | * |
| 7-72 | Me | SO₂Me | Me | Pr | |
| 7-73 | Me | SO₂Me | Me | c-Pr | |
| 7-74 | Me | SO₂Me | Me | CH₂-c-Pr | * |
| 7-75 | Me | SO₂Me | Me | CH₂-CF₃ | |
| 7-76 | Me | Me | Me | Me | |
| 7-77 | Me | Me | Et | Me | |
| 7-78 | Me | Me | CF₃ | Me | |
| 7-79 | Me | Me | OMe | Me | |
| 7-80 | Me | Me | Me | Et | |
| 7-81 | Me | Me | Me | Pr | |
| 7-82 | Me | Me | Me | c-Pr | |
| 7-83 | Me | Me | Me | CH₂-c-Pr | |
| 7-84 | Me | Me | Me | CH₂-CF₃ | |
| 7-85 | Me | Et | Me | Me | |
| 7-86 | Me | CF₃ | Me | Me | |
| 7-87 | Me | CF₃ | Et | Me | |
| 7-88 | Me | CF₃ | CF₃ | Me | |
| 7-89 | Me | CF₃ | OMe | Me | |
| 7-90 | Me | CF₃ | Me | Et | |
| 7-91 | Me | CF₃ | Me | Pr | |
| 7-92 | Me | CF₃ | Me | c-Pr | |
| 7-93 | Me | CF₃ | Me | CH₂-c-Pr | |
| 7-94 | Me | CF₃ | Me | CH₂-CF₃ | |
| 7-95 | Me | SO₂Et | Me | Me | |
| 7-96 | Me | SO₂Et | Me | Et | |
| 7-97 | Me | SO₂Et | Me | CH₂-c-Pr | |
| 7-98 | Me | SO₂Et | Me | CH₂-CF₃ | |
| 7-99 | Me | C₂F₅ | Me | Me | |
| 7-100 | Me | c-Pr | Me | Me | |
| 7-101 | OMe | Cl | Me | Me | |
| 7-102 | OMe | CF₃ | Me | Me | |
| 7-103 | OMe | CF₃ | Me | Et | |
| 7-104 | OMe | CF₃ | Me | CH₂-c-Pr | |
| 7-105 | OMe | CF₃ | Me | CH₂-CF₃ | |
| 7-106 | OMe | CHF₂ | Me | Me | |
| 7-107 | OMe | CHF₂ | Et | Me | |
| 7-108 | OMe | CHF₂ | CF₃ | Me | |
| 7-109 | OMe | CHF₂ | OMe | Me | |
| 7-110 | OMe | CHF₂ | Me | Et | |
| 7-111 | OMe | CHF₂ | Me | Pr | |
| 7-112 | OMe | CHF₂ | Me | c-Pr | |
| 7-113 | OMe | CHF₂ | Me | CH₂-c-Pr | |
| 7-114 | OMe | CHF₂ | Me | CH₂-CF₃ | |
| 7-115 | OMe | CF₂Cl | Me | Me | |
| 7-116 | OMe | SO₂Me | Me | Me | |
| 7-117 | SO₂Me | CF₃ | Me | Me | |
| 7-118 | SO₂Me | CF₃ | Et | Me | |
| 7-119 | SO₂Me | CF₃ | CF₃ | Me | |
| 7-120 | SO₂Me | CF₃ | OMe | Me | |
| 7-121 | SO₂Me | CF₃ | Me | Et | |
| 7-122 | SO₂Me | CF₃ | Me | Pr | |
| 7-123 | SO₂Me | CF₃ | Me | c-Pr | |
| 7-124 | SO₂Me | CF₃ | Me | CH₂-c-Pr | |
| 7-125 | CH₂OMe | CF₃ | Me | CH₂-CF₃ | |
| 7-126 | SMe | CF₃ | Me | Me | |
| 7-127 | SMe | CF₃ | Me | Et | |
| 7-128 | SMe | CF₃ | Me | CH₂-c-Pr | |
| 7-129 | SMe | CF₃ | Me | CH₂-CF₃ | |
| 7-130 | SMe | SO₂Me | Me | Me | |
| 7-131 | CF₃ | CF₃ | Me | Me | |
| 7-132 | CF₃ | CF₃ | Me | Et | |
| 7-133 | CF₃ | CF₃ | Me | CH₂-c-Pr | |
| 7-134 | CF₃ | CF₃ | Me | CH₂-CF₃ | |
| 7-135 | F | CF₃ | Me | Me | |
| 7-136 | Me | SO₂Me | Me | i-Bu | |

**Tabelle 8: Erfindungsgemäße Verbindungen der allgemeinen Formel (I), worin Q für Q1, R^{x} für eine Methylgruppe und R und W jeweils für Wasserstoff stehen (Z-Isomer, ggf. E-Isomer)**

| | | | | | |
|---|---|---|---|---|---|
| 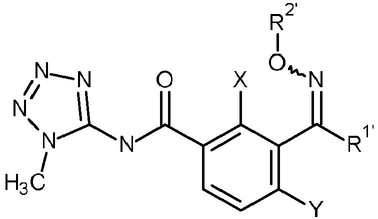 | | | | | |

| **Nr.** | **X** | **Y** | **R^{1'}** | **R^{2'}** | **Physikalische Daten** (¹H-NMR) |
|---|---|---|---|---|---|
| 8-1 | Cl | Cl | Me | Me | |
| 8-2 | Cl | Cl | Me | Et | |
| 8-3 | Cl | Cl | Me | CH₂-c-Pr | |
| 8-4 | Cl | Cl | Me | CH₂-CF₃ | |
| 8-5 | Cl | SO₂Me | Me | Me | * |
| 8-6 | Cl | SO₂Me | Me | Et | |
| 8-7 | Cl | SO₂Me | Me | CH₂-c-Pr | |
| 8-8 | Cl | SO₂Me | Me | CH₂-CF₃ | |
| 8-9 | Cl | Me | Me | Me | |
| 8-10 | Cl | Me | Me | Et | |
| 8-11 | Cl | Me | Me | CH₂-c-Pr | |
| 8-12 | Cl | Me | Me | CH₂-CF₃ | |
| 8-13 | Cl | CF₃ | Me | Me | |
| 8-14 | Cl | CF₃ | Me | Et | |
| 8-15 | Cl | CF₃ | Me | CH₂-c-Pr | |
| 8-16 | Cl | CF₃ | Me | CH₂-CF₃ | |
| 8-17 | Br | SO₂Me | Me | Me | |
| 8-18 | Br | SO₂Me | Me | Et | |
| 8-19 | Br | SO₂Me | Me | CH₂-c-Pr | |
| 8-20 | Br | SO₂Me | Me | CH₂-CF₃ | |
| 8-21 | Br | CF₃ | Me | Me | |
| 8-22 | Br | CF₃ | Me | Et | |
| 8-23 | Br | CF₃ | Me | CH₂-c-Pr | |
| 8-24 | Br | CF₃ | Me | CH₂-CF₃ | |
| 8-25 | Me | SO₂Me | Me | Me | * |
| 8-26 | Me | SO₂Me | Me | Et | * |
| 8-27 | Me | SO₂Me | Me | CH₂-c-Pr | |
| 8-28 | Me | SO₂Me | Me | CH₂-CF₃ | |
| 8-29 | Me | CF₃ | Me | Me | |
| 8-30 | Me | CF₃ | Me | Et | |
| 8-31 | Me | CF₃ | Me | CH₂-c-Pr | |
| 8-32 | Me | CF₃ | Me | CH₂-CF₃ | |
| 8-33 | OMe | CF₃ | Me | Me | |
| 8-34 | OMe | CF₃ | Me | Et | |
| 8-35 | OMe | CF₃ | Me | CH₂-c-Pr | |
| 8-36 | OMe | CF₃ | Me | CH₂-CF₃ | |
| 8-37 | OMe | CHF₂ | Me | Me | |
| 8-38 | OMe | CHF₂ | Me | Et | |
| 8-39 | OMe | CHF₂ | Me | CH₂-c-Pr | |
| 8-40 | OMe | CHF₂ | Me | CH₂-CF₃ | |
| 8-41 | SO₂Me | CF₃ | Me | Me | |
| 8-42 | SO₂Me | CF₃ | Me | Et | |
| 8-43 | SO₂Me | CF₃ | Me | CH₂-c-Pr | |
| 8-44 | SO₂Me | CF₃ | Me | CH₂-CF₃ | |
| 8-45 | Me | SO₂Me | Me | i-Bu | |

**Tabelle 9: Erfindungsgemäße Verbindungen der allgemeinen Formel (I), worin Q für Q1, R^{x} für eine Ethylgruppe und R und W jeweils für Wasserstoff stehen (Z-Isomer, ggf. E-Isomer)**

| | | | | | |
|---|---|---|---|---|---|
| 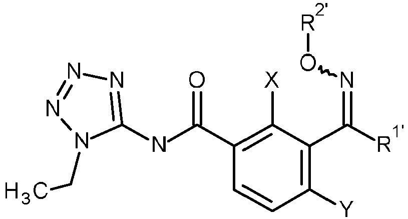 | | | | | |

| **Nr.** | **X** | **Y** | **R^{1'}** | **R^{2'}** | **Physikalische Daten** (¹H-NMR) |
|---|---|---|---|---|---|
| 9-1 | Cl | Cl | Me | Me | |
| 9-2 | Cl | Cl | Me | Et | |
| 9-3 | Cl | Cl | Me | CH₂-c-Pr | |
| 9-4 | Cl | Cl | Me | CH₂-CF₃ | |
| 9-5 | Cl | SO₂Me | Me | Me | * |
| 9-6 | Cl | SO₂Me | Me | Et | |
| 9-7 | Cl | SO₂Me | Me | CH₂-c-Pr | |
| 9-8 | Cl | SO₂Me | Me | CH₂-CF₃ | |
| 9-9 | Cl | Me | Me | Me | |
| 9-10 | Cl | Me | Me | Et | |
| 9-11 | Cl | Me | Me | CH₂-c-Pr | |
| 9-12 | Cl | Me | Me | CH₂-CF₃ | |
| 9-13 | Cl | CF₃ | Me | Me | |
| 9-14 | Cl | CF₃ | Me | Et | |
| 9-15 | Cl | CF₃ | Me | CH₂-c-Pr | |
| 9-16 | Cl | CF₃ | Me | CH₂-CF₃ | |
| 9-17 | Br | SO₂Me | Me | Me | |
| 9-18 | Br | SO₂Me | Me | Et | |
| 9-19 | Br | SO₂Me | Me | CH₂-c-Pr | |
| 9-20 | Br | SO₂Me | Me | CH₂-CF₃ | |
| 9-21 | Br | CF₃ | Me | Me | |
| 9-22 | Br | CF₃ | Me | Et | |
| 9-23 | Br | CF₃ | Me | CH₂-c-Pr | |
| 9-24 | Br | CF₃ | Me | CH₂-CF₃ | |
| 9-25 | Me | SO₂Me | Me | Me | * |
| 9-26 | Me | SO₂Me | Me | Et | |
| 9-27 | Me | SO₂Me | Me | CH₂-c-Pr | |
| 9-28 | Me | SO₂Me | Me | CH₂-CF₃ | |
| 9-29 | Me | CF₃ | Me | Me | |
| 9-30 | Me | CF₃ | Me | Et | |
| 9-31 | Me | CF₃ | Me | CH₂-c-Pr | |
| 9-32 | Me | CF₃ | Me | CH₂-CF₃ | |
| 9-33 | OMe | CF₃ | Me | Me | |
| 9-34 | OMe | CF₃ | Me | Et | |
| 9-35 | OMe | CF₃ | Me | CH₂-c-Pr | |
| 9-36 | OMe | CF₃ | Me | CH₂-CF₃ | |
| 9-37 | OMe | CHF₂ | Me | Me | |
| 9-38 | OMe | CHF₂ | Me | Et | |
| 9-39 | OMe | CHF₂ | Me | CH₂-c-Pr | |
| 9-40 | OMe | CHF₂ | Me | CH₂-CF₃ | |
| 9-41 | SO₂Me | CF₃ | Me | Me | |
| 9-42 | SO₂Me | CF₃ | Me | Et | |
| 9-43 | SO₂Me | CF₃ | Me | CH₂-c-Pr | |
| 9-44 | SO₂Me | CF₃ | Me | CH₂-CF₃ | |
| 9-45 | Me | SO₂Me | Me | i-Bu | |

**Tabelle 10: Erfindungsgemäße Verbindungen der allgemeinen Formel (I), worin Q für Q1, R^{x} für eine Propylgruppe und R und W jeweils für Wasserstoff stehen (Z-Isomer, ggf. E-Isomer)**

| | | | | | |
|---|---|---|---|---|---|
| 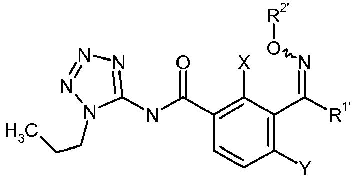 | | | | | |

| **Nr.** | **X** | **Y** | **R^{1'}** | **R^{2'}** | **Physikalische Daten** (¹H-NMR) |
|---|---|---|---|---|---|
| 10-1 | Cl | Cl | Me | Me | |
| 10-2 | Cl | Cl | Me | Et | |
| 10-3 | Cl | Cl | Me | CH₂-c-Pr | |
| 10-4 | Cl | Cl | Me | CH₂-CF₃ | |
| 10-5 | Cl | SO₂Me | Me | Me | * |
| 10-6 | Cl | SO₂Me | Me | Et | |
| 10-7 | Cl | SO₂Me | Me | CH₂-c-Pr | |
| 10-8 | Cl | SO₂Me | Me | CH₂-CF₃ | |
| 10-9 | Cl | Me | Me | Me | |
| 10-10 | Cl | Me | Me | Et | |
| 10-11 | Cl | Me | Me | CH₂-c-Pr | |
| 10-12 | Cl | Me | Me | CH₂-CF₃ | |
| 10-13 | Cl | CF₃ | Me | Me | |
| 10-14 | Cl | CF₃ | Me | Et | |
| 10-15 | Cl | CF₃ | Me | CH₂-c-Pr | |
| 10-16 | Cl | CF₃ | Me | CH₂-CF₃ | |
| 10-17 | Br | SO₂Me | Me | Me | |
| 10-18 | Br | SO₂Me | Me | Et | |
| 10-19 | Br | SO₂Me | Me | CH₂-c-Pr | |
| 10-20 | Br | SO₂Me | Me | CH₂-CF₃ | |
| 10-21 | Br | CF₃ | Me | Me | |
| 10-22 | Br | CF₃ | Me | Et | |
| 10-23 | Br | CF₃ | Me | CH₂-c-Pr | |
| 10-24 | Br | CF₃ | Me | CH₂-CF₃ | |
| 10-25 | Me | SO₂Me | Me | Me | |
| 10-26 | Me | SO₂Me | Me | Et | |
| 10-27 | Me | SO₂Me | Me | CH₂-c-Pr | |
| 10-28 | Me | SO₂Me | Me | CH₂-CF₃ | |
| 10-29 | Me | CF₃ | Me | Me | |
| 10-30 | Me | CF₃ | Me | Et | |
| 10-31 | Me | CF₃ | Me | CH₂-c-Pr | |
| 10-32 | Me | CF₃ | Me | CH₂-CF₃ | |
| 10-33 | OMe | CF₃ | Me | Me | |
| 10-34 | OMe | CF₃ | Me | Et | |
| 10-35 | OMe | CF₃ | Me | CH₂-c-Pr | |
| 10-36 | OMe | CF₃ | Me | CH₂-CF₃ | |
| 10-37 | OMe | CHF₂ | Me | Me | |
| 10-38 | OMe | CHF₂ | Me | Et | |
| 10-39 | OMe | CHF₂ | Me | CH₂-c-Pr | |
| 10-40 | OMe | CHF₂ | Me | CH₂-CF₃ | |
| 10-41 | SO₂Me | CF₃ | Me | Me | |
| 10-42 | SO₂Me | CF₃ | Me | Et | |
| 10-43 | SO₂Me | CF₃ | Me | CH₂-c-Pr | |
| 10-44 | SO₂Me | CF₃ | Me | CH₂-CF₃ | |
| 10-45 | Me | SO₂Me | Me | i-Bu | |

**Tabelle 11: Erfindungsgemäße Verbindungen der allgemeinen Formel (I), worin Q für Q2, R^{x} für eine Methylgruppe und R und W jeweils für Wasserstoff stehen (Z-Isomer, ggf. E-Isomer)**

| | | | | | |
|---|---|---|---|---|---|
| 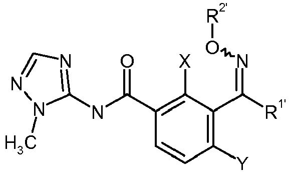 | | | | | |

| **Nr.** | **X** | **Y** | **R^{1'}** | **R^{2'}** | **Physikalische Daten** (¹H-NMR) |
|---|---|---|---|---|---|
| 11-1 | Cl | Cl | Me | Me | |
| 11-2 | Cl | Cl | Me | Et | |
| 11-3 | Cl | Cl | Me | CH₂-c-Pr | |
| 11-4 | Cl | Cl | Me | CH₂-CF₃ | |
| 11-5 | Cl | SO₂Me | Me | Me | * |
| 11-6 | Cl | SO₂Me | Me | Et | |
| 11-7 | Cl | SO₂Me | Me | CH₂-c-Pr | |
| 11-8 | Cl | SO₂Me | Me | CH₂-CF₃ | |
| 11-9 | Cl | Me | Me | Me | |
| 11-10 | Cl | Me | Me | Et | |
| 11-11 | Cl | Me | Me | CH₂-c-Pr | |
| 11-12 | Cl | Me | Me | CH₂-CF₃ | |
| 11-13 | Cl | CF₃ | Me | Me | |
| 11-14 | Cl | CF₃ | Me | Et | |
| 11-15 | Cl | CF₃ | Me | CH₂-c-Pr | |
| 11-16 | Cl | CF₃ | Me | CH₂-CF₃ | |
| 11-17 | Br | SO₂Me | Me | Me | |
| 11-18 | Br | SO₂Me | Me | Et | |
| 11-19 | Br | SO₂Me | Me | CH₂-c-Pr | |
| 11-20 | Br | SO₂Me | Me | CH₂-CF₃ | |
| 11-21 | Br | CF₃ | Me | Me | |
| 11-22 | Br | CF₃ | Me | Et | |
| 11-23 | Br | CF₃ | Me | CH₂-c-Pr | |
| 11-24 | Br | CF₃ | Me | CH₂-CF₃ | |
| 11-25 | Me | SO₂Me | Me | Me | |
| 11-26 | Me | SO₂Me | Me | Et | |
| 11-27 | Me | SO₂Me | Me | CH₂-c-Pr | |
| 11-28 | Me | SO₂Me | Me | CH₂-CF₃ | |
| 11-29 | Me | CF₃ | Me | Me | |
| 11-30 | Me | CF₃ | Me | Et | |
| 11-31 | Me | CF₃ | Me | CH₂-c-Pr | |
| 11-32 | Me | CF₃ | Me | CH₂-CF₃ | |
| 11-33 | OMe | CF₃ | Me | Me | |
| 11-34 | OMe | CF₃ | Me | Et | |
| 11-35 | OMe | CF₃ | Me | CH₂-c-Pr | |
| 11-36 | OMe | CF₃ | Me | CH₂-CF₃ | |
| 11-37 | OMe | CHF₂ | Me | Me | |
| 11-38 | OMe | CHF₂ | Me | Et | |
| 11-39 | OMe | CHF₂ | Me | CH₂-c-Pr | |
| 11-40 | OMe | CHF₂ | Me | CH₂-CF₃ | |
| 11-41 | SO₂Me | CF₃ | Me | Me | |
| 11-42 | SO₂Me | CF₃ | Me | Et | |
| 11-43 | SO₂Me | CF₃ | Me | CH₂-c-Pr | |
| 11-44 | SO₂Me | CF₃ | Me | CH₂-CF₃ | |
| 11-45 | Me | SO₂Me | Me | i-Bu | |

**Tabelle 12: Erfindungsgemäße Verbindungen der allgemeinen Formel (I), worin Q für Q3, R^{y} für Chlor und R und W jeweils für Wasserstoff stehen (Z-Isomer, ggf. E-Isomer)**

| | | | | | |
|---|---|---|---|---|---|
| 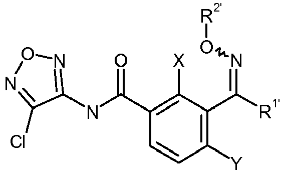 | | | | | |

| **Nr.** | **X** | **Y** | **R^{1'}** | **R^{2'}** | **Physikalische Daten** (¹H-NMR) |
|---|---|---|---|---|---|
| 12-1 | Cl | Cl | Me | Me | |
| 12-2 | Cl | Cl | Me | Et | |
| 12-3 | Cl | Cl | Me | CH₂-c-Pr | |
| 12-4 | Cl | Cl | Me | CH₂-CF₃ | |
| 12-5 | Cl | SO₂Me | Me | Me | |
| 12-6 | Cl | SO₂Me | Me | Et | |
| 12-7 | Cl | SO₂Me | Me | CH₂-c-Pr | |
| 12-8 | Cl | SO₂Me | Me | CH₂-CF₃ | |
| 12-9 | Cl | Me | Me | Me | |
| 12-10 | Cl | Me | Me | Et | |
| 12-11 | Cl | Me | Me | CH₂-c-Pr | |
| 12-12 | Cl | Me | Me | CH₂-CF₃ | |
| 12-13 | Cl | CF₃ | Me | Me | |
| 12-14 | Cl | CF₃ | Me | Et | |
| 12-15 | Cl | CF₃ | Me | CH₂-c-Pr | |
| 12-16 | Cl | CF₃ | Me | CH₂-CF₃ | |
| 12-17 | Br | SO₂Me | Me | Me | |
| 12-18 | Br | SO₂Me | Me | Et | |
| 12-19 | Br | SO₂Me | Me | CH₂-c-Pr | |
| 12-20 | Br | SO₂Me | Me | CH₂-CF₃ | |
| 12-21 | Br | CF₃ | Me | Me | |
| 12-22 | Br | CF₃ | Me | Et | |
| 12-23 | Br | CF₃ | Me | CH₂-c-Pr | |
| 12-24 | Br | CF₃ | Me | CH₂-CF₃ | |
| 12-25 | Me | SO₂Me | Me | Me | * |
| 12-26 | Me | SO₂Me | Me | Et | * |
| 12-27 | Me | SO₂Me | Me | CH₂-c-Pr | |
| 12-28 | Me | SO₂Me | Me | CH₂-CF₃ | |
| 12-29 | Me | CF₃ | Me | Me | |
| 12-30 | Me | CF₃ | Me | Et | |
| 12-31 | Me | CF₃ | Me | CH₂-c-Pr | |
| 12-32 | Me | CF₃ | Me | CH₂-CF₃ | |
| 12-33 | OMe | CF₃ | Me | Me | |
| 12-34 | OMe | CF₃ | Me | Et | |
| 12-35 | OMe | CF₃ | Me | CH₂-c-Pr | |
| 12-36 | OMe | CF₃ | Me | CH₂-CF₃ | |
| 12-37 | OMe | CHF₂ | Me | Me | |
| 12-38 | OMe | CHF₂ | Me | Et | |
| 12-39 | OMe | CHF₂ | Me | CH₂-c-Pr | |
| 12-40 | OMe | CHF₂ | Me | CH₂-CF₃ | |
| 12-41 | SO₂Me | CF₃ | Me | Me | |
| 12-42 | SO₂Me | CF₃ | Me | Et | |
| 12-43 | SO₂Me | CF₃ | Me | CH₂-c-Pr | |
| 12-44 | SO₂Me | CF₃ | Me | CH₂-CF₃ | |
| 12-45 | Me | SO₂Me | Me | i-Bu | |

**Tabelle 13: Erfindungsgemäße Verbindungen der allgemeinen Formel (I), worin Q für Q3, R^{y} für Methyl und R und W jeweils für Wasserstoff stehen (Z-Isomer, ggf. E-Isomer)**

| | | | | | |
|---|---|---|---|---|---|
| 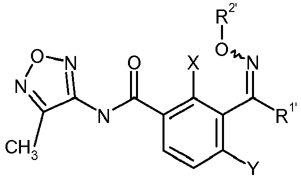 | | | | | |

| **Nr.** | **X** | **Y** | **R^{1'}** | **R^{2'}** | **Physikalische Daten** (¹H-NMR) |
|---|---|---|---|---|---|
| 13-1 | Cl | Cl | Me | Me | |
| 13-2 | Cl | Cl | Me | Et | |
| 13-3 | Cl | Cl | Me | CH₂-c-Pr | |
| 13-4 | Cl | Cl | Me | CH₂-CF₃ | |
| 13-5 | Cl | SO₂Me | Me | Me | * |
| 13-6 | Cl | SO₂Me | Me | Et | |
| 13-7 | Cl | SO₂Me | Me | CH₂-c-Pr | |
| 13-8 | Cl | SO₂Me | Me | CH₂-CF₃ | |
| 13-9 | Cl | Me | Me | Me | |
| 13-10 | Cl | Me | Me | Et | |
| 13-11 | Cl | Me | Me | CH₂-c-Pr | |
| 13-12 | Cl | Me | Me | CH₂-CF₃ | |
| 13-13 | Cl | CF₃ | Me | Me | |
| 13-14 | Cl | CF₃ | Me | Et | |
| 13-15 | Cl | CF₃ | Me | CH₂-c-Pr | |
| 13-16 | Cl | CF₃ | Me | CH₂-CF₃ | |
| 13-17 | Br | SO₂Me | Me | Me | |
| 13-18 | Br | SO₂Me | Me | Et | |
| 13-19 | Br | SO₂Me | Me | CH₂-c-Pr | |
| 13-20 | Br | SO₂Me | Me | CH₂-CF₃ | |
| 13-21 | Br | CF₃ | Me | Me | |
| 13-22 | Br | CF₃ | Me | Et | |
| 13-23 | Br | CF₃ | Me | CH₂-c-Pr | |
| 13-24 | Br | CF₃ | Me | CH₂-CF₃ | |
| 13-25 | Me | SO₂Me | Me | Me | * |
| 13-26 | Me | SO₂Me | Me | Et | * |
| 13-27 | Me | SO₂Me | Me | CH₂-c-Pr | |
| 13-28 | Me | SO₂Me | Me | CH₂-CF₃ | |
| 13-29 | Me | CF₃ | Me | Me | |
| 13-30 | Me | CF₃ | Me | Et | |
| 13-31 | Me | CF₃ | Me | CH₂-c-Pr | |
| 13-32 | Me | CF₃ | Me | CH₂-CF₃ | |
| 13-33 | OMe | CF₃ | Me | Me | |
| 13-34 | OMe | CF₃ | Me | Et | |
| 13-35 | OMe | CF₃ | Me | CH₂-c-Pr | |
| 13-36 | OMe | CF₃ | Me | CH₂-CF₃ | |
| 13-37 | OMe | CHF₂ | Me | Me | |
| 13-38 | OMe | CHF₂ | Me | Et | |
| 13-39 | OMe | CHF₂ | Me | CH₂-c-Pr | |
| 13-40 | OMe | CHF₂ | Me | CH₂-CF₃ | |
| 13-41 | SO₂Me | CF₃ | Me | Me | |
| 13-42 | SO₂Me | CF₃ | Me | Et | |
| 13-43 | SO₂Me | CF₃ | Me | CH₂-c-Pr | |
| 13-44 | SO₂Me | CF₃ | Me | CH₂-CF₃ | |
| 13-45 | Me | SO₂Me | Me | i-Bu | |

**Tabelle 14: Erfindungsgemäße Verbindungen der allgemeinen Formel (I), worin Q für Q4, R^{z} für Methyl und R und W jeweils für Wasserstoff stehen (Z-Isomer, ggf. E-Isomer)**

| | | | | | |
|---|---|---|---|---|---|
| 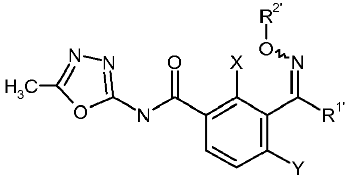 | | | | | |

| **Nr.** | **X** | **Y** | **R^{1'}** | **R^{2'}** | **Physikalische Daten** (¹H-NMR) |
|---|---|---|---|---|---|
| 14-1 | Cl | Cl | Me | Me | |
| 14-2 | Cl | Cl | Me | Et | |
| 14-3 | Cl | Cl | Me | CH₂-c-Pr | |
| 14-4 | Cl | Cl | Me | CH₂-CF₃ | |
| 14-5 | Cl | SO₂Me | Me | Me | * |
| 14-6 | Cl | SO₂Me | Me | Et | |
| 14-7 | Cl | SO₂Me | Me | CH₂-c-Pr | |
| 14-8 | Cl | SO₂Me | Me | CH₂-CF₃ | |
| 14-9 | Cl | Me | Me | Me | |
| 14-10 | Cl | Me | Me | Et | |
| 14-11 | Cl | Me | Me | CH₂-c-Pr | |
| 14-12 | Cl | Me | Me | CH₂-CF₃ | |
| 14-13 | Cl | CF₃ | Me | Me | |
| 14-14 | Cl | CF₃ | Me | Et | |
| 14-15 | Cl | CF₃ | Me | CH₂-c-Pr | |
| 14-16 | Cl | CF₃ | Me | CH₂-CF₃ | |
| 14-17 | Br | SO₂Me | Me | Me | |
| 14-18 | Br | SO₂Me | Me | Et | |
| 14-19 | Br | SO₂Me | Me | CH₂-c-Pr | |
| 14-20 | Br | SO₂Me | Me | CH₂-CF₃ | |
| 14-21 | Br | CF₃ | Me | Me | |
| 14-22 | Br | CF₃ | Me | Et | |
| 14-23 | Br | CF₃ | Me | CH₂-c-Pr | |
| 14-24 | Br | CF₃ | Me | CH₂-CF₃ | |
| 14-25 | Me | SO₂Me | Me | Me | |
| 14-26 | Me | SO₂Me | Me | Et | |
| 14-27 | Me | SO₂Me | Me | CH₂-c-Pr | |
| 14-28 | Me | SO₂Me | Me | CH₂-CF₃ | |
| 14-29 | Me | CF₃ | Me | Me | |
| 14-30 | Me | CF₃ | Me | Et | |
| 14-31 | Me | CF₃ | Me | CH₂-c-Pr | |
| 14-32 | Me | CF₃ | Me | CH₂-CF₃ | |
| 14-33 | OMe | CF₃ | Me | Me | |
| 14-34 | OMe | CF₃ | Me | Et | |
| 14-35 | OMe | CF₃ | Me | CH₂-c-Pr | |
| 14-36 | OMe | CF₃ | Me | CH₂-CF₃ | |
| 14-37 | OMe | CHF₂ | Me | Me | |
| 14-38 | OMe | CHF₂ | Me | Et | |
| 14-39 | OMe | CHF₂ | Me | CH₂-c-Pr | |
| 14-40 | OMe | CHF₂ | Me | CH₂-CF₃ | |
| 14-41 | SO₂Me | CF₃ | Me | Me | |
| 14-42 | SO₂Me | CF₃ | Me | Et | |
| 14-43 | SO₂Me | CF₃ | Me | CH₂-c-Pr | |
| 14-44 | SO₂Me | CF₃ | Me | CH₂-CF₃ | |
| 14-45 | Me | SO₂Me | Me | i-Bu | |

Die verwendeten Abkürzungen bedeuten:
Et = Ethyl Me = Methyl n-Pr = n-Propyl i-Pr = Isopropyl c-Pr = cyclo-Propyl Ph = Phenyl Bn = Benzyl Bu = Butyl c = cyclo

NMR-Daten ausgewählter Beispiele angegeben nach dem NMR-Peak-Listenverfahren
Die ¹H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁); δ₂ (Intensität₂);........; δᵢ (Intensitätᵢ);......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren wurde Tetramethylsilan und/oder die chemische Verschiebung des Lösungsmittels, besondern im Falle von Spektren, die in DMSO gemessen werden, benutzt. Daher kann in NMR-Peaklisten der Tetramethylsilan-Peak vorkommen.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

| |
|---|
| Beispiel 1-5: ¹H-NMR(400,0 MHz, d₆-DMSO): δ = 11,861(0,6);7,818(0,7);7,797(1,2);7,740(2,1);7,720(1,3);4,185(1,0);4,168(3,2);4,150(3,2);4,133(1,0);3,997(16,0);3,310(13,3);2,510(4,5);2,505(9,7);2,501(13,4);2,496(9,3);2,491(4,2);2,113(11,1);2,072(15,1);1,267(3,6);1,250(8,1);1,232(3,5) |
| Beispiel 1-8: ¹H-NMR(400,0 MHz, d₆-DMSO): δ = 11,859(0,8);7,816(0,7);7,795(1,2);7,739(2,1);7,718(1,3);3,996(16,0);3,969(2,0);3,951(2,0);3,310(30,4);2,518(0,6);2,510(7,4);2,505(15,7);2,500(21,7);2,496(15,2);2,491(6,8);2,130(10,7);1,145(0,6);0,533(1,4);0,528(1,5);0,523(0,6);0,518(0,7);0,512(1,5);0,508(1,3);0,498(0,6);0,308(0,6);0,297(1,6);0,293(1,6);0,285(1,4);0,281(1,7) |
| Beispiel 1-9: ¹H-NMR(400,0 MHz, d₆-DMSO): δ = 11,881(0,8);7,856(0,7);7,835(1,2);7,768(2,0);7,747(1,3);5,751(1,0);4,840(0,6);4,817(2,0);4,794(2,1);4,772(0,7);3,996(16,0);3,310(39,5);2,523(0,6);2,518(0,9);2,510(10,8);2,505(23,1);2,500(31,9);2,496(22,3);2,491(10,0);2,192(10,9);2,072(1,2) |
| Beispiel 1-15: ¹H-NMR(400,0 MHz, CDCl₃): δ = 8,163(2,1);8,142(2,5);7,873(1,9);7,852(1,6);7,261(38,1);4,141(12,7);3,823(15,7);3,100(16,0);2,340(13,5);1,285(0,5);0,000(14,4) |
| Beispiel 1-22: ¹H-NMR(400,0 MHz, d₆-DMSO): δ = 12,009(1,1);8,125(0,6);8,105(1,4);8,079(0,7);5,751(9,1);4,017(16,0);3,970(2,3);3,953(2,4);3,346(16,1);2,518(0,6);2,510(7,7);2,505(16,6);2,500(22,9);2,496(16,0);2,491(7,2);2,164(6,2);1,146(0,6);0,542(1,3);0,538(1,5);0,530(0,7);0,528(0,5);0,522(1,4);0,518(1,3);0,302(0,6);0,291(1,3);0,287(1,1);0,279(1,4);0,275(0,9);0,000(1,1) |
| Beispiel 1-67: ¹H-NMR(400,0 MHz, CDCl₃): δ = 8,096(1,0);8,075(1,4);7,939(1,5);7,918(1,2);7,260(38,5);4,126(9,5);3,965(16,0);3,219(14,2);2,472(8,7);2,221(14,5);0,000(14,8) |
| Beispiel 1-71: ¹H-NMR(400,0 MHz, CDCl₃): δ = 8,081(1,2);8,061(1,6);7,937(1,8);7,917(1,4);7,261(32,7);4,220(1,3);4,202(4,1);4,185(4,2);4,167(1,3);4,123(11,9);3,204(15,7);2,466(9,9);2,221(16,0);1,337(4,6);1,319(10,1);1,302(4,5);0,000(12,9) |
| Beispiel 1-74: ¹H-NMR(400,0 MHz, d₆-DMSO): δ = 7,987(0,9);7,967(1,6);7,918(1,3);7,898(0,7);5,753(7,7);4,010(16,0);3,956(1,7);3,951(1,7);3,938(1,8);3,934(1,7);3,311(15,0);3,275(10,5);3,247(1,2);2,518(0,6);2,510(7,3);2,505(15,7);2,500(21,7);2,496(15,1);2,491(6,8);2,375(1,0);2,331(8,0);2,146(11,6);2,116(1,4);1,144(0,6);0,536(1,3);0,531(1,5);0,520(0,6);0,516(1,5);0,511(1,4);0,294(0,6);0,287(0,8);0,283(1,3);0,278(1,2);0,271(1,5);0,266(0,9);0,260(0,6);0,000(5,0) |
| Beispiel 2- 1 |
| : ¹H-NMR(400,0 MHz, d₆-DMSO): δ = 11,765(1,4);7,815(0,8);7,795(1,4);7,744(2,5);7,723(1,4);4,384(1,0);4,366(3,3);4,347(3,3);4,329(1,1);3,907(16,0);3,889(0,6);3,313(26,3);2,523(0,7);2,510(9,4);2,505(19,9);2,501(27,5);2,496(19,6);2,492(9,1);2,112(12,7);2,083(0,6);1,484(3,7);1,466(8,3);1,447(3,7);0,0 00(0,5) |
| Beispiel 2-15: ¹H-NMR(400,0 MHz, CDCl₃): δ = 8,169(1,9);8,149(2,2);7,899(1,4);7,878(1,2);7,263(38,0);4,533(0,6);4,515(2,0);4,497(2,1);4,479(0,7);3,986(16,0);3,270(14,2);2,262(11,9);1,646(3,2);1,628(7,3);1,610(3,1);0,000(14,7) |
| Beispiel 2-19: ¹H-NMR(400,0 MHz, CDCl₃): δ = 8,160(2,1);8,140(2,5);7,892(1,6);7,872(1,4);7,263(34,8);4,532(0,7);4,514(2,2);4,496(2,3);4,478(0,8);4,241(1,0);4,223(3,0);4,206(3,3);4,188(1,1);3,256(16,0);2,261(13,6);1,645(3,6);1,627(8,2);1,609(3,5);1,347(4,8);1,330(10,3);1,312(4,6);0,000(13,1) |
| Beispiel 2-22: ¹H-NMR(400,0 MHz, d₆-DMSO): δ = 11,913(4,1);8,126(1,6);8,105(3,5);8,075(1,6);8,054(0,8);5,752(9,0);4,402(2,0);4,384(6,4);4,366(6,5);4,348(2,1);3,972(4,6);3,969(4,6);3,954(4,9);3,952(4,6);3,394(0,7);3,345(63,8);2,669(0,6);2,550(0,8);2,545(0,7);2,540(0,6);2,523(2,1);2,518(3,2);2,509(34,2);2,505(71,6);2,500(98,2);2,496(68,7);2,491(31,0);2,327(0,7);2,165(14,5);1,492(7,1);1,474(16,0);1,456(7,0);1,166(0,8);1,163(0,8);1,158(0,8);1,154(0,6);1,146(1,4);1,137(0,6);1,133(0,8);1,128(0,8);1,126(0,9);0,552(0,8);0,543(3,1);0,539(3,5);0,531(1,7);0,528(1,3);0,523(3,3);0,519(3,1);0,510(0,9);0,302(1,4);0,291(3,3);0,279(3,3);0,275(2,2);0,267(1,0);0,000(4,6) |
| Beispiel 2-67: ¹H-NMR(400,0 MHz, CDCl₃): δ = 8,092(1,0);8,071(1,3);7,925(1,4);7,904(1,1);7,260(43,7);5,299(10,0);4,488(1,2);4,484(1,3);4,470(1,2);4,466(1,3);3,964(16,0);3,218(14,4);2,472(8,9);2,221(14,6);2,004(0,9);1,651(2,9);1,633(6,3);1,615(2,8);0,000(17,1) |
| Beispiel 2-71: ¹H-NMR(400,0 MHz, CDCl₃): δ = 8,085(1,2);8,065(1,5);7,908(1,2);7,888(1,0);7,261(59,3);4,510(0,5);4,507(0,5);4,492(1,5);4,489(1,5);4,473(1,6);4,470(1,6);4,455(0,6);4,452(0,5);4,222(1,3);4,204(4,3);4,187(4,3);4,169(1,4);3,206(15,7);2,466(9,0);2,224(16,0);1,655(3,3);1,636(7,4);1,618(3,2);1,339(4,7);1,321(10,2);1,303(4,6);0,008(0,6);0,000(21,8);-0,009(0,6) |
| Beispiel 2-74: ¹H-NMR(400,0 MHz, d₆-DMSO): δ = 11,689(1,7);7,990(1,2);7,970(2,1);7,911(1,5);7,890(0,9);5,753(8,2);4,385(1,1);4,367(3,7);4,349(3,8);4,330(1,2);3,956(2,2);3,951(2,3);3,938(2,3);3,934(2,2);3,311(33,8);3,274(14,3);2,523(0,7);2,518(1,0);2,510(11,8);2,505(25,4);2,500(35,2);2,496(24,4);2,491(10,8);2,328(10,9);2,147(16,0);1,493(4,6);1,475(10,9);1,456(4,6);1,144(0,8);0,537(1,8);0,532(2,0);0,528(0,8);0,521(0,7);0,516(1,9);0,512(1,8);0,507(0,5);0,293(0,8);0,287(1,0);0,283(1,7);0,278(1,5);0,274(1,2);0,271(1,9);0,266(1,2);0,260(0,7);0,000(7,5) |
| Beispiel 2-136: ¹H-NMR(400,0 MHz, d₆-DMSO): δ = 11,682(2,9);8,312(3,3);7,990(1,4);7,970(2,3);7,910(1,7);7,890(1,0);4,384(1,2);4,366(3,7);4,348(3,7);4,330(1,2);3,902(2,6);3,898(2,8);3,884(2,8);3,881(2,7);3,335(18,2);3,255(14,1);2,523(1,0);2,509(18,0);2,505(37,3);2,500(51,0);2,496(36,9);2,492(17,5);2,319(11,8);2,145(15,1);1,974(0,9);1,957(1,1);1,940(0,9);1,493(4,3);1,475(9,4);1,457(4,2);0,933(16,0);0,917(15,5);0,008(0,6);0,000(17,6);-0,009(0,7) |
| Beispiel 3-1: ¹H-NMR(400,0 MHz, d₆-DMSO): δ = 11,750(1,2);7,802(0,7);7,781(1,5);7,744(2,7);7,723(1,3);5,752(4,2);4,323(1,6);4,305(2,4);4,287(1,7);3,907(16,0);3,309(31,3);2,523(0,5);2,518(0,7);2,510(9,7);2,505(20,8);2,500(28,9);2,496(20,2);2,491(9,1);2,113(12,6);2,072(0,8);1,907(1,0);1,888(1,7);1,870(1,7);1,852(1,0);0,895(3,5);0,877(7,7);0,858(3,3);0,000(0,6) |
| Beispiel 3-5: ¹H-NMR(400,0 MHz, d₆-DMSO): δ = 11,747(1,3);7,797(0,9);7,776(1,9);7,740(3,5);7,719(1,7);5,751(1,0);4,322(2,1);4,305(3,0);4,287(2,1);4,186(1,4);4,168(4,7);4,151(4,7);4,133(1,5);3,310(36,8);2,523(0,5);2,518(0,8);2,510(9,7);2,505(20,9);2,501(29,0);2,496(20,2);2,491(9,0);2,114(16,0);2,072(7,7);1,907(1,2);1,889(2,2);1,871(2,2);1,853(1,3);1,267(5,3);1,250(11,9);1,232(5,1);0,895(4,5);0,876(9,9);0,858(4,2);0,000(1,8) |
| Beispiel 3-8: ¹H-NMR(400,0 MHz, d₆-DMSO): δ = 11,746(1,7);7,795(1,0);7,774(2,1);7,738(3,6);7,717(1,7);5,752(0,5);4,321(2,2);4,303(3,3);4,285(2,2);3,969(4,2);3,951(4,2);3,308(81,7);2,523(1,8);2,518(2,7);2,510(27,3);2,505(56,6);2,500(77,1);2,496(53,8);2,491(24,1);2,131(16,0);2,072(0,7);1,905(1,3);1,887(2,4);1,869(2,4);1,851(1,3);1,164(0,6);1,161(0,6);1,156(0,5);1,144(1,0);1,131(0,5);1,123(0,5);0,894(4,7);0,875(10,0);0,857(4,4);0,544(0,8);0,533(2,2);0,529(2,3);0,524(1,0);0,518(1,1);0,513(2,4);0,508(2,0);0,498(0,9);0,308(0,9);0,297(2,5);0,294(2,4);0,286(2,2);0,282(2,6);0,271(0,6);0,000(1,7) |
| Beispiel 3-9: ¹H-NMR(400,0 MHz, d₆-DMSO): δ = 11,767(1,4);7,836(1,0);7,815(1,8);7,768(3,3);7,747(1,8);5,751(1,4);4,840(0,9);4,818(2,9);4,795(3,0);4,773(1,0);4,321(2,2);4,304(3,2);4,285(2,2);3,309(62,7);3,257(0,6);2,523(1,4);2,518(2,0);2,510(19,5);2,505(40,5);2,500(55,3);2,496(38,3);2,491(16,8);2,194(16,0);2,072(2,5);1,906(1,3);1,888(2,3);1,870(2,4);1,852(1,3);0,894(4,7);0,876(10,1);0,857(4,3);0,000(1,1) |
| Beispiel 3-15: ¹H-NMR(400,0 MHz, CDCl₃): δ = 8,150(2,0);8,130(2,3);7,852(2,0);7,832(1,8);7,261(29,5);4,452(1,4);4,434(2,2);4,415(1,4);3,820(16,0);3,097(15,7);2,337(13,9);2,065(1,1);2,047(2,1);2,028(2,1);2,010(1,2);1,285(0,9);1,256(0,7);1,020(1,0);1,012(3,8);1,003(1,2);0,994(7,7);0,975(3,6);0,000(11,0) |
| Beispiel 3-19: ¹H-NMR(400,0 MHz, CDCl₃): δ = 8,173(1,0);8,153(1,2);7,888(1,9);7,867(1,7);7,519(0,7);7,260(134,4);6,996(0,7);4,444(0,7);4,425(1,1);4,406(0,7);4,244(1,0);4,227(3,1);4,209(3,3);4,192(1,1);3,249(16,0);2,265(15,0);2,060(0,9);2,041(1,5);2,023(1,5);2,004(0,9);1,350(4,8);1,333(10,6);1,315(4,6);1,285(0,7);1,011(3,6);0,992(7,8);0,974(3,4);0,008(1,5);0,000(51,6);-0,009(1,4) |
| Beispiel 3-22: ¹H-NMR(400,0 MHz, d₆-DMSO): δ = 11,898(3,0);8,127(1,7);8,107(3,1);8,063(1,3);8,043(0,7);5,752(16,0);4,340(3,1);4,322(4,6);4,304(3,1);3,973(3,4);3,968(3,5);3,955(3,6);3,951(3,3);3,345(22,9);3,318(47,5);2,669(0,7);2,523(2,0);2,518(2,9);2,509(38,3);2,505(82,3);2,500(114,3);2,496(79,8);2,491(35,7);2,332(0,5);2,327(0,8);2,322(0,5);2,165(12,9);1,914(1,8);1,896(3,2);1,878(3,2);1,860(1,8);1,165(0,7);1,163(0,7);1,157(0,7);1,154(0,5);1,145(1,3);1,137(0,5);1,133(0,7);1,128(0,7);1,125(0,8);0,902(6,7);0,883(14,6);0,865(6,3);0,551(0,7);0,543(2,6);0,539(3,1);0,531(1,5);0,528(1,1);0,523(2,8);0,518(2,7);0,510(0,8);0,508(0,8);0,302(1,1);0,291(2,8);0,287(2,3);0,279(2,9);0,275(1,8);0,267(0,9);0,000(11,5) |
| Beispiel 4-1: ¹H-NMR(400,0 MHz, d₆-DMSO): δ = 11,294(0,8);7,741(0,6);7,708(0,8);3,903(16,0);3,764(8,6);3,309(26,7);2,523(0,5);2,518(0,8);2,509(9,9);2,505(21,1);2,500(29,2);2,496(20,5);2,491(9,2);2,105(7,5);0,000(0,6) |
| Beispiel 4-5: ¹H-NMR(400,0 MHz, d₆-DMSO): δ = 11,292(1,3);7,894(0,8);7,757(0,6);7,736(1,0);7,704(1,5);7,683(0,7);5,751(0,9);4,182(1,9);4,165(6,3);4,147(6,4);4,130(2,0);3,764(15,2);3,310(73,9);2,523(1,0);2,518(1,5);2,510(19,0);2,505(40,9);2,500(56,8);2,496(39,8);2,491(18,0);2,107(12,9);2,072(3,4);1,266(7,3);1,249(16,0);1,231(7,1);0,000(0,7) |
| Beispiel 4-8: ¹H-NMR(400,0 MHz, d₆-DMSO): δ = 3,486(16,0);2,522(0,9);2,517(1,3);2,513(0,9) |
| Beispiel 4-9: ¹H-NMR(400,0 MHz, d₆-DMSO): δ = 11,313(1,8);7,893(0,9);7,797(0,7);7,776(1,2);7,732(1,6);7,711(0,9);4,836(1,5);4,813(5,0);4,791(5,2);4,768(1,8);3,765(16,0);3,361(0,7);3,309(100,1);2,669(0,6);2,523(1,7);2,518(2,6);2,509(30,1);2,505(64,1);2,500(89,2);2,496(62,4);2,491(27,7);2,327(0,6);2,186(15,5);0,000(1,0) |
| Beispiel 4-15: ¹H-NMR(400,0 MHz, CDCl₃): δ = 8,113(2,3); 8,093(2,6); 7,818(1,6); 7,798(1,4); 7,588(1,0);7,261(28,3); 3,997(16,0); 3,872(11,6); 3,245(15,1); 2,242(12,8); 0,000(10,4) |
| Beispiel 4-19: ¹H-NMR(400,0 MHz, CDCl₃): δ = 8,130(2,3);8,110(2,6);7,806(2,1);7,786(2,1);7,778(2,6);7,261(49,3);4,248(0,8);4,232(2,5);4,231(2,6);4,214(2,6);4,196(0,9);3,928(12,8);3,241(16,0);2,250(14,3);1,353(4,7);1,335(10,0);1,317(4,6);0,008(0,5);0,000(18,0);-0,009(0,6) |
| Beispiel 4-22: ¹H-NMR(400,0 MHz, d₆-DMSO): δ = 8,098(0,6);8,078(0,9);8,043(0,5);8,023(1,5);7,991(0,9);7,934(0,7);5,751(11,1);3,968(2,9);3,959(0,9);3,951(3,2);3,935(0,5);3,784(16,0);3,337(9,8);3,312(2,3);2,523(0,7);2,510(13,1);2,505(27,5);2,501(37,4);2,496(26,5);2,492(12,3);2,161(4,1);2,135(2,8);1,146(0,8);1,126(0,5);0,541(1,7);0,537(1,9);0,530(1,3);0,521(1,9);0,517(1,7);0,509(0,8);0,301(0,7);0,290(1,9);0,279(2,0);0,267(0,7);0,000(1,3) |
| Beispiel 4-74: ¹H-NMR(400,0 MHz, d₆-DMSO): δ = 11,231(1,1);8,312(4,4);7,964(0,8);7,944(1,2);7,900(0,9);7,862(0,8);7,843(0,6);3,953(2,9);3,949(3,0);3,935(3,0);3,931(2,9);3,777(11,4);3,310(41,3);3,266(15,3);2,523(0,7);2,518(1,1);2,509(16,1);2,505(35,6);2,500(49,9);2,496(35,1);2,491(15,9);2,326(10,5);2,141(16,0);1,164(0,6);1,161(0,5);1,156(0,5);1,144(1,0);1,132(0,6);1,126(0,5);1,124(0,6);0,540(0,5);0,535(2,2);0,531(2,6);0,526(1,0);0,519(0,9);0,515(2,5);0,510(2,3);0,505(0,7);0,499(0,6);0,293(1,0);0,286(1,3);0,282(2,1);0,277(2,0);0,274(1,5);0,270(2,5);0,265(1,5);0,259(0,9);0,000(10,9) |
| Beispiel 4-136: ¹H-NMR(400,0 MHz, d₆-DMSO): δ = 11,226(0,7);7,963(0,7);7,943(1,0);7,902(0,7);7,862(0,6);3,900(2,3);3,895(2,4);3,882(2,5);3,878(2,3);3,776(10,5);3,310(19,3);3,246(12,2);2,518(0,7);2,509(9,0);2,505(19,7);2,500(27,5);2,496(19,1);2,491(8,5);2,317(8,1);2,140(12,4);1,974(0,8);1,957(1,0);1,940(0,8);0,932(16,0);0,916(15,4);0,000(15,2) |
| Beispiel 5-1: ¹H-NMR(400,0 MHz, d₆-DMSO): δ = 11,843(1,1);7,723(10,6);5,751(4,3);3,905(16,0);3,309(19,5);2,510(5,6);2,505(12,0);2,501(16,5);2,496(11,5);2,491(5,1);2,110(14,7);2,072(1,1) |
| Beispiel 5-5: ¹H-NMR(400,0 MHz, d₆-DMSO): □ = 11,839(1,2);7,718(12,5);4,184(1,2);4,166(3,9);4,149(4,0);4,131(1,2);3,310(40,6);2,523(0,5);2,518(0,8);2,510(9,2);2,505(19,6);2,501(27,2);2,496(18,8);2,491(8,4);2,112(16,0);1,266(4,4);1,248(9,9);1,231(4,3) |
| Beispiel 5-8: ¹H-NMR(400,0 MHz, d₆-DMSO): δ =3,472(16,0);2,522(0,9);2,517(1,3);2,513(0,9) |
| Beispiel 5-9: ¹H-NMR(400,0 MHz, d₆-DMSO): δ = 11,855(1,3);7,775(0,7);7,754(6,1);7,749(6,1);7,728(0,7);4,836(0,8);4,814(2,5);4,791(2,6);4,768(0,9);3,309(38,4);2,523(0,6);2,518(0,9);2,510(10,3);2,505(21,9);2,501(30,6);2,496(21,5);2,491(9,6);2,192(16,0) |
| Beispiel 5-15: ¹H-NMR(400,0 MHz, CDCl₃): δ = 8,073(1,1);8,053(1,4);7,840(0,8);7,820(0,6);7,262(18,3);3,831(13,3);3,079(16,0);2,514(8,6);2,467(0,6);2,302(10,6);1,581(1,2);0,000(7,1) |
| Beispiel 5-22: ¹H-NMR(400,0 MHz, d₆-DMSO): δ = 11,987(2,2);8,115(3,4);8,095(4,7);7,988(4,3);7,967(3,3);3,968(3,1);3,951(3,3);3,344(15,8);3,308(33,4);2,523(0,7);2,518(1,0);2,510(12,9);2,505(28,0);2,500(39,1);2,496(27,5);2,491(12,4);2,162(16,0);1,144(0,9);1,124(0,5);0,542(1,8);0,538(2,1);0,530(1,0);0,527(0,7);0,521(2,0);0,517(1,8);0,510(0,5);0,507(0,5);0,300(0,8);0,290(1,9);0,286(1,5);0,278(1,9);0,274(1,2);0,266(0,6);0,000(2,2) |
| Beispiel 5-67: ¹H-NMR(400,0 MHz, CDCl₃): δ = 7,840(0,9); 7,819(1,1); 7,677(1,9); 7,656(1,5); 7,261(36,6);3,956(16,0); 3,191(14,4); 2,428(9,7); 2,161(12,2); 1,591(2,5); 0,000(13,6) |
| Beispiel 5-71: ¹H-NMR(400,0 MHz, CDCl₃): δ = 7,851(1,1);7,831(1,0);7,681(2,0);7,668(0,7);7,661(1,6);7,261(43,5);3,956(16,0);3,808(4,6);3, 192(14,7);3,034(4,4);2,429(10,7);2,371(3,2);2,232(3,5);2,160(11,8);1,603(3,4);0,008(0,6);0,000(16,9) |
| Beispiel 5-74: ¹H-NMR(400,0 MHz, d₆-DMSO): δ = 11,803(1,7);7,983(1,6);7,981(1,6);7,962(2,1);7,961(2,1);7,830(2,5);7,809(1,9);4,038(0,5);4,021(0,5);3,954(2,1);3,949(2,2);3,936(2,2);3,931(2,1);3,310(33,4);3,272(14,0);2,523(0,7);2,518(1,0);2,510(12,1);2,505(26,1);2,500(36,1);2,496(25,1);2,491(11,2);2,313(10,5);2,145(16,0);1,988(2,5);1,193(0,8);1,175(1,6);1,157(0,9);1,141(0,8);0,858(0,9);0,534(1,7);0,530(2,0);0,526(0,8);0,519(0,7);0,514(1,8);0,510(1,7);0,292(0,8);0,285(1,0);0,281(1,6);0,276(1,5);0,273(1,1);0,269(1,8);0,264(1,1);0,258(0,7);0,000(16,7) |
| Beispiel 6-1: ¹H-NMR(400,0 MHz, d₆-DMSO): δ = 11,503(0,9);7,768(0,7);7,747(2,1);7,726(3,1);7,705(1,1);3,904(16,0);3,309(36,0);2,523(0,6);2,518(0,9);2,510(11,4);2,505(24,6);2,500(34,2);2,496(23,7);2,491(10,5);2,387(18,8);2,110(12,6);2,072(5,8);0,000(0,6) |
| Beispiel 6-5: ¹H-NMR(400,0 MHz, d₆-DMSO): δ = 11,500(1,4);7,763(1,0);7,742(2,8);7,722(4,1);7,702(1,4);4,183(1,4);4,165(4,6);4,148(4,7);4,130(1,4);3,307(23,9);2,523(0,9);2,518(1,3);2,509(15,4);2,505(32,8);2,500(45,4);2,496(31,5);2,491(14,1);2,387(23,6);2,111(16,0);1,266(5,2);1,248(11,6);1,230(5,1);0,008(1,1);0,000(33,7);-0,009(0,9) |
| Beispiel 6-8: ¹H-NMR(400,0 MHz, d₆-DMSO): δ = 11,500(1,3);7,761(1,0);7,740(3,0);7,720(4,2);7,700(1,4);3,966(2,9);3,949(2,9);3,309(49,4);2,523(0,8);2,518(1,1);2,510(13,2);2,505(27,7);2,500(38,2);2,496(26,7);2,491(11,9);2,386(23,5);2,129(16,0);2,072(0,7);1,163(0,5);1,143(0,9);1,122(0,5);0,542(0,7);0,532(2,1);0,527(2,2);0,522(1,0);0,517(1,0);0,511(2,3);0,507(2,0);0,497(0,9);0,306(0,9);0,296(2,4);0,292(2,4);0,284(2,1);0,280(2,5);0,269(0,6) |
| Beispiel 6-9: ¹H-NMR(400,0 MHz, d₆-DMSO): δ = 11,519(1,2);7,802(1,2);7,781(2,6);7,751(3,9);7,730(1,7);4,836(0,9);4,813(2,9);4,790(3,0);4,768(1,0);3,309(53,9);2,523(0,9);2,518(1,2);2,510(13,7);2,505(29,1);2,501(40,3);2,496(28,2);2,491(12,6);2,386(24,4);2,191(16,0) |
| Beispiel 6-15: ¹H-NMR(400,0 MHz, d₆-DMSO): δ = 11,638(1,5);8,116(1,5);8,096(2,3);8,028(1,5);8,008(1,0);5,753(0,7);3,918(16,0);3,349(13,6);3,311(30,4);2,510(17,4);2,505(33,0);2,501(43,3);2,496(30,8);2,492(14,9);2,400(19,6);2,327(0,6);2,146(9,4);0,000(2,9) |
| Beispiel 6-19: ¹H-NMR(400,0 MHz, CDCl₃): δ = 8,070(1,5);8,050(1,9);7,849(1,0);7,829(0,8);7,263(32,8);4,238(0,9);4,220(3,1);4,203(3,3);4,185(1,1);3,236(16,0);2,510(13,1);2,233(12,6);1,572(4,3);1,346(4,4);1,328(9,3);1,310(4,2);0,000(12,7) |
| Beispiel 6-22: ¹H-NMR(400,0 MHz, d₆-DMSO): δ = 11,640(2,7);8,112(2,6);8,092(4,1);8,022(2,6);8,002(1,7);5,751(12,4);3,968(4,8);3,951(5,3);3,341(37,1);3,337(39,9);2,523(1,6);2,509(24,3);2,505(50,1);2,500(68,0);2,496(48,4);2,491(23,0);2,399(35,3);2,163(16,0);1,164(0,8);1,162(0,8);1,156(0,8);1,152(0,6);1,144(1,4);1,136(0,7);1,132(0,8);1,126(0,8);1,124(0,9);0,552(0,7);0,550(0,8);0,541(3,1);0,537(3,4);0,530(1,7);0,527(1,4);0,521(3,2);0,517(3,1);0,509(1,0);0,507(0,9);0,300(1,3);0,289(3,3);0,285(2,8);0,278(3,3);0,274(2,2);0,266(1,1);0,000(2,3) |
| Beispiel 6-67: ¹H-NMR(400,0 MHz, CDCl₃): δ = 7,903(0,9); 7,882(1,0); 7,691(1,4); 7,670(1,1); 7,261(42,6);3,960(16,0); 3,199(14,3); 2,509(14,4); 2,427(8,8); 2,175(13,3); 1,643(1,2); 0,000(16,2) |
| Beispiel 6-71: ¹H-NMR(400,0 MHz, CDCl₃): δ = 8,375(0,9);7,889(1,6);7,869(2,0);7,684(2,0);7,663(1,7);7,261(38,3);4,215(1,4);4,197(4,2);4,179(4,3);4,162(1,5);3,184(15,8);2,509(16,0);2,419(11,8);2,175(16,0);1,602(2,6);1,332(4,5);1,314(9,1);1,297(4,4);0,008(1,1);0,000(13,9);-0,008(0,8) |
| Beispiel 6-74: ¹H-NMR(400,0 MHz, d₆-DMSO): δ = 11,453(1,5);8,312(4,2);7,974(1,4);7,973(1,4);7,954(2,0);7,953(2,0);7,854(1,9);7,834(1,3);3,953(2,1);3,948(2,2);3,935(2,2);3,930(2,1);3,309(28,5);3,269(14,0);2,523(0,7);2,518(1,1);2,510(13,5);2,505(29,3);2,500(40,7);2,496(28,3);2,491(12,6);2,395(22,6);2,306(10,2);2,144(16,0);1,141(0,8);0,534(1,7);0,530(2,0);0,526(0,8);0,519(0,7);0,514(1,9);0,510(1,8);0,291(0,7);0,285(1,0);0,281(1,6);0,276(1,5);0,273(1,1);0,269(1,8);0,264(1,1);0,258(0,7);0,008(0,9);0,000(31,0);-0,009(0,9) |
| Beispiel 7-1: ¹H-NMR(400,0 MHz, d₆-DMSO): δ = 7,695(2,9);7,691(3,2);3,900(16,0);3,308(7,7);2,518(0,7);2,510(7,8);2,505(16,5);2,500(22,8);2,496(15,9);2,491(7,3);2,481(8,7);2,098(14,2);0,008(0,5);0,000(17,7);-0,009(0,5) |
| Beispiel 7-5: ¹H-NMR(400,0 MHz, d₆-DMSO): □= 7,690(3,3);7,687(3,5);4,178(1,2);4,161(4,1);4,143(4,1);4,126(1,3);3,306(17,8);2,523(0,7);2,518(1,0);2,509(11,9);2,505(25,5);2,500(35,5);2,496(24,7);2,491(11,2);2,480(9,0);2,100(16,0);1,262(4,7);1,245(10,6);1,227(4,5);0,000(15,1) |
| Beispiel 7-8: ¹H-NMR(400,0 MHz, d₆-DMSO): δ = 7,705(3,7);7,702(3,9);3,978(3,7);3,961(3,7);3,329(51,6);2,540(0,6);2,535(1,0);2,526(12,8);2,522(27,3);2,517(38,1);2,512(26,7);2,508(12,1);2,496(9,9);2,133(16,0);1,156(0,8);0,556(0,6);0,545(1,8);0,541(2,0);0,536(0,9);0,531(0,9);0,525(2,0);0,521(1,8);0,510(0,8);0,320(0,8);0,310(2,1);0,306(2,1);0,298(1,8);0,294(2,2);0,283(0,5) |
| Beispiel 7-9: ¹H-NMR(400,0 MHz, d₆-DMSO): δ = 12,301(0,6);7,749(0,6);7,728(2,3);7,716(2,6);7,695(0,6);4,833(0,8);4,810(2,6);4,788(2,7);4,765(0,9);3,309(42,2);2,523(1,1);2,518(1,6);2,510(17,3);2,505(36,2);2,501(49,7);2,496(34,7);2,491 (15,8);2,480(9,3);2,179(16,0);0,000(0,9) |
| Beispiel 7-15: ¹H-NMR(400,0 MHz, CDCl₃): δ = 8,080(1,7);8,060(2,0);7,840(1,6);7,820(1,3);7,261(33,6);3,816(16,0);3,081(15,6);2,525(12,3);2,321(14,6);1,286(0,8);1,256(0,7);0,000(13,1) |
| Beispiel 7-19: ¹H-NMR(400,0 MHz, CDCl₃): δ = 8,100(1,1);8,080(1,3);7,880(1,0);7,859(0,8);7,260(74,5);4,235(0,7);4,234(0,7);4,218(2,3);4,216(2,3);4,200(2,4);4,199(2,3);4,182(0,8);3,234(14,8);2,526(12,2);2,248(16,0);1,343(4,4);1,326(9,4);1,308(4,2);0,008(1,0);0,000(28,6);-0,009(0,7) |
| Beispiel 7-22: ¹H-NMR(400,0 MHz, d₆-DMSO): δ = 12,441(0,9);8,083(1,7);8,063(2,4);8,043(1,4);8,022(3,0);7,991(2,7);7,970(2,4);7,968(2,0);7,947(1,4);3,966(3,2);3,961(3,6);3,948(3,6);3,943(3,5);3,935(1,4);3,378(0,6);3,333(22,7);3,323(18,8);3,312(14,4);2,523(1,8);2,518(2,7);2,510(29,3);2,505(60,1);2,500(81,6);2,496(58,6);2,491(29,8);2,327(0,5);2,149(16,0);2,134(9,3);1,880(0,8);1,161(0,7);1,159(0,8);1,154(0,8);1,149(0,8);1,141(1,3);1,137(0,9);1,129(0,9);1,124(0,9);1,121(0,9);0,547(0,8);0,539(2,7);0,534(3,7);0,530(2,3);0,527(2,1);0,519(3,1);0,514(3,4);0,298(1,2);0,286(3,4);0,283(3,3);0,275(3,4);0,264(1,1);0,000(3,3) |
| Beispiel 7-71: ¹H-NMR(400,0 MHz, CDCl₃): δ = 8,120(1,4);8,099(2,4);8,052(1,9);8,051(1,9);8,031(1,1);8,030(1,1);7,261(34,5);4,237(1,1);4,219(3,5);4,201(3,7);4,184(1,2);3,208(15,5);2,561(10,1);2,229(16,0);1,350(4,3);1,333(9,1);1,315(4,2);0,000(12,8) |
| Beispiel 7-74: ¹H-NMR(400,0 MHz, d₆-DMSO): δ = 8,312(5,3);7,947(1,5);7,927(1,9);7,799(1,5);7,778(1,1);3,951(2,0);3,942(2,1);3,933(2,2);3,925(2,0);3,311(32,9);3,262(13,8);3,246(1,5);2,523(0,8);2,518(1,2);2,510(15,0);2,505(32,5);2,500(45,2);2,496(31,7);2,491(14,9);2,486(13,2);2,373(1,1);2,279(8,3);2,133(16,0);2,116(1,7);1,139(0,8);0,532(1,8);0,528(2,1);0,523(0,9);0,516(0,8);0,512(1,9);0,507(1,9);0,289(0,8);0,282(1,1);0,278(1,8);0,273(1,7);0,270(1,3);0,267(2,1);0,262(1,2);0,255(0,7);0,000(8,8) |
| Beispiel 8-5: ¹H-NMR(400,0 MHz, CDCl₃): δ = 8,163(2,1); 8,142(2,5); 7,873(1,9); 7,852(1,6); 7,261(38,1);4,141(12,7); 3,823(15,7); 3,100(16,0); 2,340(13,5); 1,285(0,5); 0,000(14,4) |
| Beispiel 8-25: ¹H-NMR(400,0 MHz, CDCl₃): δ = 8,092(1,1);8,071(1,3);7,886(1,4);7,866(1,2);7,260(51,7);7,256(0,6);4,129(9,3);3,816(16,0);3,064(15,6);2,422(9,1);2,293(14,5);0,008(0,6);0,000(19,5);-0,009(0,5) |
| Beispiel 8-26: ¹H-NMR(400,0 MHz, CDCl₃): δ= 8,090(1,1);8,070(1,3);7,855(1,2);7,835(1,0);7,268(0,6);7,267(0,6);7,2663(0,7);7,2655(0,9);7,260(90,0);7,253(0,6);5,299(1,3);4,135(9,1);4,122(0,8);4,113(1,4);4,095(1,4);4,090(0,6);4,078(0,5);4,072(1,4);4,054(1,4);4,045(0,7);4,028(0,7);3,085(16,0);3,063(1,8);2,430(1,2);2,409(9,1);2,284(15,5);2,263(1,7);2,005(13,3);1,197(4,1);1,185(0,7);1,179(8,7);1,168(1,1);1,162(4,0);0,008(1,1);0,005(0,5);0,000(34,5);-0,009(0,9) |
| Beispiel 9-5: ¹H-NMR(400,0 MHz, CDCl₃): δ = 8,149(2,1); 8,128(2,5); 7,858(1,7); 7,838(1,5); 7,262(25,9); 4,530(0,8); 4,512(2,7); 4,493(2,8); 4,475(0,9); 3,984(1,4); 3,817(15,6); 3,267(1,3); 3,098(16,0); 2,338(12,6); 2,258(1,1); 1,652(3,5); 1,642(0,5); 1,633(8,0); 1,624(0,8); 1,615(3,5); 0,000(9,7) |
| Beispiel 9-25: ¹H-NMR(400,0 MHz, CDCl₃): δ = 8,081(1,6);8,061(2,0);7,870(1,8);7,849(1,5);7,262(36,1);4,507(1,0);4,489(3,0);4,471(3,1);4,453(1,0);3,966(0,6);3,815(16,0);3,064(15,7);2,420(11,4);2,291(15,0);2,200(0,6);1,661(3,7);1,642(8,0);1,624(3,6);0,000(13,5) |
| Beispiel 10-5: ¹H-NMR(400,0 MHz, CDCl₃): δ = 8,150(2,0); 8,130(2,3); 7,852(2,0); 7,832(1,7); 7,261(29,5); 4,452(1,4); 4,434(2,2); 4,415(1,4); 3,820(16,0); 3,097(15,6); 2,337(13,9); 2,065(1,1); 2,047(2,0); 2,028(2,1); 2,010(1,2); 1,285(0,8); 1,256(0,7); 1,020(1,0); 1,012(3,8); 1,003(1,2); 0,994(7,7); 0,975(3,6); 0,000(11,0) |
| Beispiel 11-5: ¹H-NMR(400,0 MHz, CDCl₃): δ = 8,117(2,4); 8,097(2,7); 7,782(1,7); 7,762(1,6); 7,406(0,8); 7,262(22,7); 3,887(12,1); 3,851(16,0); 3,100(16,0); 2,324(13,8); 0,000(8,4) |
| Beispiel 12-25: ¹H-NMR(400,0 MHz, CDCl₃): δ=7,840(0,9);7,819(1,1);7,677(1,9);7,656(1,5);7,261(36,6);3,956(16,0);3,191(14,4);2,428(9,7);2,161(12,2);1,591(2,5);0,000(13,6) |
| Beispiel 12-26: 1H-NMR(400,0 MHz, CDCl₃): δ = 8,371(0,6); 7,871(1,1); 7,850(1,4); 7,675(1,8); 7,654(1,5); 7,262(33,5); 4,133(0,7); 4,116(0,7); 4,106(1,4); 4,089(1,4); 4,058(1,4); 4,040(1,4); 4,031(0,7); 4,013(0,7); 3,056(16,0); 2,365(10,6); 2,209(13,5); 1,627(1,5); 1,195(4,2); 1,178(8,9); 1,160(4,0); 0,000(13,0) |
| Beispiel 13-5: ¹H-NMR(400,0 MHz, CDCl₃): δ = 8,073(1,1); 8,053(1,4); 7,840(0,8); 7,820(0,6); 7,262(18,3); 3,831(13,3); 3,079(16,0); 2,514(8,6); 2,467(0,6); 2,302(10,6); 1,581(1,2); 0,000(7,1) |
| Beispiel 13-25: ¹H-NMR(400,0 MHz, CDCl₃): δ = 7,932(1,1);7,912(1,3);7,685(1,3);7,665(1,1);7,261(37,8);3,812(16,0);3,040(15,5);2,514(10,8);2,372(9,0);2,242(13,9);1,607(0,8);0,000(13,7) |
| Beispiel 13-26: ¹H-NMR(400,0 MHz, CDCl₃): δ = 8,275(0,9); 7,930(1,7); 7,910(1,9); 7,683(1,8); 7,663(1,5); 7,261(35,5); 4,136(0,8); 4,127(0,6); 4,119(0,9); 4,109(1,5); 4,101(0,5); 4,092(1,5); 4,080(0,7); 4,074(0,7); 4,063(1,5); 4,053(0,6); 4,045(1,6); 4,036(0,9); 4,028(0,6); 4,018(0,8); 3,062(16,0); 2,516(12,0); 2,364(11,9); 2,231(15,0); 1,599(1,7); 1,200(4,4); 1,182(8,7); 1,165(4,2); 0,000(12,9); -0,008(0,8) |
| Beispiel 14-5: ¹H-NMR(400,0 MHz, CDCl₃): δ = 8,080(1,7); 8,060(2,0); 7,840(1,5); 7,820(1,3); 7,261(33,6); 3,816(16,0); 3,081(15,6); 2,525(12,3); 2,321(14,6); 1,286(0,7); 1,256(0,7); 0,000(13,1) |

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gew.-Teile einer Verbindung der Formel (I),
   5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-15, 1-71, 1-74, 2-15, 2-19, 2-67, 2-71, 2-74, 2-136, 3-15, 3-19, 4-74, 5-15, 5-67, 5-71, 5-74, 6-15, 6-19, 6-67, 6-71, 6-74, 7-15, 7-19, 7-71, 7-74, 8-1, 8-25, 9-5, 9-25, 10-5 und 12-25 bei einer Aufwandmenge von 320 g/ha jeweils eine mindestens 80 %-ige Wirkung gegen Abutilon theophrasti und Veronica persica.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 1-15, 1-71, 1-74, 2-15, 2-19, 2-67, 2-71, 2-74, 2-136, 3-15, 3-19, 4-19, 4-136, 5-15, 7-15, 7-19, 7-74, 8-1, 8-25, 8-26, 9-5, 9-25, 10-5 und 10-5 bei einer Aufwandmenge von 80 g/ha jeweils eine mindestens 80 %-ige Wirkung gegen Abutilon theophrasti und Veronica persica.

### 3. Vergleichsversuche

Zusätzlich wurden Vergleichsversuche zwischen erfindungsgemäßen und den struktrurell ähnlichsten aus WO 2013/064459 A1 (D1) bekannten Verbindungen unter den oben genannten Bedingungen an unterschiedlichen Schadpflanzen durchgeführt. Die in nachfolgenden Tabellen aufgeführten Ergebnisse zeigen die Überlegenheit dieser erfindungsgemäßen Verbindungen.

Die hier verwendeten Abkürzungen bedeuten:

| | | | |
|---|---|---|---|
| ALOMY | Alopecurus myosuroides | AVEFA | Avena fatua |
| CYPES | Cyperus esculentus | ECHCG | Echinochloa crus galli |
| MATIN | Matricaria inodora | POLCO | Polygonum convolvulus |
| STEME | Stellaria media | VIOTR | Viola tricolor |
| a.i. | active ingredient (Wirkstoff) | | |

Vergleichsversuche im Vorauflauf

| Verbindung Nr. | Dosierung [g a.i. / ha] | Herbizide Wirkung gegen | | |
|---|---|---|---|---|
| | | AVEFA | CYPES | VIOTR |
| 1-5, erfindungsgemäß | 320 | 90% | 90% | 100% |
| 1-2, aus D1 | 320 | 0% | 40% | 0% |

| Verbindung Nr. | Dosierung [g a.i. / ha] | Herbizide Wirkung gegen | | |
|---|---|---|---|---|
| | | AVEFA | STEME | VIOTR |
| 1-8, erfindungsgemäß | 320 | 90% | 100% | 100% |
| 1-4, aus D1 | 320 | 0% | 20% | 50% |

| Verbindung Nr. | Dosierung [g a.i. / ha] | Herbizide Wirkung gegen | | |
|---|---|---|---|---|
| | | AVEFA | CYPES | VIOTR |
| 2-1, erfindungsgemäß | 320 | 100% | 100% | 100% |
| 1-40, aus D1 | 320 | 50% | 30% | 50% |

| Verbindung Nr. | Dosierung [g a.i. / ha] | Herbizide Wirkung gegen | | |
|---|---|---|---|---|
| | | AVEFA | CYPES | VIOTR |
| 3-1, erfindungsgemäß | 320 | 80% | 100% | 100% |
| 1-79, aus D1 | 320 | 50% | 30% | 40% |

| Verbindung Nr. | Dosierung [g a.i. / ha] | Herbizide Wirkung gegen | | |
|---|---|---|---|---|
| | | AVEFA | STEME | VIOTR |
| 3-5, erfindungsgemäß | 320 | 70% | 100% | 100% |
| 1-80, aus D1 | 320 | 10% | 60% | 20% |

| Verbindung Nr. | Dosierung [g a.i. / ha] | Herbizide Wirkung gegen | | |
|---|---|---|---|---|
| | | CYPES | ECHCG | VIOTR |
| 3-19, erfindungsgemäß | 80 | 80% | 90% | 100% |
| 1-93, aus D1 | 80 | 20% | 40% | 20% |

| Verbindung Nr. | Dosierung [g a.i. / ha] | Herbizide Wirkung gegen | | |
|---|---|---|---|---|
| | | ECHCG | STEME | VIOTR |
| 4-5, erfindungsgemäß | 320 | 90% | 80% | 100% |
| 2-2, aus D1 | 320 | 0% | 40% | 0% |

| Verbindung Nr. | Dosierung [g a.i. / ha] | Herbizide Wirkung gegen | | |
|---|---|---|---|---|
| | | ECHCG | POLCO | STEME |
| 6-1, erfindungsgemäß | 320 | 60% | 40% | 60% |
| 3-1, aus D1 | 320 | 0% | 0% | 0% |

| Verbindung Nr. | Dosierung [g a.i. / ha] | Herbizide Wirkung gegen | | |
|---|---|---|---|---|
| | | AVEFA | CYPES | VIOTR |
| 6-19, erfindungsgemäß | 320 | 30% | 80% | 100% |
| 3-15, aus D1 | 320 | 0% | 20% | 60% |

| Verbindung Nr. | Dosierung [g a.i. / ha] | Herbizide Wirkung gegen | | |
|---|---|---|---|---|
| | | AVEFA | POLCO | VIOTR |
| 10-6, erfindungsgemäß | 320 | 40% | 60% | 100% |
| 1-93, aus D1 | 320 | 0% | 20% | 30% |

| Verbindung Nr. | Dosierung [g a.i. / ha] | Herbizide Wirkung gegen | | |
|---|---|---|---|---|
| | | AVEFA | CYPES | VIOTR |
| 13-6, erfindungsgemäß | 320 | 60% | 60% | 100% |
| 3-15, aus D1 | 320 | 0% | 20% | 60% |

Vergleichsversuche im Nachauflauf

| Verbindung Nr. | Dosierung [g a.i. / ha] | Herbizide Wirkung gegen | |
|---|---|---|---|
| | | MATIN | VIOTR |
| 1-5, erfindungsgemäß | 20 | 90% | 90% |
| 1-2, aus D1 | 20 | 60% | 60% |

| Verbindung Nr. | Dosierung [g a.i. / ha] | Herbizide Wirkung gegen | |
|---|---|---|---|
| | | ALOMY | MATIN |
| 1-8, erfindungsgemäß | 80 | 70% | 100% |
| 1-4, aus D1 | 80 | 10% | 70% |

| Verbindung Nr. | Dosierung [g a.i. / ha] | Herbizide Wirkung gegen | |
|---|---|---|---|
| | | ALOMY | VIOTR |
| 2-1, erfindungsgemäß | 80 | 60% | 100% |
| 1-40, aus D1 | 80 | 40% | 60% |

| Verbindung Nr. | Dosierung [g a.i. / ha] | Herbizide Wirkung gegen | |
|---|---|---|---|
| | | MATIN | VIOTR |
| 3-1, erfindungsgemäß | 20 | 80% | 100% |
| 1-79, aus D1 | 20 | 60% | 30% |

| Verbindung Nr. | Dosierung [g a.i. / ha] | Herbizide Wirkung gegen | |
|---|---|---|---|
| | | MATIN | VIOTR |
| 3-5, erfindungsgemäß | 80 | 90% | 100% |
| 1-80, aus D1 | 80 | 60% | 60% |

| Verbindung Nr. | Dosierung [g a.i. / ha] | Herbizide Wirkung gegen | |
|---|---|---|---|
| | | MATIN | VIOTR |
| 3-8, erfindungsgemäß | 80 | 70% | 80% |
| 1-82, aus D1 | 80 | 20% | 20% |

| Verbindung Nr. | Dosierung [g a.i. / ha] | Herbizide Wirkung gegen | |
|---|---|---|---|
| | | MATIN | VIOTR |
| 3-15, erfindungsgemäß | 20 | 90% | 90% |
| 1-92, aus D1 | 20 | 70% | 60% |

| Verbindung Nr. | Dosierung [g a.i. / ha] | Herbizide Wirkung gegen | |
|---|---|---|---|
| | | ALOMY | VIOTR |
| 3-19, erfindungsgemäß | 80 | 50% | 100% |
| 1-93, aus D1 | 80 | 0% | 80% |

| Verbindung Nr. | Dosierung [g a.i. / ha] | Herbizide Wirkung gegen | |
|---|---|---|---|
| | | ALOMY | VIOTR |
| 4-5, erfindungsgemäß | 80 | 30% | 90% |
| 2-2, aus D1 | 80 | 0% | 40% |

| Verbindung Nr. | Dosierung [g a.i. / ha] | Herbizide Wirkung gegen | |
|---|---|---|---|
| | | ALOMY | VIOTR |
| 4-19, erfindungsgemäß | 20 | 80% | 100% |
| 2-15, aus D1 | 20 | 40% | 80% |

| Verbindung Nr. | Dosierung [g a.i. / ha] | Herbizide Wirkung gegen | |
|---|---|---|---|
| | | MATIN | VIOTR |
| 6-15, erfindungsgemäß | 80 | 80% | 100% |
| 3-14, aus D1 | 80 | 0% | 60% |

| Verbindung Nr. | Dosierung [g a.i. / ha] | Herbizide Wirkung gegen | |
|---|---|---|---|
| | | MATIN | VIOTR |
| 6-19, erfindungsgemäß | 80 | 70% | 100% |
| 3-15, aus D1 | 80 | 30% | 60% |

| Verbindung Nr. | Dosierung [g a.i. / ha] | Herbizide Wirkung gegen | |
|---|---|---|---|
| | | ALOMY | MATIN |
| 10-5, erfindungsgemäß | 80 | 60% | 100% |
| 1-92, aus D1 | 80 | 10% | 80% |

| Verbindung Nr. | Dosierung [g a.i. / ha] | Herbizide Wirkung gegen | |
|---|---|---|---|
| | | MATIN | VIOTR |
| 10-6, erfindungsgemäß | 20 | 90% | 100% |
| 1-93, aus D1 | 20 | 40% | 60% |

| Verbindung Nr. | Dosierung [g a.i. / ha] | Herbizide Wirkung gegen | |
|---|---|---|---|
| | | MATIN | VIOTR |
| 13-5, erfindungsgemäß | 80 | 60% | 90% |
| 3-14, aus D1 | 80 | 0% | 60% |

| Verbindung Nr. | Dosierung [g a.i. / ha] | Herbizide Wirkung gegen | |
|---|---|---|---|
| | | MATIN | VIOTR |
| 13-6, erfindungsgemäß | 80 | 60% | 100% |
| 3-14, aus D1 | 80 | 30% | 60% |

## Patentansprüche

1. Substituierte Ketoxim-benzoylamide der Formel (I) 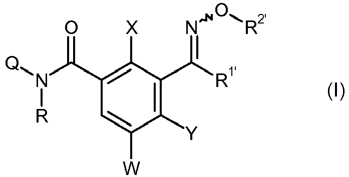 worin die Symbole und Indizes folgende Bedeutungen haben:
Q bedeutet einen Rest Q1, Q2, Q3 oder Q4, 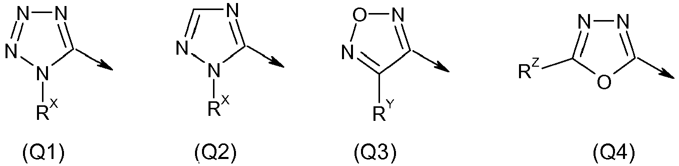
R bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, R¹(O)CO-(C₁-C₆)-Alkyl, R¹O(O)C-O-(C₁-C₆)-Alkyloxy, R²(O)₂SO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²(O)ₙS-(C₁-C₆)-Alkyl, R¹O(O)2S-(C₁-C₆)-Alkyl, (R¹)₂N(O)₂S-(C₁-C₆)-Alkyl, R¹(O)C, R¹O(O)C, (R¹)₂N(O)C, R¹O, (R¹)₂N, R¹O(O)C(R¹)N, (R¹)₂N(O)C(R¹)N, R²(O)₂S oder jeweils durch s Reste aus der Gruppe bestehend aus Methyl, Ethyl, Methoxy, Nitro, Trifluormethyl und Halogen substituiertes Benzyl,
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl oder Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl oder Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R³ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁴ bedeutet (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet Wasserstoff oder (C₁-C₄)-Alkyl,
R⁶ bedeutet (C₁-C₄)-Alkyl,
R⁷ bedeutet Acetoxy, Acetamido, N-Methylacetamido, Benzoyloxy, Benzamido, N-Methylbenzamido, Methoxycarbonyl, Ethoxycarbonyl, Benzoyl, Methylcarbonyl, Piperidinylcarbonyl, Morpholinylcarbonyl, Trifluormethylcarbonyl, Aminocarbonyl, Methylaminocarbonyl, Dimethylaminocarbonyl, Methylsulfenyl, Methylsulfinyl, Methylsulfonyl, (C₃-C₆)-Cycloalkyl oder jeweils durch s Reste aus der Gruppe bestehend aus Methyl, Ethyl, Methoxy, Trifluormethyl und Halogen substituiertes Heteroaryl oder Heterocyclyl,
R⁸ bedeutet (C₁-C₆)-Alkyl, Halogen-(C₁-C₆)-alkyl, (C₁-C₆)-Alkyl-O-(C₁-C₆)-alkyl, (C₂-C₆)-Alkenyl oder (C₃-C₆)-Alkinyl,
R⁹ bedeutet (C₁-C₆)-Alkyl, Halogen-(C₁-C₆)-alkyl, (C₂-C₆)-Alkenyl oder (C₃-C₆)-Alkinyl,
R¹⁰ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl oder (C₃-C₆)-Cycloalkyl-(C₁-C₆)-alkyl,
R¹¹ bedeutet (C₁-C₆)-Alkyl, (C₂-C₆)-Alkenyl oder (C₂-C₆)-Alkinyl,
R^{1'} bedeutet Cyano, (C₁-C₆)-Alkyl, (C₃-C₆)-Cycloalkyl, Halogen-(C₁-C₆)-alkyl, OR⁸, SR⁸, NR⁸R⁹,
R^{2'} bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, wobei die letzten sechs Reste jeweils durch s Reste aus der Gruppe bestehend aus Cyano, Halogen, Nitro, Rhodano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind,
oder R^{2'} bedeutet Phenyl, Phenyl-(C₁-C₆)-alkyl, Heteroaryl, (C₁-C₆)-Alkyl-heteroaryl, Heterocyclyl, (C₁-C₆)-Alkyl-heterocyclyl, (C₁-C₆)-Alkyl-O-heteroaryl, (C₁-C₆)-Alkyl-O-heterocyclyl, (C₁-C₆)-Alkyl-NR¹⁰-heteroaryl, (C₁-C₆)-Alkyl-NR¹⁰-heterocyclyl, wobei die zehn letztgenannten Reste jeweils durch s Reste aus der Gruppe bestehend aus Cyano, Halogen, Nitro, Rhodano, (C₁-C₆)-Alkyl, Halogen-(C₁-C₆)-alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, wobei die sechs vorstehend genannten Reste jeweils durch s Reste aus der Gruppe bestehend aus Nitro, Cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-Cycloalkyl, Heteroaryl, Heterocyclyl und Phenyl substituiert sind, wobei die vier letztgenannten Reste selber wieder durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
oder R^{X} bedeutet (C₃-C₇)-Cycloalkyl, Heteroaryl, Heterocyclyl oder Phenyl, wobei die vier vorstehend genannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl-S(O)ₙ, (C₁-C₆)-Alkoxy, Halogen-(C₁-C₆)-alkoxy und (C₁-C₆)-Alkoxy-(C₁-C₄)-alkyl substituiert sind,
R^{Y} bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, (C₁-C₆)-Alkoxy, Halogen-(C₁-C₆)-alkoxy, (C₂-C₆)-Alkenyloxy, (C₂-C₆)-Alkinyloxy, Cyano, Nitro, Methylsulfenyl, Methylsulfinyl, Methylsulfonyl, Acetylamino, Benzoylamino, Methoxycarbonyl, Ethoxycarbonyl, Methoxycarbonylmethyl, Ethoxycarbonylmethyl, Benzoyl, Methylcarbonyl, Piperidinylcarbonyl, Trifluormethylcarbonyl, Halogen, Amino, Aminocarbonyl, Methylaminocarbonyl, Dimethylaminocarbonyl, Methoxymethyl, oder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl und wobei Heterocyclyl n Oxogruppen trägt,
R^{Z} bedeutet Wasserstoff, (C₁-C₆)-Alkyl, R¹O-(C₁-C₆)-Alkyl, R⁷CH₂, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, R¹O, R¹(H)N, Methoxycarbonyl, Ethoxycarbonyl, Methylcarbonyl, Dimethylamino, Trifluormethylcarbonyl, Acetylamino, Methylsulfenyl, Methylsulfinyl, Methylsulfonyl oder jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl, wobei Heterocyclyl n Oxogruppen trägt,
W bedeutet Wasserstoff, Halogen, Nitro, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, Halogen-(C₃-C₇)-cycloalkyl, (C₁-C₆)-Alkoxy, Halogen-(C₁-C₆)-alkoxy, (C₁-C₆)-Alkyl-(O)ₙS-, (C₁-C₆)-Halogenalkyl-(O)ₙS-, (C₁-C₆)-Alkoxy-(C₁-C₄)-alkyl, (C₁-C₆)-Alkoxy-(C₁-C₄)-halogenalkyl, R¹(O)C, R¹(R¹ON=)C, R¹O(O)C, (R¹)₂N, R¹(O)C(R¹)N oder R²(O)₂S(R¹)N,
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R¹O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R¹O(O)CO, (R¹)₂N(O)CO, (R¹)₂N, R¹(O)C(R¹)N, R²(O)₂S(R¹)N, R¹O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R¹O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R¹O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R¹O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R¹O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R¹O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R¹O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R¹O(O)CO, (R¹)₂N(O)CO, (R¹)₂N, R¹(O)C(R¹)N, R²(O)₂S(R¹)N, R¹O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R¹O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R¹⁰(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R¹O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R¹O(O)C(R¹)N-(C₁-C₆)-Alkyl, (R¹)₂N(O)C(R¹)N-(C₁-C₆)-Alkyl, R¹O(O)2S(R¹)N-(C₁-C₆)-Alkyl, (R¹)₂N(O)2S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R¹O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
n bedeutet 0, 1 oder 2,
s bedeutet 0, 1, 2 oder 3.

2. Substituierte Ketoxim-benzoylamide der Formel (I) nach Anspruch 1, worin die Symbole und Indices folgende Bedeutungen haben:
Q bedeutet einen Rest Q1, Q2, Q3 oder Q4, 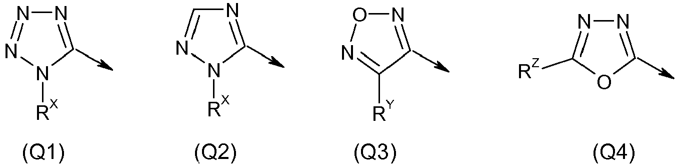
R bedeutet Wasserstoff, (C₁-C₆)-Alkyl, Halogen-(C₁-C₆)-alkyl, R¹(O)C-(C₁-C₆)-Alkyl, R¹O(O)C-(C₁-C₆)-Alkyl, R¹O(O)C-O-(C₁-C₆)-Alkyloxy, R¹O-(C₁-C₆)-Alkyl, R¹(O)C, R¹O(O)C, R¹O, R²(O)₂S oder jeweils durch s Reste aus der Gruppe bestehend aus Methyl, Ethyl, Methoxy, Nitro, Trifluormethyl und Halogen substituiertes Benzyl,
R¹ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkenyl, (C₁-C₆)-Alkyl-O-(C₁-C₆)-alkyl, Phenyl, Heteroaryl oder Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₁-C₆)-Alkyl-O-(C₁-C₆)-alkyl, Phenyl, Heteroaryl oder Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R³ bedeutet Wasserstoff, (C₁-C₆)-Alkyl, Halogen-(C₁-C₆)-alkyl, (C₃-C₆)-Cycloalkyl oder Phenyl,
R⁴ bedeutet (C₁-C₆)-Alkyl, Halogen-(C₁-C₆)-alkyl, (C₃-C₆)-Cycloalkyl oder Phenyl,
R⁵ bedeutet Wasserstoff oder (C₁-C₄)-Alkyl,
R⁶ bedeutet (C₁-C₄)-Alkyl,
R⁷ bedeutet Acetoxy, Benzoyloxy, Methoxycarbonyl, Ethoxycarbonyl, Methylcarbonyl, Trifluormethylcarbonyl, Methylsulfenyl, Methylsulfinyl, Methylsulfonyl, (C₃-C₆)-Cycloalkyl oder jeweils durch s Reste aus der Gruppe bestehend aus Methyl, Ethyl, Methoxy, Trifluormethyl und Halogen substituiertes Heteroaryl oder Heterocyclyl,
R⁸ bedeutet (C₁-C₆)-Alkyl, Halogen-(C₁-C₆)-alkyl, (C₁-C₆)-Alkyl-O-(C₁-C₆)-alkyl, (C₂-C₆)-Alkenyl oder (C₃-C₆)-Alkinyl,
R⁹ bedeutet (C₁-C₆)-Alkyl oder Halogen-(C₁-C₆)-alkyl,
R¹⁰ bedeutet Wasserstoff, (C₁-C₆)-Alkyl, Halogen-(C₁-C₆)-alkyl oder (C₃-C₆)-Cycloalkyl,
R¹¹ bedeutet (C₁-C₆)-Alkyl,
R^{1'} bedeutet (C₁-C₆)-Alkyl, (C₃-C₆)-Cycloalkyl, Halogen-(C₁-C₆)-alkyl, OR⁸, SR⁸, NR⁸R⁹,
R^{2'} bedeutet Wasserstoff, (C₁-C₆)-Alkyl, (C₂-C₆)-Alkenyl, (C₂-C₆)-Alkinyl, (C₃-C₆)-Cycloalkyl, (C₃-C₆)-Cycloalkenyl, (C₃-C₆)-Cycloalkyl-(C₁-C₆)-alkyl, wobei die letzten sechs Reste jeweils durch s Reste aus der Gruppe bestehend aus Cyano, Halogen, OR¹⁰, S(O)ₙR¹¹, N(R¹⁰)₂, COR¹⁰, CO₂R¹⁰ und (C₁-C₄)-Alkoxy-(C₂-C₆)-alkoxycarbonyl substituiert sind,
oder R^{2'} bedeutet Phenyl, Heteroaryl, Heterocyclyl, wobei die drei letztgenannten Reste jeweils durch s Reste aus der Gruppe bestehend aus Cyano, Halogen, Nitro, Rhodano, (C₁-C₆)-Alkyl, Halogen-(C₁-C₆)-alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ und (C₁-C₄)-Alkoxy-(C₂-C₆)-alkoxycarbonyl substituiert sind, und wobei Heterocyclyl n Oxogruppen trägt,
R^{X} bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, wobei die fünf vorstehend genannten Reste jeweils durch s Reste aus der Gruppe bestehend aus Cyano, (R⁶)₃Si, (R⁵O)₂(O)P, R²(O)ₙS, (R¹)₂N, R¹O, R¹(O)C, R¹O(O)C, R¹(O)CO, R²O(O)CO, (C₃-C₆)-Cycloalkyl, Heteroaryl, Heterocyclyl und Phenyl substituiert sind, wobei die vier letztgenannten Reste selber wieder durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
oder R^{X} bedeutet (C₃-C₇)-Cycloalkyl, Heteroaryl, Heterocyclyl oder Phenyl, wobei die vier vorstehend genannten Reste jeweils durch s Reste aus der Gruppe bestehend aus Halogen, Nitro, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiert sind,
R^{Y} bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₇)-Cycloalkyl, (C₁-C₆)-Alkoxy, Cyano, Methylsulfenyl, Methylsulfinyl, Methylsulfonyl, Acetylamino, Methoxycarbonyl, Ethoxycarbonyl, Methoxycarbonylmethyl, Ethoxycarbonylmethyl, Benzoyl, Methylcarbonyl, Trifluormethylcarbonyl, Halogen, Amino, Methylaminocarbonyl, Dimethylaminocarbonyl, Methoxymethyl, oder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Heteroaryl, Heterocyclyl oder Phenyl und wobei Heterocyclyl n Oxogruppen trägt,
R^{Z} bedeutet Wasserstoff, (C₁-C₆)-Alkyl, R¹O-(C₁-C₆)-Alkyl, R⁷CH₂, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, R¹O, R¹(H)N, Methoxycarbonyl, Ethoxycarbonyl, Methylcarbonyl, Dimethylamino, Trifluormethylcarbonyl, Acetylamino, Methylsulfenyl, Methylsulfinyl, Methylsulfonyl oder jeweils durch s Reste aus der Gruppe bestehend aus Halogen, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiertes Heteroaryl, Heterocyclyl, Benzyl oder Phenyl, wobei Heterocyclyl n Oxogruppen trägt,
W bedeutet Wasserstoff, Halogen, (C₁-C₆)-Alkyl, Halogen-(C₁-C₆)-alkyl, (C₂-C₆)-Alkenyl, (C₂-C₆)-Alkinyl, (C₃-C₇)-Cycloalkyl, (C₁-C₆)-Alkoxy, Halogen-(C₁-C₆)-alkoxy, (C₁-C₆)-Alkyl-(O)ₙS-, (C₁-C₆)-Alkoxy-(C₁-C₄)-alkyl, (C₁-C₆)-Alkoxy-(C₁-C₄)-halogenalkyl, R¹(O)C, (R¹)₂N, R¹(O)C(R¹)N oder R²(O)₂S(R¹)N,
X bedeutet Nitro, Halogen, Cyano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, R¹(O)CO, R¹O(O)CO, (R¹)₂N, R¹(O)C(R¹)N, R²(O)₂S(R¹)N, R¹O(O)C(R¹)N, R²(O)ₙS, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R¹O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R¹O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, (R⁵O)₂(O)P-(C₁-C₆)-Alkyl, Phenyl, Heteroaryl oder Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
Y bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, R¹(O)CO, R²(O)₂SO, R¹O(O)CO, (R¹)₂N(O)CO, (R¹)₂N, R¹(O)C(R¹)N, R²(O)₂S(R¹)N, R¹O(O)C(R¹)N, R²(O)ₙS, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R¹O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R¹O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, (R⁵O)₂(O)P-(C₁-C₆)-Alkyl, Phenyl, Heteroaryl, Heterocyclyl, wobei die drei letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
n bedeutet 0, 1 oder 2,
s bedeutet 0, 1, 2 oder 3.

3. Substituierte Ketoxim-benzoylamide der Formel (I) nach Anspruch 1 oder 2, worin die Symbole und Indices folgende Bedeutungen haben:
Q bedeutet einen Rest Q1, Q2, Q3 oder Q4, 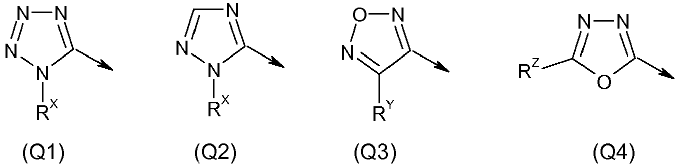
R bedeutet Wasserstoff, (C₁-C₆)-Alkyl, Halogen-(C₁-C₆)-alkyl, R¹(O)C-(C₁-C₆)-Alkyl, R¹O(O)C-O-(C₁-C₆)-Alkyloxy, R¹(O)C, R¹O(O)C oder R²(O)₂S,
R¹ bedeutet Wasserstoff, (C₁-C₆)-Alkyl, Halogen-(C₁-C₆)-alkyl, (C₃-C₆)-Cycloalkyl, (C₁-C₆)-Alkyl-O-(C₁-C₆)-alkyl, Phenyl, Heteroaryl oder Heterocyclyl, wobei die drei letztgenannten Reste jeweils durch s Reste aus der Gruppe bestehend aus Halogen, (C₁-C₆)-Alkyl, Halogen-(C₁-C₆)-alkyl und R³O(O)C substituiert sind, und wobei Heterocyclyl n Oxogruppen trägt,
R² bedeutet (C₁-C₆)-Alkyl, Halogen-(C₁-C₆)-alkyl, (C₃-C₆)-Cycloalkyl, (C₁-C₆)-Alkyl-O-(C₁-C₆)-alkyl, Phenyl, Heteroaryl oder Heterocyclyl, wobei die drei letztgenannten Reste jeweils durch s Reste aus der Gruppe bestehend aus Halogen, (C₁-C₆)-Alkyl, Halogen-(C₁-C₆)-alkyl, R³O(O)C, R³O, R⁴(O)ₙS und R³O-(C₁-C₆)-Alkyl substituiert sind, und wobei Heterocyclyl n Oxogruppen trägt,
R³ bedeutet Wasserstoff, (C₁-C₆)-Alkyl oder Halogen-(C₁-C₆)-alkyl,
R⁴ bedeutet (C₁-C₆)-Alkyl oder Halogen-(C₁-C₆)-alkyl,
R⁵ bedeutet Wasserstoff oder (C₁-C₄)-Alkyl,
R⁶ bedeutet (C₁-C₄)-Alkyl,
R⁷ bedeutet Acetoxy, Methylsulfenyl oder (C₃-C₆)-Cycloalkyl,
R⁸ bedeutet (C₁-C₆)-Alkyl oder Halogen-(C₁-C₆)-alkyl,
R⁹ bedeutet (C₁-C₆)-Alkyl,
R¹⁰ bedeutet Wasserstoff, (C₁-C₆)-Alkyl oder Halogen-(C₁-C₆)-alkyl,
R¹¹ bedeutet (C₁-C₆)-Alkyl,
R^{1'} bedeutet (C₁-C₆)-Alkyl, (C₃-C₆)-Cycloalkyl, Halogen-(C₁-C₆)-alkyl, OR⁸, SR⁸, NR⁸R⁹
R^{2'} bedeutet Wasserstoff, (C₁-C₆)-Alkyl, (C₃-C₆)-Cycloalkyl, (C₃-C₆)-Cycloalkyl-(C₁-C₆)-alkyl, wobei die letzten drei Reste jeweils durch s Reste aus der Gruppe bestehend aus Cyano, Halogen, OR¹⁰, S(O)ₙR¹¹, N(R¹⁰)₂, COR¹⁰, CO₂R¹⁰ und (C₁-C₄)-Alkoxy-(C₂-C₆)-alkoxycarbonyl substituiert sind,
oder R^{2'} bedeutet Phenyl, Heteroaryl, Heterocyclyl, wobei die drei letztgenannten Reste jeweils durch s Reste aus der Gruppe bestehend aus Cyano, Halogen, (C₁-C₆)-Alkyl, Halogen-(C₁-C₆)-alkyl, OR¹⁰, S(O)ₙR¹¹, N(R¹⁰)₂, COR¹⁰ und CO₂R¹⁰ substituiert sind, und wobei Heterocyclyl n Oxogruppen trägt,
R^{X} bedeutet (C₁-C₆)-Alkyl, Halogen-(C₁-C₆)-alkyl, (C₂-C₆)-Alkenyl, (C₂-C₆)-Alkinyl, wobei die vier vorstehend genannten Reste jeweils durch s Reste aus der Gruppe bestehend aus Cyano, (R⁶)₃Si, (R⁵O)₂(O)P, R²(O)ₙS, (R¹)₂N, R¹O, R¹(O)C, R¹O(O)C, (C₃-C₆)-Cycloalkyl, Heteroaryl, Heterocyclyl und Phenyl substituiert sind, wobei die vier letztgenannten Reste selber wieder durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiert sind, und wobei Heterocyclyl n Oxogruppen trägt,
oder R^{X} bedeutet (C₃-C₇)-Cycloalkyl, Heteroaryl, Heterocyclyl oder Phenyl, wobei die vier vorstehend genannten Reste jeweils durch s Reste aus der Gruppe bestehend aus Halogen, Cyano, (C₁-C₆)-Alkyl, Halogen-(C₁-C₆)-alkyl, (C₃-C₆)-Cycloalkyl, (C₁-C₆)-Alkyl- S(O)ₙ, (C₁-C₆)-Alkoxy, Halogen-(C₁-C₆)-alkoxy und (C₁-C₆)-Alkoxy-(C₁-C₄)-alkyl substituiert sind,
R^{Y} bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₇)-Cycloalkyl, (C₁-C₆)-Alkoxy, Methylsulfenyl, Methylsulfinyl, Methylsulfonyl, Acetylamino, Methoxycarbonyl, Ethoxycarbonyl, Methoxycarbonylmethyl, Ethoxycarbonylmethyl, Benzoyl, Methylcarbonyl, Trifluormethylcarbonyl, Halogen, Methoxymethyl, oder jeweils durch s Reste aus der Gruppe bestehend aus (C₁-C₆)-Alkyl, Halogen-(C₁-C₆)-alkyl, (C₁-C₆)-Alkoxy, Halogen-(C₁-C₆)-alkoxy und Halogen substituiertes Phenyl,
R^{Z} bedeutet Wasserstoff, (C₁-C₆)-Alkyl, R¹O-(C₁-C₆)-Alkyl, R⁷CH₂, (C₃-C₇)-Cycloalkyl, Halogen-(C₁-C₆)-alkyl, (C₂-C₆)-Alkenyl, Halogen-(C₂-C₆)-alkenyl, (C₂-C₆)-Alkinyl, R¹O, R¹(H)N, Methoxycarbonyl, Ethoxycarbonyl, Methylcarbonyl, Dimethylamino, Trifluormethylcarbonyl, Acetylamino, Methylsulfenyl, Methylsulfinyl, Methylsulfonyl oder jeweils durch s Reste aus der Gruppe bestehend aus Halogen, Cyano, (C₁-C₆)-Alkyl, Halogen-(C₁-C₆)-alkyl, (C₃-C₆)-Cycloalkyl, (C₁-C₆)-Alkyl-S(O)ₙ, (C₁-C₆)-Alkoxy und Halogen-(C₁-C₆)-alkoxy substituiertes Phenyl,
W bedeutet Wasserstoff, Halogen, (C₁-C₆)-Alkyl, Halogen-(C₁-C₆)-alkyl, (C₃-C₇)-Cycloalkyl, (C₁-C₆)-Alkoxy, Halogen-(C₁-C₆)-alkoxy, (C₁-C₆)-Alkyl-S(O)ₙ oder (C₁-C₆)-Alkoxy-(C₁-C₄)-alkyl,
X bedeutet Halogen, (C₁-C₆)-Alkyl, Halogen-(C₁-C₆)-alkyl, (C₃-C₆)-Cycloalkyl, R¹(O)C, R¹O(O)C, (R¹)₂N(O)C, R¹O, (R¹)₂N, R²(O)ₙS, R¹O-(C₁-C₆)-Alkyl, (R¹)₂N-(C₁-C₆)-Alkyl, R²(O)ₙS-(C₁-C₆)-Alkyl, Phenyl, wobei der letztgenannte Rest jeweils durch s Reste aus der Gruppe bestehend aus Halogen, (C₁-C₆)-Alkyl, Halogen-(C₁-C₆)-alkyl, R¹O(O)C, R¹O, (R¹)₂N, R²(O)ₙS und R¹O-(C₁-C₆)-Alkyl substituiert ist,
Y bedeutet Wasserstoff, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, R¹(O)C, R¹O(O)C, R¹O, (R¹)₂N, R²(O)ₙS, (R⁵O)₂(O)P, R¹O-(C₁-C₆)-Alkyl, (R¹)₂N-(C₁-C₆)-Alkyl, R²(O)ₙS-(C₁-C₆)-Alkyl, Phenyl, wobei der letztgenannte Rest jeweils durch s Reste aus der Gruppe bestehend aus Halogen, (C₁-C₆)-Alkyl, Halogen-(C₁-C₆)-alkyl, R¹O(O)C, R¹O, (R¹)₂N, R²(O)ₙS und R¹O-(C₁-C₆)-Alkyl substituiert ist,
n bedeutet 0, 1 oder 2,
s bedeutet 0, 1, 2 oder 3.

4. Substituierte Ketoxim-benzoylamide der Formel (I) nach Anspruch 1 oder 2, worin die Symbole und Indices folgende Bedeutungen haben:
Q bedeutet einen Rest Q1, Q2, Q3 oder Q4, 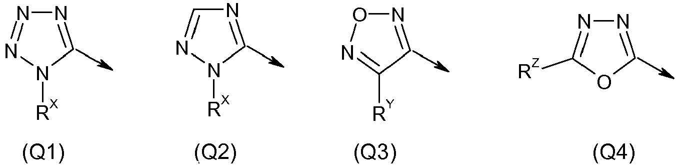
R bedeutet Wasserstoff,
R^{1'} bedeutet Methyl, Ethyl, Trifluormethyl oder cyclo-Propyl,
R^{2'} bedeutet Methyl, Ethyl, 2,2,2-Trifluorethyl, cyclo-Propylmethyl oder
Trifluormethyl,
R^{x} bedeutet Methyl, Ethyl, Propyl, Methoxyethyl, 2-Methoxy-2-methyl-1-propyl, - oder Phenyl,
R^{y} bedeutet Chlor, Methyl oder Ethyl,
R^{z} bedeutet Chlor, Methyl oder Ethyl,
X bedeutet Fluor, Chlor, Brom, Methyl, Methoxy, Methylsulfonyl, Methoxymethyl, Methylsulfenyl, Trifluormethyl oder cyclo-Propyl,
Y bedeutet Chlor, Methyl, Ethyl, cyclo-Propyl, Allyl, Vinyl, Trifluormethyl, Difluormethyl, Chlordifluormethyl, Pentafluorethyl, Methoxy, Methylsulfenyl, Methylsulfinyl, Methylsulfonyl oder Ethylsulfonyl,
W bedeutet Wasserstoff, Fluor, Chlor, Methyl oder Trifluormethyl.

5. Herbizide Mittel, **gekennzeichnet durch** einen herbizid wirksamen Gehalt an mindestens einem Ketoxim-benzoylamid der Formel (I) gemäß einem der Ansprüche 1 bis 4.

6. Herbizide Mittel nach Anspruch 5 in Mischung mit Formulierungshilfsmitteln.

7. Herbizide Mittel nach Anspruch 5 oder 6 enthaltend mindestens einen weiteren pestizid wirksamen Stoff aus der Gruppe Insektizide, Akarizide, Herbizide, Fungizide, Safener und Wachstumsregulatoren.

8. Herbizide Mittel nach Anspruch 7 enthaltend einen Safener.

9. Herbizide Mittel nach einem der Ansprüche 7 bis 8 enthaltend ein weiteres Herbizid.

10. Verfahren zur Bekämpfung unerwünschter Pflanzen, **dadurch gekennzeichnet, daß** man eine wirksame Menge mindestens eines Ketoxim-benzoylamids der Formel (I) gemäß einem der Ansprüche 1 bis 4 oder eines herbiziden Mittels nach einem der Ansprüche 5 bis 9 auf die Pflanzen oder auf den Ort des unerwünschten Pflanzenwachstums appliziert.

11. Verwendung von Ketoxim-benzoylamiden der Formel (I) gemäß einem der Ansprüche 1 bis 4 oder von herbiziden Mitteln nach einem der Ansprüche 5 bis 9 zur Bekämpfung unerwünschter Pflanzen.

12. Verwendung nach Anspruch 11, **dadurch gekennzeichnet, daß** die Ketoxim-benzoylamide der Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

## Claims

1. Substituted ketoxime benzoylamides of the formula (I) 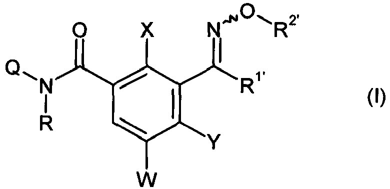 in which the symbols and indices are defined as follows:
Q is a Q1, Q2, Q3 or Q4 radical, 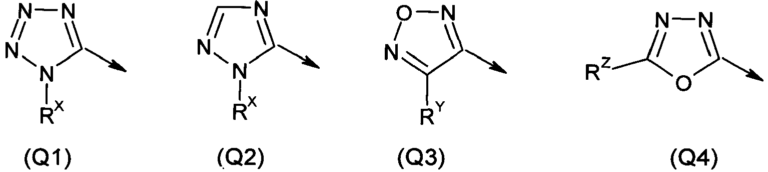
R is hydrogen, (C₁-C₆) -alkyl, halo- (C₁-C₆) -alkyl, (C₂-C₆) -alkenyl, halo- (C₂-C₆) -alkenyl, (C₂-C₆) -alkynyl, halo-(C₃-C₆) -alkynyl, (C₃-C₆) -cycloalkyl, halo- (C₃-C₆) - cycloalkyl, (C₃-C₆) -cycloalkyl- (C₁-C₆) -alkyl, halo- (C₃-C₆) -cycloalkyl- (C₁-C₆) -alkyl, R¹(O)C-(C₁-C₆) -alkyl, R¹O(O)C-(C₁-C₆) -alkyl, (R¹)₂N(O)C-(C₁-C₆) -alkyl, NC-(C₁-C₆)-alkyl, R¹O-(C₁-C₆) -alkyl, R¹(O)CO-(C₁-C₆)-alkyl, R¹O(O)C-O-(C₁-C₆) -alkyloxy, R²(O)₂SO-(C₁-C₆) -alkyl, (R¹)₂N-(C₁-C₆) -alkyl, R¹(O)C(R¹)N-(C₁-C₆) -alkyl, R²(O)₂S(R¹)N-(C₁-C₆) -alkyl, R²(O)ₙS-(C₁-C₆) -alkyl, R¹O(O)₂S-(C₁-C₆) -alkyl, (R¹)₂N(O)₂S-(C₁-C₆)-alkyl, R¹(O)C, R¹O(O)C, (R¹)₂N(O)C, R¹O, (R¹)₂N, R¹O(O)C(R¹)N, (R¹)₂N(O)C(R¹)N, R²(O)₂S, or benzyl substituted in each case by s radicals from the group consisting of methyl, ethyl, methoxy, nitro, trifluoromethyl and halogen,
R¹ is hydrogen, (C₁-C₆)-alkyl, halo- (C₁-C₆) -alkyl, (C₂-C₆)-alkenyl, halo- (C₂-C₆) -alkenyl, (C₂-C₆) -alkynyl, halo-(C₃-C₆) -alkynyl, (C₃-C₆) -cycloalkyl, (C₃-C₆)-cycloalkenyl, halo- (C₃-C₆) -cycloalkyl, (C₃-C₆)-cycloalkyl- (C₁-C₆) -alkyl, (C₁-C₆) -alkyl-O-(C₁-C₆) -alkyl, cycloalkyl-(C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O- (C₁-C₆) -alkyl, heterocyclyl-O-(C₁-C₆)-alkyl, phenyl-N(R³)-(C₁-C₆) -alkyl, heteroaryl-N(R³)-(C₁-C₆) -alkyl, heterocyclyl-N(R³)-(C₁-C₆) -alkyl, phenyl-S(O)n-(C₁-C₆) -alkyl, heteroaryl-S(O)ₙ-(C₁-C₆) - alkyl or heterocyclyl-S(O)ₙ-(C₁-C₆) -alkyl, where the fifteen latter radicals are each substituted by s radicals from the group consisting of nitro, halogen, cyano, thiocyanato, (C₁-C₆)-alkyl, halo-(C₁-C₆)-alkyl, (C₃-C₆) -cycloalkyl, R³O(O)C, (R³)₂N(O)C, R³O, (R³)₂N, R⁴(O)ₙS, R³O(O)₂S, (R³)₂N(O)₂S and R³O-(C₁-C₆) -alkyl, and where heterocyclyl bears n oxo groups,
R² is (C₁-C₆) -alkyl, halo- (C₁-C₆) -alkyl, (C₂-C₆)-alkenyl, halo- (C₂-C₆) -alkenyl, (C₂-C₆) -alkynyl, halo- (C₃-C₆)-alkynyl, (C₃-C₆) -cycloalkyl, (C₃-C₆) -cycloalkenyl, halo- (C₃-C₆) -cycloalkyl, (C₃-C₆) -cycloalkyl- (C₁-C₆) - alkyl, (C₁-C₆) -alkyl-O-(C₁-C₆) -alkyl, cycloalkyl- (C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, phenyl-N(R³)-(C₁-C₆)-alkyl, heteroaryl-N(R³)-(C₁-C₆)-alkyl, heterocyclyl-N(R³)-(C₁-C₆)-alkyl, phenyl-S(O)ₙ-(C₁-C₆)-alkyl, heteroaryl-S(O)ₙ-(C₁-C₆)-alkyl or heterocyclyl-S(O)ₙ-(C₁-C₆)-alkyl, where the fifteen latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R³O(O)C, (R³)₂N(O)C, R³O, (R³)₂N, R⁴(O)ₙS, R³O(O)₂S, (R³)₂N(O)₂S and R³O-(C₁-C₆)-alkyl, and where heterocyclyl bears n oxo groups,
R³ is hydrogen, (C₁-C₆)-alkyl, halo- (C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl or phenyl,
R⁴ is (C₁-C₆)-alkyl, halo- (C₁-C₆)-alkyl, (C₂-C₆)-alkenyl, (C₂-C₆)-alkynyl, (C₃-C₆)-cycloalkyl, (C₃-C₆)-cycloalkyl-(C₁-C₆)-alkyl or phenyl,
R⁵ is hydrogen or (C₁-C₄)-alkyl,
R⁶ is (C₁-C₄)-alkyl,
R⁷ is acetoxy, acetamido, N-methylacetamido, benzoyloxy, benzamido, N-methylbenzamido, methoxycarbonyl, ethoxycarbonyl, benzoyl, methylcarbonyl, piperidinylcarbonyl, morpholinylcarbonyl, trifluoromethylcarbonyl, aminocarbonyl, methylaminocarbonyl, dimethylaminocarbonyl, methylsulfenyl, methylsulfinyl, methylsulfonyl, (C₃-C₆)-cycloalkyl, or heteroaryl or heterocyclyl each substituted by s radicals from the group consisting of methyl, ethyl, methoxy, trifluoromethyl and halogen,
R⁸ is (C₁-C₆)-alkyl, halo- (C₁-C₆)-alkyl, (C₁-C₆)-alkyl-O-(C₁-C₆)-alkyl, (C₂-C₆)-alkenyl or (C₃-C₆)-alkynyl,
R⁹ is (C₁-C₆)-alkyl, halo-(C₁-C₆)-alkyl, (C₂-C₆)-alkenyl or (C₃-C₆)-alkynyl,
R¹⁰ is hydrogen, (C₁-C₆)-alkyl, halo- (C₁-C₆)-alkyl, (C₂-C₆) -alkenyl, (C₂-C₆)-alkynyl, (C₃-C₆)-cycloalkyl or (C₃-C₆) -cycloalkyl- (C₁-C₆)-alkyl,
R¹¹ is (C₁-C₆)-alkyl, (C₂-C₆)-alkenyl or (C₂-C₆)-alkynyl,
R^{1'} is cyano, (C₁-C₆)-alkyl, (C₃-C₆)-cycloalkyl, halo-(C₁-C₆)-alkyl, OR⁸, SR⁸, NR⁸R⁹,
R^{2'} is hydrogen, (C₁-C₆)-alkyl, (C₂-C₆)-alkenyl, (C2-C₆)-alkynyl, (C₃-C₆)-cycloalkyl, (C₃-C₆)-cycloalkenyl, (C₃-C₆)-cycloalkyl-(C₁-C₆)-alkyl, where the last six radicals are each substituted by s radicals from the group consisting of cyano, halogen, nitro, thiocyanato, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ and (C₁-C₄) alkoxy-(C₂-C₆)-alkoxycarbonyl,
or R²' is phenyl, phenyl-(C₁-C₆)-alkyl, heteroaryl, (C₁-C₆) -alkylheteroaryl, heterocyclyl, (C₁-C₆)-alkylheterocyclyl, (C₁-C₆)-alkyl-O-heteroaryl, (C₁-C₆)-alkyl-O-heterocyclyl, (C₁-C₆)-alkyl-NR¹⁰-heteroaryl, (C₁-C₆)-alkyl-NR¹⁰-heterocyclyl, where the ten latter radicals are each substituted by s radicals from the group consisting of cyano, halogen, nitro, thiocyanato, (C₁-C₆)-alkyl, halo-(C₁-C₆)-alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ and (C₁-C₄)-alkoxy-(C₂-C₆)-alkoxycarbonyl, and where heterocyclyl bears n oxo groups,
R^{X} is (C₁-C₆)-alkyl, halo- (C₁-C₆)-alkyl, (C₂-C₆)-alkenyl, halo-(C₂-C₆)-alkenyl, (C₂-C₆)-alkynyl, halo- (C₃-C₆)-alkynyl, where the six aforementioned radicals are each substituted by s radicals from the group consisting of nitro, cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C(R¹)N, R²(O)₂S(R¹)N, (C₃-C₆)-cycloalkyl, heteroaryl, heterocyclyl and phenyl, where the four latter radicals themselves are in turn substituted by s radicals from the group consisting of (C₁-C₆)-alkyl, halo-(C₁-C₆)-alkyl, (C₁-C₆)-alkoxy, halo-(C₁-C₆)-alkoxy and halogen, and where heterocyclyl bears n oxo groups,
or R^{X} is (C₃-C₇)-cycloalkyl, heteroaryl, heterocyclyl or phenyl, where the four aforementioned radicals are each substituted by s radicals from the group consisting of halogen, nitro, cyano, (C₁-C₆)-alkyl, halo-(C₁-C₆)-alkyl, (C₃-C₆)-cycloalkyl, (C₁-C₆)-alkyl-S(O)ₙ, (C₁-C₆)-alkoxy, halo-(C₁-C₆)-alkoxy and (C₁-C₆)-alkoxy-(C₁-C₉)-alkyl,
R^{Y} is hydrogen, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₇)-cycloalkyl, (C₁-C₆)-alkoxy, halo- (C₁-C₆)-alkoxy, (C₂-C₆)-alkenyloxy, (C₂-C₆)-alkynyloxy, cyano, nitro, methylsulfenyl, methylsulfinyl, methylsulfonyl, acetylamino, benzoylamino, methoxycarbonyl, ethoxycarbonyl, methoxycarbonylmethyl, ethoxycarbonylmethyl, benzoyl, methylcarbonyl, piperidinylcarbonyl, trifluoromethylcarbonyl, halogen, amino, aminocarbonyl, methylaminocarbonyl, dimethylaminocarbonyl, methoxymethyl, or heteroaryl, heterocyclyl or phenyl each substituted by s radicals from the group consisting of (C₁-C₆)-alkyl, halo-(C₁-C₆)-alkyl, (C₁-C₆)-alkoxy, halo-(C₁-C₆)-alkoxy and halogen, and where heterocyclyl bears n oxo groups,
R^{Z} is hydrogen, (C₁-C₆)-alkyl, R¹O-(C₁-C₆)-alkyl, R⁷CH₂, (C₃-C₇)-cycloalkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, R¹O, R¹(H)N, methoxycarbonyl, ethoxycarbonyl, methylcarbonyl, dimethylamino, trifluoromethylcarbonyl, acetylamino, methylsulfenyl, methylsulfinyl, methylsulfonyl, or heteroaryl, heterocyclyl, benzyl or phenyl each substituted by s radicals from the group consisting of halogen, nitro, cyano, (C₁-C₆)-alkyl, halo-(C₁-C₆)-alkyl, (C₃-C₆)-cycloalkyl, (C₁-C₆)-alkylS(O)ₙ, (C₁-C₆)-alkoxy, halo-(C₁-C₆)-alkoxy and (C₁-C₆)-alkoxy-(C₁-C₄)-alkyl, where heterocyclyl bears n oxo groups,
W is hydrogen, halogen, nitro, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₇)-cycloalkyl, halo-(C₃-C₇)-cycloalkyl, (C₁-C₆)-alkoxy, halo-(C₁-C₆)-alkoxy, (C₁-C₆)-alkyl-(O)ₙS-, (C₁-C₆)-haloalkyl- (O)ₙS-, (C₁-C₆)-alkoxy-(C₁-C₄)-alkyl, (C₁-C₆)-alkoxy-(C₁-C₄)-haloalkyl, R¹(O)C, R¹(R¹ON=)C, R¹O(O)C, (R¹)₂N, R¹(O)C(R¹)N or R²(O)₂S(R¹)N,
X is nitro, halogen, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, R¹(O)C, R¹(R1N=)C, R¹O(O)C, (R¹)₂N(O)C, R¹(R¹O)N(O)C, (R¹)₂N(R¹)N(O)C, R¹(O)C(R¹)N(O)C, R¹O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R¹O(O)CO, (R¹)₂N(O)CO, (R¹)₂N, R¹(O)C(R¹)N, R²(O)₂S(R¹)N, R¹O(O)C(R¹)N, (R¹)₂N(O)C(R¹)N, R¹O(O)₂S(R¹)N, (R¹)₂N(O)₂S(R¹)_{N}, R²(O)ₙS, R¹O(O)₂S, (R¹)₂N(O)₂S, R¹(O)C(R¹)N(O)₂S, R¹O(O)C(R¹)N(O)₂S, (R¹)₂N(O)C(R¹)N(O)₂S, (R⁵O)₂(O)P, R¹(O)C-(C₁-C₆)-alkyl, R¹O(O)C-(C₁-C₆)-alkyl, (R¹)₂N(O)C-(C₁-C₆)-alkyl, (R¹O)(R¹)N(O)C-(C₁-C₆)-alkyl, (R¹)₂N(R¹)N(O)C-(C₁-C₆)-alkyl, R¹(O)C(R¹)N(O)C-(C₁-C₆)-alkyl, R¹O(O)C(R¹)N(O)C-(C₁-C₆)-alkyl, (R¹)₂N(O)C(R¹)N(O)C-(C₁-C₆)-alkyl, R²(O)₂S(R¹)N(O)C-(C₁-C₆)-alkyl, R¹O(O)₂S(R¹)N(O)C-(C₁-C₆)-alkyl, (R¹)₂N(O)₂S(R¹)N(O)C-(C₁-C₆)-alkyl, NC-(C₁-C₆)-alkyl, R¹O-(C₁-C₆)-alkyl, R¹(O)CO-(C₁-C₆)-alkyl, R²(O)₂SO-(C₁-C₆)-alkyl, R¹O(O)CO-(C₁-C₆)-alkyl, (R¹)₂N(O)CO-(C₁-C₆)-alkyl, (R¹)₂N-(C₁-C₆)-alkyl, R¹(O)C(R¹)N-(C₁-C₆)-alkyl, R²(O)₂S(R¹)N-(C₁-C₆)-alkyl, R¹O(O)C(R¹)N- (C₁-C₆)-alkyl, (R¹)₂N(O)C(R¹)N-(C₁-C₆)-alkyl, R¹O(O)₂S(R¹)N-(C₁-C₆)-alkyl, (R¹)₂N(O)₂S(R¹)N-(C₁-C₆)-alkyl, R²(O)ₙS-(C₁-C₆)-alkyl, R¹O(O)₂S-(C₁-C₆)-alkyl, (R¹)₂N(O)₂S-(C₁-C₆)-alkyl, R¹(O)C(R¹)N(O)₂S-(C₁-C₆)-alkyl, R¹O(O)C(R¹)N(O)₂S-(C₁-C₆)-alkyl, (R¹)₂N(O)C(R¹)N(O)₂S-(C₁-C₆)-alkyl, (R⁵O)₂(O)P-(C₁-C₆)-alkyl, phenyl, heteroaryl, heterocyclyl, phenyl-(C₁-C₆)-alkyl, heteroaryl-(C₁-C₆)-alkyl, heterocyclyl-(C₁-C₆)-alkyl, where the six latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl bears n oxo groups,
Y is hydrogen, nitro, halogen, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo-(C₃-C₆)-alkynyl, (C₃-C₆)-cycloalkyl, halo-(C₃-C₆)-cycloalkyl, (C₃-C₆)-cycloalkyl-(C₁-C₆)-alkyl, halo-(C₃-C₆)-cycloalkyl-(C₁-C₆)-alkyl, R¹(O)C, R¹(R¹ON=)C, R¹O(O)C, (R¹)₂N(O)C, R¹(R¹⁰)N(O)C, (R¹)₂N(R¹)N(O)C, R¹(O)C(R¹)N(O)C, R¹O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R¹O(O)CO, (R¹)₂N(O)CO, (R¹)₂N, R¹(O)C(R¹)N, R²(P)₂S(R¹)N, R¹O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R¹O(O)C(R¹)N(O)₂S, (R¹)₂N(O)C(R¹)N(O)₂S, (R⁵O)₂(O)P, R¹(O)C-(C₁-C₆)-alkyl, R¹O(O)C-(C₁-C₆)-alkyl, (R¹)₂N(O)C-(C₁-C₆)-alkyl, (R¹O)(R¹)N(O)C-(C₁-C₆)-alkyl, (R¹)₂N(R¹)N(O)C-(C₁-C₆)-alkyl, R¹(O)C(R¹)N(O)C-(C₁-C₆)-alkyl, R¹O(O)C(R¹)N(O)C-(C₁-C₆)-alkyl, (R¹)₂N(O)C(R¹)N(O)C-(C₁-C₆)-alkyl, R²(O)₂S(R¹)N(O)C-(C₁-C₆)-alkyl, R¹O(O)₂S(R¹)N(O)C-(C₁-C₆)-alkyl, (R¹)₂N(O)₂S(R¹)N(O)C-(C₁-C₆)-alkyl, NC-(C₁-C₆)-alkyl, R¹O-(C₁-C₆)-alkyl, R¹(O)CO-(C₁-C₆)-alkyl, R²(O)₂SO-(C₁-C₆)-alkyl, R¹O(O)CO-(C₁-C₆)-alkyl, (R¹)₂N(O)CO-(C₁-C₆)-alkyl, (R¹)₂N-(C₁-C₆)-alkyl, R¹(O)C(R¹)N-(C₁-C₆)-alkyl, R²(O)₂S(R¹)N-(C₁-C₆)-alkyl, R¹O(O)C(R¹)N-(C₁-C₆)-alkyl, (R¹)₂N(O)C(R¹)N-(C₁-C₆)-alkyl, R¹O(O)₂S(R¹)N-(C₁-C₆)-alkyl, (R¹)₂N(O)₂S(R¹)N-(C₁-C₆)-alkyl, R²(O)ₙS-(C₁-C₆)-alkyl, R¹O(O)₂S-(C₁-C₆)-alkyl, (R¹)₂N(O)₂S-(C₁-C₆)-alkyl, R¹(O)C(R¹)N(O)₂S-(C₁-C₆)-alkyl, R¹O(O)C(R¹)N(O)₂S-(C₁-C₆)-alkyl, (R¹)₂N(O)C(R¹)N(O)₂S-(C₁-C₆)-alkyl, (R⁵O)₂(O)P-(C₁-C₆)-alkyl, phenyl, heteroaryl, heterocyclyl, phenyl-(C₁-C₆)-alkyl, heteroaryl-(C₁-C₆)-alkyl, heterocyclyl-(Ci-C₆)-alkyl, where the six latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl bears n oxo groups,
n is 0, 1 or 2,
s is 0, 1, 2 or 3.

2. Substituted ketoxime benzoylamides of the formula (I) according to Claim 1, in which the symbols and indices are defined as follows:
Q is a Q1, Q2, Q3 or Q4 radical, 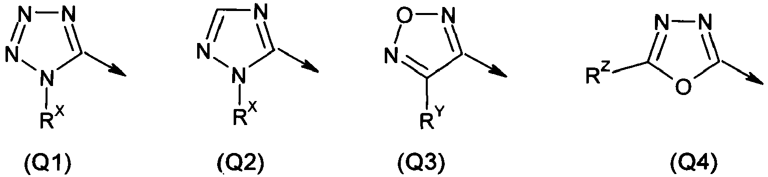
R is hydrogen, (C₁-C₆)-alkyl, halo- (C₁-C₆)-alkyl, R¹(O)C-(C₁-C₆)-alkyl, R¹O(O)C-(C₁-C₆)-alkyl, R¹O(O)C-O-(C₁-C₆)-alkyloxy, R¹O-(C₁-C₆)-alkyl, R¹(O)C, R¹O(O)C, R¹O, R²(O)₂S, or benzyl substituted in each case by s radicals from the group consisting of methyl, ethyl, methoxy, nitro, trifluoromethyl and halogen,
R¹ is hydrogen, (C₁-C₆)-alkyl, halo- (C₁-C₆)-alkyl, (C₂-C₆)-alkenyl, (C₂-C₆)-alkynyl, (C₃-C₆)-cycloalkyl, (C₃-C₆)-cycloalkenyl, (C₁-C₆)-alkyl-O-(C₁-C₆)-alkyl, phenyl, heteroaryl or heterocyclyl, where the three latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R³O(O)C, (R³)₂N(O)C, R³O, (R³)₂N, R⁴(O)ₙS, R³O(O)₂S, (R³)₂N(O)₂S and R³O-(C₁-C₆)-alkyl, and where heterocyclyl bears n oxo groups,
R² is (C₁-C₆)-alkyl, halo-(C₁-C₆)-alkyl, (C₂-C₆)-alkenyl, (C₂-C₆)-alkynyl, (C₃-C₆)-cycloalkyl, (C₁-C₆)-alkyl-O-(C₁-C₆)-alkyl, phenyl, heteroaryl or heterocyclyl, where the three latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R³O(O)C, (R³)₂N(O)C, R³O, (R³)₂N, R⁴(O)ₙS, R³O(O)₂S, (R³)₂N(O)₂S and R³O-(C₁-C₆)-alkyl, and where heterocyclyl bears n oxo groups,
R³ is hydrogen, (C₁-C₆)-alkyl, halo-(C₁-C₆)-alkyl, (C₃-C₆)-cycloalkyl or phenyl,
R⁴ is (C₁-C₆)-alkyl, halo-(C₁-C₆)-alkyl, (C₃-C₆)-cycloalkyl or phenyl,
R⁵ is hydrogen or (C₁-C₄)-alkyl,
R⁶ is (C₁-C₄)-alkyl,
R⁷ is acetoxy, benzoyloxy, methoxycarbonyl, ethoxycarbonyl, methylcarbonyl, trifluoromethylcarbonyl, methylsulfenyl, methylsulfinyl, methylsulfonyl, (C₃-C₆)-cycloalkyl, or heteroaryl or heterocyclyl each substituted by s radicals from the group consisting of methyl, ethyl, methoxy, trifluoromethyl and halogen,
R⁸ is (C₁-C₆)-alkyl, halo- (C₁-C₆)-alkyl, (C₁-C₆)-alkyl-O-(C₁-C₆)-alkyl, (C₂-C₆)-alkenyl or (C₃-C₆)-alkynyl,
R⁹ is (C₁-C₆)-alkyl or halo-(C₁-C₆)-alkyl,
R¹⁰ is hydrogen, (C₁-C₆)-alkyl, halo-(C₁-C₆)-alkyl or (C₃-C₆)-cycloalkyl,
R¹¹ is (C₁-C₆)-alkyl,
R^{1'} is (C₁-C₆)-alkyl, (C₃-C₆)-cycloalkyl, halo-(C₁-C₆)-alkyl, OR⁸, SR⁸, NR⁸R⁹,
R^{2'} is hydrogen, (C₁-C₆)-alkyl, (C₂-C₆)-alkenyl, (C₂-C₆)-alkynyl, (C₃-C₆)-cycloalkyl, (C₃-C₆)-cycloalkenyl, (C₃-C₆)-cycloalkyl-(C₁-C₆)-alkyl, where the last six radicals are each substituted by s radicals from the group consisting of cyano, halogen, OR¹⁰, S(O)ₙR¹¹, N(R¹⁰)₂, COR¹⁰, CO₂R¹⁰ and (C₁-C₄)-alkoxy-(C₂-C₆)-alkoxycarbonyl,
or R^{2'} is phenyl, heteroaryl, heterocyclyl, where the three latter radicals are each substituted by s radicals from the group consisting of cyano, halogen, nitro, thiocyanato, (C₁-C₆)-alkyl, halo-(C₁-C₆)-alkyl, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ and (C₁-C₄)-alkoxy-(C₂-C₆)-alkoxycarbonyl, and where heterocyclyl bears n oxo groups,
R^{X} is (C₁-C₆)-alkyl, halo-(C₁-C₆)-alkyl, (C₂-C₆)-alkenyl, halo-(C₂-C₆)-alkenyl, (C₂-C₆)-alkynyl, where the five aforementioned radicals are each substituted by s radicals from the group consisting of cyano, (R⁶)₃Si, (R⁵O)₂(O)P, R²(O)ₙS, (R¹)₂N, R¹O, R¹(O)C, R¹O(O)C, R¹(O)CO, R²O(O)CO, (C₃-C₆)-cycloalkyl, heteroaryl, heterocyclyl and phenyl, where the four latter radicals themselves are in turn substituted by s radicals from the group consisting of (C₁-C₆)-alkyl, halo-(C₁-C₆)-alkyl, (C₁-C₆)-alkoxy, halo-(C₁-C₆)-alkoxy and halogen, and where heterocyclyl bears n oxo groups,
or R^{X} is (C₃-C₇)-cycloalkyl, heteroaryl, heterocyclyl or phenyl, where the four aforementioned radicals are each substituted by s radicals from the group consisting of halogen, nitro, cyano, (C₁-C₆)-alkyl, halo-(C₁-C₆)-alkyl, (C₃-C₆)-cycloalkyl, (C₁-C₆)-alkyl-S(O)ₙ, (C₁-C₆)-alkoxy, halo-(C₁-C₆)-alkoxy and (C₁-C₆)-alkoxy-(C₁-C₄)-alkyl,
R^{Y} is hydrogen, (C₁-C₆)-alkyl, halo-(C₁-C₆)-alkyl, (C₂-C₆)-alkenyl, (C₂-C₆)-alkynyl, (C₃-C₇)-cycloalkyl, (C₁-C₆)-alkoxy, cyano, methylsulfenyl, methylsulfinyl, methylsulfonyl, acetylamino, methoxycarbonyl, ethoxycarbonyl, methoxycarbonylmethyl, ethoxycarbonylmethyl, benzoyl, methylcarbonyl, trifluoromethylcarbonyl, halogen, amino, methylaminocarbonyl, dimethylaminocarbonyl, methoxymethyl, or heteroaryl, heterocyclyl or phenyl each substituted by s radicals from the group consisting of (C₁-C₆) -alkyl, halo- (C₁-C₆) -alkyl, (C₁-C₆) - alkoxy, halo- (C₁-C₆) -alkoxy and halogen, and where heterocyclyl bears n oxo groups,
R^{Z} is hydrogen, (C₁-C₆)-alkyl, R¹O-(C₁-C₆) -alkyl, R⁷CH₂, (C₃-C₇)-cycloalkyl, halo- (C₁-C₆) -alkyl, (C₂-C₆)-alkenyl, halo- (C₂-C₆)-alkenyl, (C₂-C₆)-alkynyl, R¹O, R¹(H)N, methoxycarbonyl, ethoxycarbonyl, methylcarbonyl, dimethylamino, trifluoromethylcarbonyl, acetylamino, methylsulfenyl, methylsulfinyl, methylsulfonyl, or heteroaryl, heterocyclyl, benzyl or phenyl each substituted by s radicals from the group consisting of halogen, cyano, (C₁-C₆)-alkyl, halo- (C₁-C₆)-alkyl, (C₃-C₆)-cycloalkyl, (C₁-C₆)-alkyl-S(O)ₙ, (C₁-C₆) -alkoxy, halo-(C₁-C₆) -alkoxy and (C₁-C₆)-alkoxy-(C₁-C₄)-alkyl, where heterocyclyl bears n oxo groups,
W is hydrogen, halogen, (C₁-C₆)-alkyl, halo- (C₁-C₆) - alkyl, (C₂-C₆)-alkenyl, (C₂-C₆)-alkynyl, (C₃-C₇) - cycloalkyl, (C₁-C₆) -alkoxy, halo- (C₁-C₆) -alkoxy, (C₁-C₆) - alkyl-(O)ₙS-, (C₁-C₆)-alkoxy- (C₁-C₄)-alkyl, (C₁-C₆)-alkoxy-(C₁-C₄)-haloalkyl, R¹(O)C, (R¹)₂N, R¹(O)C(R¹)N or R²(O)₂S(R¹)N,
X is nitro, halogen, cyano, (C₁-C₆)-alkyl, halo-(C₁-C₆)-alkyl, (C₂-C₆)-alkenyl, halo- (C₂-C₆)-alkenyl, (C₂-C₆)-alkynyl, (C₃-C₆) -cycloalkyl, halo- (C₃-C₆) -cycloalkyl, (C₃-C₆) -cycloalkyl- (C₁-C₆) -alkyl, halo- (C₃-C₆) - cycloalkyl- (C₁-C₆)-alkyl, R¹(O)C, R¹(R¹ON=)C, R¹O(O)C, (R¹)₂N(O)C, R¹O, R¹(O)CO, R¹O(O)CO, (R¹)₂N, R¹(O)C(R¹)N, R²(O)₂S(R¹)N, R¹O(O)C(R¹)N, R²(O)ₙS, (R⁵O)₂(O)P, R¹(O)C-(C₁-C₆)-alkyl, R¹O(O)C-(C₁-C₆)-alkyl, (R¹)₂N(O)C-(C₁-C₆)-alkyl, NC-(C₁-C₆)-alkyl, R¹O-(C₁-C₆)-alkyl, R¹(O)CO-(C₁-C₆)-alkyl, R²(O)₂SO-(C₁-C₆)-alkyl, R¹O(O)CO-(C₁-C₆) -alkyl, (R¹)₂N(O)CO-(C₁-C₆)-alkyl, (R¹)₂N-(C₁-C₆) -alkyl, R¹(O)C(R¹)N-(C₁-C₆)-alkyl, R²(O)₂S(R¹)N-(C₁-C₆) -alkyl, R¹O(O)C(R¹)N-(C₁-C₆)-alkyl, (R¹)₂N(O)C(R¹)N-(C₁-C₆) -alkyl, R¹O(O)₂S(R¹)N-(C₁-C₆)-alkyl, (R¹)₂N(O)₂S(R¹)N-(C₁-C₆)-alkyl, R²(O)ₙS-(C₁-C₆) -alkyl, (R⁵O)₂(O)P-(C₁-C₆) -alkyl, phenyl, heteroaryl or heterocyclyl, where the three latter radicals are each substituted by s radicals from the group consisting of nitro, halogen, cyano, thiocyanato, (C₁-C₆)-alkyl, halo- (C₁-C₆) -alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl bears n oxo groups,
Y is hydrogen, nitro, halogen, cyano, thiocyanato, (C₁-C₆)-alkyl, halo- (C₁-C₆) -alkyl, (C₂-C₆)-alkenyl, halo-(C₂-C₆)-alkenyl, (C₂-C₆)-alkynyl, halo- (C₃-C₆) -alkynyl, (C₃-C₆) -cycloalkyl, halo- (C₃-C₆) -cycloalkyl, (C₃-C₆)-cycloalkyl- (C₁-C₆) -alkyl, halo- (C₃-C₆) -cycloalkyl- (C₁-C₆)-alkyl, R¹(O)C, R¹(R¹ON=)C, R¹O(O)C, (R¹)₂N(O)C, R¹O, R¹(O)CO, R²(O)₂SO, R¹O(O)CO, (R¹)₂N(O)CO, (R¹)₂N, R¹(O)C(R¹)N, R²(O)₂S(R¹)N, R¹O(O)C(R¹)N, R²(O)ₙS, (R⁵O)₂(O)P, R¹(O)C-(C₁-C₆)-alkyl, R¹O(O)C-(C₁-C₆)-alkyl, (R¹)₂N(O)C-(C₁-C₆)-alkyl, NC-(C₁-C₆) -alkyl, R¹O-(C₁-C₆)-alkyl, R¹(O)CO-(C₁-C₆)-alkyl, R²(O)₂SO-(C₁-C₆)-alkyl, R¹O(O)CO-(C₁-C₆) -alkyl, (R¹)₂N(O)CO-(C₁-C₆) -alkyl, (R¹)₂N-(C₁-C₆)-alkyl, R¹(O)C(R¹)N-(C₁-C₆)-alkyl, R²(O)₂S(R¹)N-(C₁-C₆)-alkyl, R¹O(O)C(R¹)N-(C₁-C₆)-alkyl, (R¹)₂N(O)C(R¹)N-(C₁-C₆)-alkyl, R¹O(O)₂S(R¹)N- (C₁-C₆)-alkyl, (R¹)₂N(O)₂S(R¹)N-(C₁-C₆)-alkyl, R²(O)ₙS-(C₁-C₆) -alkyl, (R⁵O)₂(O)P-(C₁-C₆) -alkyl, phenyl, heteroaryl, heterocyclyl, where the three latter radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl bears n oxo groups,
n is 0, 1 or 2,
s is 0, 1, 2 or 3.

3. Substituted ketoxime benzoylamides of the formula (I) according to Claim 1 or 2, in which the symbols and indices are defined as follows:
Q is a Q1, Q2, Q3 or Q4 radical, 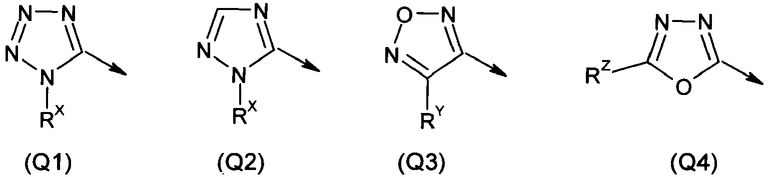
R is hydrogen, (C₁-C₆) -alkyl, halo- (C₁-C₆) -alkyl, R¹(O)C-(C₁-C₆)-alkyl, R¹O(O)C-O-(C₁-C₆)-alkyloxy, R¹(O)C, R¹O(O)C or R²(O)₂S,
R¹ is hydrogen, (C₁-C₆)-alkyl, halo- (C₁-C₆)-alkyl, (C₃-C₆)-cycloalkyl, (C₁-C₆)-alkyl-O-(C₁-C₆)-alkyl, phenyl, heteroaryl or heterocyclyl, where the three latter radicals are each substituted by s radicals from the group consisting of halogen, (C₁-C₆)-alkyl, halo-(C₁-C₆)-alkyl and R³O(O)C, and where heterocyclyl bears n oxo groups,
R² is (C₁-C₆) -alkyl, halo- (C₁-C₆) -alkyl, (C₃-C₆)-cycloalkyl, (C₁-C₆)-alkyl-O-(C₁-C₆)-alkyl, phenyl, heteroaryl or heterocyclyl, where the three latter radicals are each substituted by s radicals from the group consisting of halogen, (C₁-C₆)-alkyl, halo- (C₁-C₆) - alkyl, R³O(O)C, R³O, R⁴(O)ₙS and R³O-(C₁-C₆) -alkyl, and where heterocyclyl bears n oxo groups,
R³ is hydrogen, (C₁-C₆)-alkyl or halo- (C₁-C₆)-alkyl,
R⁴ is (C₁-C₆)-alkyl or halo- (C₁-C₆) -alkyl,
R⁵ is hydrogen or (C₁-C₄)-alkyl,
R⁶ is (C₁-C₄)-alkyl,
R⁷ is acetoxy, methylsulfenyl or (C₃-C₆)-cycloalkyl,
R⁸ is (C₁-C₆)-alkyl or halo- (C₁-C₆) -alkyl,
R⁹ is (C₁-C₆)-alkyl,
R¹⁰ is hydrogen, (C₁-C₆)-alkyl or halo- (C₁-C₆) -alkyl,
R¹¹ is (C₁-C₆)-alkyl,
R^{1'} is (C₁-C₆)-alkyl, (C₃-C₆) -cycloalkyl, halo- (C₁-C₆) - alkyl, OR⁸, SR⁸, NR⁸R⁹,
R^{2'} is hydrogen, (C₁-C₆)-alkyl, (C₃-C₆)-cycloalkyl, (C₃-C₆)-cycloalkyl-(C₁-C₆)-alkyl, where the last three radicals are each substituted by s radicals from the group consisting of cyano, halogen, OR¹⁰, S(O)ₙR¹¹, N(R¹⁰)₂, COR¹⁰, CO₂R¹⁰ and (C₁-C₄)-alkoxy-(C₂-C₆)-alkoxycarbonyl,
or R^{2'} is phenyl, heteroaryl, heterocyclyl, where the three latter radicals are each substituted by s radicals from the group consisting of cyano, halogen, (C₁-C₆)-alkyl, halo-(C₁-C₆)-alkyl, OR¹⁰, S(O)ₙR¹¹, N(R¹⁰)₂, COR¹⁰ and CO₂R¹⁰, and where heterocyclyl bears n oxo groups,
R^{x} is (C₁-C₆)-alkyl, halo-(C₁-C₆)-alkyl, (C₂-C₆)-alkenyl, (C₂-C₆)-alkynyl, where the four aforementioned radicals are each substituted by s radicals from the group consisting of cyano, (R⁶)₃Si, (R⁵O)₂(O)P, R²(O)ₙS, (R¹)₂N, R¹O, R¹(O)C, R¹O(O)C, (C₃-C₆) -cycloalkyl, heteroaryl, heterocyclyl and phenyl, where the four latter radicals themselves are in turn substituted by s radicals from the group consisting of (C₁-C₆)-alkyl, halo- (C₁-C₆)-alkyl, (C₁-C₆)-alkoxy, halo- (C₁-C₆) -alkoxy and halogen, and where heterocyclyl bears n oxo groups, or R^{x} is (C₃-C₇)-cycloalkyl, heteroaryl, heterocyclyl or phenyl, where the four aforementioned radicals are each substituted by s radicals from the group consisting of halogen, cyano, (C₁-C₆)-alkyl, halo-(C₁-C₆) -alkyl, (C₃-C₆)-cycloalkyl, (C₁-C₆) -alkyl-S(O)ₙ, (C₁-C₆)-alkoxy, halo- (C₁-C₆)-alkoxy and (C₁-C₆) -alkoxy- (C₁-C₄)-alkyl,
R^{Y} is hydrogen, (C₁-C₆) -alkyl, halo-(C₁-C₆)-alkyl, (C₂-C₆)-alkenyl, (C₂-C₆)-alkynyl, (C₃-C₇)-cycloalkyl, (C₁-C₆)-alkoxy, methylsulfenyl, methylsulfinyl, methylsulfonyl, acetylamino, methoxycarbonyl, ethoxycarbonyl, methoxycarbonylmethyl, ethoxycarbonylmethyl, benzoyl, methylcarbonyl, trifluoromethylcarbonyl, halogen, methoxymethyl, or phenyl substituted in each case by s radicals from the group consisting of (C₁-C₆)-alkyl, halo-(C₁-C₆)-alkyl, (C₁-C₆)-alkoxy, halo- (C₁-C₆)-alkoxy and halogen,
R^{z} is hydrogen, (C₁-C₆) -alkyl, R¹O-(C₁-C₆) -alkyl, R⁷CH₂, (C₃-C₇)-cycloalkyl, halo-(C₁-C₆)-alkyl, (C₂-C₆)-alkenyl, halo-(C₂-C₆)-alkenyl, (C₂-C₆)-alkynyl, R¹O, R¹(H)N, methoxycarbonyl, ethoxycarbonyl, methylcarbonyl, dimethylamino, trifluoromethylcarbonyl, acetylamino, methylsulfenyl, methylsulfinyl, methylsulfonyl, or phenyl substituted in each case by s radicals from the group consisting of halogen, cyano, (C₁-C₆)-alkyl, halo-(C₁-C₆)-alkyl, (C₃-C₆)-cycloalkyl, (C₁-C₆)-alkyl-S(O)ₙ, (C₁-C₆)-alkoxy and halo-(C₁-C₆)-alkoxy,
W is hydrogen, halogen, (C₁-C₆)-alkyl, halo-(C₁-C₆)- alkyl, (C₃-C₇)-cycloalkyl, (C₁-C₆)-alkoxy, halo-(C₁-C₆)- alkoxy, (C₁-C₆)-alkyl-S(O)ₙ or (C₁-C₆)-alkoxy-(C₁-C₉)-alkyl,
X is halogen, (C₁-C₆)-alkyl, halo-(C₁-C₆)-alkyl, (C₃-C₆)-cycloalkyl, R¹(O)C, R¹O(O)C, (R¹)₂N(O)C, R¹O, (R¹)₂N, R²(O)ₙS, R¹O-(C₁-C₆)-alkyl, (R¹)₂N-(C₁-C₆)-alkyl, R²(O)ₙS-(C₁-C₆)-alkyl, phenyl, where the latter radical is substituted in each case by s radicals from the group consisting of halogen, (C₁-C₆)-alkyl, halo-(C₁-C₆)-alkyl, R¹O(O)C, R¹O, (R¹)₂N, R²(O)ₙS and R¹O-(C₁-C₆)-alkyl,
Y is hydrogen, halogen, cyano, (C₁-C₆) -alkyl, halo-(C₁-C₆)-alkyl, (C₂-C₆)-alkenyl, (C₂-C₆)-alkynyl, (C₃-C₆)-cycloalkyl, halo-(C₃-C₆) -cycloalkyl, (C₃-C₆)-cycloalkyl-(C₁-C₆)-alkyl, R¹(O)C, R¹O(O)C, R¹O, (R¹)₂N, R²(O)ₙS, (R⁵O)₂(O)P, R¹O-(C₁-C₆)-alkyl, (R¹)₂N-(C₁-C₆)-alkyl, R²(O)ₙS-(C₁-C₆)-alkyl, phenyl, where the latter radical is substituted in each case by s radicals from the group consisting of halogen, (C₁-C₆)-alkyl, halo-(C₁-C₆)-alkyl, R¹O(O)C, R¹O, (R¹)₂N, R²(O)ₙS and R¹O-(C₁-C₆)-alkyl,
n is 0, 1 or 2,
s is 0, 1, 2 or 3.

4. Substituted ketoxime benzoylamides of the formula (I) according to Claim 1 or 2, in which the symbols and indices are defined as follows:
Q is a Q1, Q2, Q3 or Q4 radical, 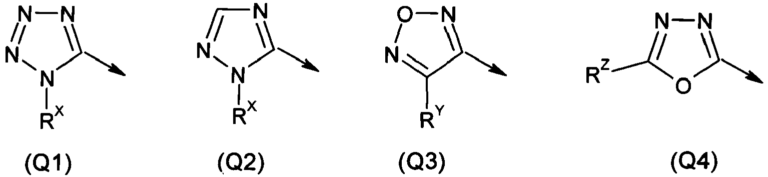
R is hydrogen,
R^{1'} is methyl, ethyl, trifluoromethyl or cyclopropyl,
R^{2'} is methyl, ethyl, 2,2,2-trifluoroethyl, cyclopropylmethyl or trifluoromethyl,
R^{x} is methyl, ethyl, propyl, methoxyethyl, 2-methoxy-2-methyl-1-propyl or phenyl,
R^{y} is chlorine, methyl or ethyl,
R^{z} is chlorine, methyl or ethyl,
X is fluorine, chlorine, bromine, methyl, methoxy, methylsulfonyl, methoxymethyl, methylsulfenyl, trifluoromethyl or cyclopropyl,
Y is chlorine, methyl, ethyl, cyclopropyl, allyl, vinyl, trifluoromethyl, difluoromethyl, chlorodifluoromethyl, pentafluoroethyl, methoxy, methylsulfenyl, methylsulfinyl, methylsulfonyl or ethylsulfonyl,
W is hydrogen, fluorine, chlorine, methyl or trifluoromethyl.

5. Herbicidal compositions **characterized by** a herbicidally active content of at least one ketoxime benzoylamide of the formula (I) according to any of Claims 1 to 4.

6. Herbicidal compositions according to Claim 5 in a mixture with formulation auxiliaries.

7. Herbicidal compositions according to Claim 5 or 6, comprising at least one further pesticidally active substance from the group consisting of insecticides, acaricides, herbicides, fungicides, safeners, and growth regulators.

8. Herbicidal compositions according to Claim 7, comprising a safener.

9. Herbicidal compositions according to either of Claims 7 and 8, comprising a further herbicide.

10. Method of controlling unwanted plants, **characterized in that** an effective amount of at least one ketoxime benzoylamide of the formula (I) according to any of Claims 1 to 4 or of a herbicidal composition according to any of Claims 5 to 9 is applied to the plants or to the site of the unwanted vegetation.

11. Use of ketoxime benzoylamides of the formula (I) according to any of Claims 1 to 4 or of herbicidal compositions according to any of Claims 5 to 9 for controlling unwanted plants.

12. Use according to Claim 11, **characterized in that** the ketoxime benzoylamides of the formula (I) are used for controlling unwanted plants in crops of useful plants.

13. Use according to Claim 12, **characterized in that** the useful plants are transgenic useful plants.

## Revendications

1. Cétoxime-benzoylamides substitués de formule (I) 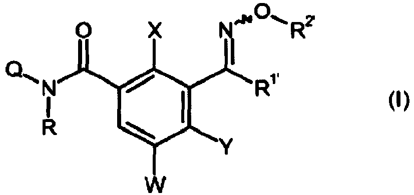 dans lesquels les symboles et les indices ont les significations suivantes :
Q signifie un radical Q1, Q2, Q3 ou Q4, 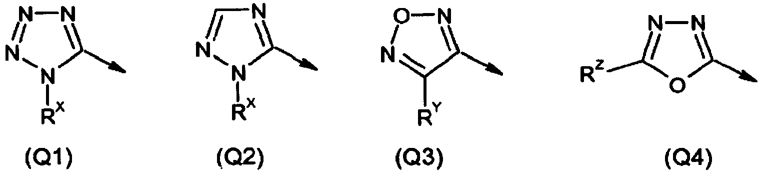
R signifie hydrogène, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), halogéno-cycloalkyle en (C₃-C₆) , cycloalkyle en (C₃-C₆) -alkyle en (C₁-C₆), halogéno-cycloalkyle en (C₃-C₆) -alkyle en (C₁-C₆), R¹(O)C-alkyle en (C₁-C₆) , R¹O(O)C-alkyle en (C₁-C₆), (R¹)₂N(O)C-alkyle en (C₁-C₆), NC-alkyle en (C₁-C₆), R¹O-alkyle en (C₁-C₆), R¹(O)CO-alkyle en (C₁-C₆), R¹O(O)C-O-alkyloxy en (C₁-C₆), R²(O)₂SO-alkyle en (C₁-C₆), (R¹)₂N-alkyle en (C₁-C₆), R¹(O)C(R¹)N-alkyle en (C₁-C₆), R²(O)₂S(R¹)N-alkyle en (C₁-C₆), R²(O)ₙS-alkyle en (C₁-C₆), R¹O(O)₂S-alkyle en (C₁-C₆), (R¹)₂N(O)₂S-alkyle en (C₁-C₆), R¹(O)C, R¹O(O)C, (R¹)₂N(O)C, R¹O, (R¹)₂N, R¹O(O)C(R¹)N, (R¹)₂N(O)C(R¹)N, R²(O)₂S ; ou benzyle substitué par s radicaux du groupe constitué par méthyle, éthyle, méthoxy, nitro, trifluorométhyle et halogène,
R¹ signifie hydrogène, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), cycloalcényle en (C₃-C₆), halogéno-cycloalkyle en (C₃-C₆), cycloalkyle en (C₃-C₆)-alkyle en (C₁-C₆), alkyle en (C₁-C₆)-O-alkyle en (C₁-C₆), cycloalkyl-alkyle en (C₁-C₆)-O-alkyle en (C₁-C₆), phényle, phényl-alkyle en (C₁-C₆), hétéroaryle, hétéroaryl-alkyle en (C₁-C₆), hétérocyclyle, hétérocyclyl-alkyle en (C₁-C₆), phényl-O-alkyle en (C₁-C₆), hétéroaryl-O-alkyle en (C₁-C₆), hétérocyclyl-O-alkyle en (C₁-C₆), phényl-N(R³) -alkyle en (C₁-C₆), hétéroaryl-N(R³) -alkyle en (C₁-C₆), hétérocyclyl-N(R³)-alkyle en (C₁-C₆), phényl-S(O)ₙ-alkyle en (C₁-C₆), hétéroaryl-S(O)ₙ-alkyle en (C₁-C₆) ou hétérocyclyl-S(O)ₙ-alkyle en (C₁-C₆), les quinze derniers radicaux cités étant chacun substitués par s radicaux du groupe constitué par nitro, halogène, cyano, rhodano, alkyle en (C₁-C₆), halogéno-alkyle en (C₁-C₆), cycloalkyle en (C₃-C₆) , R³O(O)C, (R³)₂N(O)C, R³O, (R³)₂N, R⁴(O)ₙS, R³O(O)₂S, (R³)₂N(O)₂S et R³O-alkyle en (C₁-C₆), et l'hétérocyclyle portant n groupes oxo,
R² signifie alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), cycloalcényle en (C₃-C₆), halogéno-cycloalkyle en (C₃-C₆), cycloalkyle en (C₃-C₆)-alkyle en (C₁-C₆), alkyle en (C₁-C₆)-O-alkyle en (C₁-C₆), cycloalkyl-alkyle en (C₁-C₆)-O-alkyle en (C₁-C₆), phényle, phényl-alkyle en (C₁-C₆), hétéroaryle, hétéroaryl-alkyle en (C₁-C₆), hétérocyclyle, hétérocyclyl-alkyle en (C₁-C₆), phényl-O-alkyle en (C₁-C₆), hétéroaryl-O-alkyle en (C₁-C₆), hétérocyclyl-O-alkyle en (C₁-C₆), phényl-N(R³) -alkyle en (C₁-C₆), hétéroaryl-N(R³)-alkyle en (C₁-C₆), hétérocyclyl-N(R³)-alkyle en (C₁-C₆), phényl-S(O)ₙ-alkyle en (C₁-C₆), hétéroaryl-S(O)ₙ-alkyle en (C₁-C₆)ou hétérocyclyl-S(O)ₙ-alkyle en (C₁-C₆), les quinze derniers radicaux cités étant chacun substitués par s radicaux du groupe constitué par nitro, halogène, cyano, rhodano, alkyle en (C₁-C₆), halogéno-alkyle en (C₁-C₆), cycloalkyle en (C₃-C₆) , R³O(O)C, (R³)₂N(O)C, R³O, (R³)₂N, R⁴(O)ₙS, R³O(O)₂S, (R³)₂N(O)₂S et R³O-alkyle en (C₁-C₆), et l'hétérocyclyle portant n groupes oxo,
R³ signifie hydrogène, alkyle en (C₁-C₆), halogéno-alkyle en (C₁-C₆), alcényle en (C₂-C₆), alcynyle en (C₂-C₆), cycloalkyle en (C₃-C₆), cycloalkyle en (C₃-C₆)-alkyle en (C₁-C₆)ou phényle,
R⁴ signifie alkyle en (C₁-C₆), halogéno-alkyle en (C₁-C₆), alcényle en (C₂-C₆), alcynyle en (C₂-C₆), cycloalkyle en (C₃-C₆), cycloalkyle en (C₃-C₆)-alkyle en (C₁-C₆)ou phényle,
R⁵ signifie hydrogène ou alkyle en (C₁-C₄),
R⁶ signifie alkyle en (C₁-C₄),
R⁷ signifie acétoxy, acétamido, N-méthylacétamido, benzoyloxy, benzamido, N-méthylbenzamido, méthoxycarbonyle, éthoxycarbonyle, benzoyle, méthylcarbonyle, pipéridinylcarbonyle, morpholinylcarbonyle, trifluorométhylcarbonyle, aminocarbonyle, méthylaminocarbonyle, diméthylaminocarbonyle, méthylsulfényle, méthylsulfinyle, méthylsulfonyle, cycloalkyle en (C₃-C₆) ; ou hétéroaryle ou hétérocyclyle, chacun substitués par s radicaux du groupe constitué par méthyle, éthyle, méthoxy, trifluorométhyle et halogène,
R⁸ signifie alkyle en (C₁-C₆), halogéno-alkyle en (C₁-C₆), alkyle en (C₁-C₆)-O-alkyle en (C₁-C₆), alcényle en (C₂-C₆)ou alcynyle en (C₃-C₆),
R⁹ signifie alkyle en (C₁-C₆), halogéno-alkyle en (C₁-C₆), alcényle en (C₂-C₆) ou alcynyle en (C₃-C₆),
R¹⁰ signifie hydrogène, alkyle en (C₁-C₆), halogéno-alkyle en (C₁-C₆), alcényle en (C₂-C₆), alcynyle en (C₂-C₆), cycloalkyle en (C₃-C₆) ou cycloalkyle en (C₃-C₆)-alkyle en (C₁-C₆),
R¹¹ signifie alkyle en (C₁-C₆), alcényle en (C₂-C₆) ou alcynyle en (C₂-C₆),
R^{1'} signifie cyano, alkyle en (C₁-C₆), cycloalkyle en (C₃-C₆), halogéno-alkyle en (C₁-C₆), OR⁸, SR⁸, NR⁸R⁹,
R^{2'} signifie hydrogène, alkyle en (C₁-C₆), alcényle en (C₂-C₆), alcynyle en (C₂-C₆), cycloalkyle en (C₃-C₆), cycloalcényle en (C₃-C₆), cycloalkyle en (C₃-C₆) -alkyle en (C₁-C₆), les six derniers radicaux cités étant substitués par s radicaux du groupe constitué par cyano, halogène, nitro, rhodano, OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ et alcoxy en (C₁-C₄)-alcoxycarbonyle en (C₂-C₆),
ou R^{2'} signifie phényle, phényl-alkyle en (C₁-C₆), hétéroaryle, alkyle en (C₁-C₆)-hétéroaryle, hétérocyclyle, alkyle en (C₁-C₆)-hétérocyclyle, alkyle en (C₁-C₆)-O-hétéroaryle, alkyle en (C₁-C₆)-O-hétérocyclyle, alkyle en (C₁-C₆)-NR¹⁰-hétéroaryle, alkyle en (C₁-C₆) -NR¹⁰-hétérocyclyle, les dix derniers radicaux cités étant chacun substitués par s radicaux du groupe constitué par cyano, halogène, nitro, rhodano, alkyle en (C₁-C₆), halogéno-alkyle en (C₁-C₆), OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ et alcoxy en (C₁-C₄)-alcoxycarbonyle en (C₂-C₆), et l'hétérocyclyle portant n groupes oxo,
R^{x} signifie alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), les dix radicaux cités précédemment étant chacun substitués par s radicaux du groupe constitué par nitro, cyano, (R⁶)₃Si, (R⁵O)₂(O)P, R²(O)ₙS, (R¹)₂N, R¹O, R¹(O)C, R¹O(O)C, R¹(O)CO, R²O(O)CO, R¹(O)C (R¹)N, R²(O)₂S(R¹)N, cycloalkyle en (C₃-C₆) , hétéroaryle, hétérocyclyle et phényle, les quatre derniers radicaux cités étant eux-mêmes substitués par s radicaux du groupe constitué par alkyle en (C₁-C₆), halogéno-alkyle en (C₁-C₆), alcoxy en (C₁-C₆), halogéno-alcoxy en (C₁-C₆) et halogène, et l'hétérocyclyle portant n groupes oxo, ou R^{x} signifie cycloalkyle en (C₃-C₇), hétéroaryle, hétérocyclyle ou phényle, les quatre radicaux cités précédemment étant chacun substitués par s radicaux du groupe constitué par halogène, nitro, cyano, alkyle en (C₁-C₆), halogéno-alkyle en (C₁-C₆), cycloalkyle en (C₃-C₆) , alkyle en (C₁-C₆)-S(O)ₙ, alcoxy en (C₁-C₆), halogéno-alcoxy en (C₁-C₆)et alcoxy en (C₁-C₆)-alkyle en (C₁-C₄),
R^{Y} signifie hydrogène, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₇), alcoxy en (C₁-C₆), halogéno-alcoxy en (C₁-C₆), alcényloxy en (C₂-C₆), alcynyloxy en (C₂-C₆), cyano, nitro, méthylsulfényle, méthylsulfinyle, méthylsulfonyle, acétylamino, benzoylamino, méthoxycarbonyle, éthoxycarbonyle, méthoxycarbonylméthyle, éthoxycarbonylméthyle, benzoyle, méthylcarbonyle, pipéridinylcarbonyle, trifluorométhylcarbonyle, halogène, amino, aminocarbonyle, méthylaminocarbonyle, diméthylaminocarbonyle, méthoxyméthyle ; ou hétéroaryle, hétérocyclyle ou phényle, chacun substitués par s radicaux du groupe constitué par alkyle en (C₁-C₆), halogéno-alkyle en (C₁-C₆), alcoxy en (C₁-C₆), halogéno-alcoxy en (C₁-C₆) et halogène ; et l'hétérocyclyle portant n groupes oxo, R^{z} signifie hydrogène, alkyle en (C₁-C₆), R¹O-alkyle en (C₁-C₆), R⁷CH₂, cycloalkyle en (C₃-C₇), halogéno-alkyle en (C₁-C₆), alcényle en (C₂-C₆), halogéno-alcényle en (C₂-C₆), alcynyle en (C₂-C₆), halogéno-alcynyle en (C₃-C₆), R¹O, R¹(H)N, méthoxycarbonyle, éthoxycarbonyle, méthylcarbonyle, diméthylamino, trifluorométhylcarbonyle, acétylamino, méthylsulfényle, méthylsulfinyle, méthylsulfonyle ; ou hétéroaryle, hétérocyclyle, benzyle ou phényle, chacun substitués par s radicaux du groupe constitué par halogène, nitro, cyano, alkyle en (C₁-C₆), halogéno-alkyle en (C₁-C₆), cycloalkyle en (C₃-C₆), alkyle en (C₁-C₆)-S(O)ₙ, alcoxy en (C₁-C₆), halogéno-alcoxy en (C₁-C₆)et alcoxy en (C₁-C₆)-alkyle en (C₁-C₄); l'hétérocyclyle portant n groupes oxo,
W signifie hydrogène, halogène, nitro, cyano, rhodano, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₇), halogéno-cycloalkyle en (C₃-C₇), alcoxy en (C₁-C₆), halogéno-alcoxy en (C₁-C₆), alkyle en (C₁-C₆)-(O)ₙS-, halogénoalkyle en (C₁-C₆)-(O)ₙS-, alcoxy en (C₁-C₆)-alkyle en (C₁-C₄), alcoxy en (C₁-C₆)-halogénoalkyle en (C₁-C₄), R¹(O)C, R¹(R¹ON=)C, R¹O(O)C, (R¹)₂N, R¹(O)C(R¹)N ou R²(O)₂S(R¹)N,
X signifie nitro, halogène, cyano, rhodano, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), halogéno-cycloalkyle en (C₃-C₆), cycloalkyle en (C₃-C₆) -alkyle en (C₁-C₆), halogéno-cycloalkyle en (C₃-C₆)-alkyle en (C₁-C₆), R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R¹O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R¹O(O)CO, (R¹)₂N(O)CO, (R¹)₂N, R¹(O)C(R¹)N, R²(O)₂S(R¹)N, R¹O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R¹O(O)C(R¹)N(O)₂S, (R¹)₂N(O)C(R¹)N(O)₂S, (R⁵O)₂(O)P, R¹(O)C-alkyle en (C₁-C₆), R¹O(O)C-alkyle en (C₁-C₆), (R¹)₂N(O)C-alkyle en (C₁-C₆), (R¹O)(R¹)N(O)C-alkyle en (C₁-C₆), (R¹)₂N(R¹)N(O)C-alkyle en (C₁-C₆), R¹(O)C(R¹)N(O)C-alkyle en (C₁-C₆), R¹O(O)C(R¹)N(O)C-alkyle en (C₁-C₆), (R¹)₂N(O)C(R¹)N(O)C-alkyle en (C₁-C₆), R²(O)₂S(R¹)N(O)C-alkyle en (C₁-C₆), R¹O(O)₂S(R¹)N(O)C-alkyle en (C₁-C₆), (R¹)₂N(O)₂S(R¹)N(O)C-alkyle en (C₁-C₆), NC-alkyle en (C₁-C₆), R¹O-alkyle en (C₁-C₆), R¹(O)CO-alkyle en (C₁-C₆), R²(O)₂SO-alkyle en (C₁-C₆), R¹O(O)CO-alkyle en (C₁-C₆), (R¹)₂N(O)CO-alkyle en (C₁-C₆), (R¹)₂N-alkyle en (C₁-C₆), R¹(O)C(R¹)N-alkyle en (C₁-C₆), R²(O)₂S(R¹)N-alkyle en (C₁-C₆), R¹O(O)C(R¹)N-alkyle en (C₁-C₆), (R¹)₂N(O)C(R¹)N-alkyle en (C₁-C₆), R¹O(O)₂S(R¹)N-alkyle en (C₁-C₆), (R¹)₂N(O)₂S(R¹)N-alkyle en (C₁-C₆), R²(O)ₙS-alkyle en (C₁-C₆), R¹O(O)₂S-alkyle en (C₁-C₆), (R¹)₂N(O)₂S-alkyle en (C₁-C₆), R¹(O)C(R¹)N(O)₂S-alkyle en (C₁-C₆), R¹O(O)C(R¹)N(O)₂S-alkyle en (C₁-C₆), (R¹)₂N(O)C(R¹)N(O)₂S-alkyle en (C₁-C₆), (R⁵O)₂(O) P-alkyle en (C₁-C₆), phényle, hétéroaryle, hétérocyclyle, phényl-alkyle en (C₁-C₆), hétéroaryl-alkyle en (C₁-C₆), hétérocyclyl-alkyle en (C₁-C₆), les six derniers radicaux cités étant chacun substitués par s radicaux du groupe constitué par nitro, halogène, cyano, rhodano, alkyle en (C₁-C₆), halogéno-alkyle en (C₁-C₆), cycloalkyle en (C₃-C₆), R¹O(O)C, (R¹)₂N(O)C, R¹O, (R¹)₂N, R²(O)ₙS, R¹O(O)₂S, (R¹)₂N(O)₂S et R¹O-alkyle en (C₁-C₆), l'hétérocyclyle portant n groupes oxo,
Y signifie hydrogène, nitro, halogène, cyano, rhodano, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆) -alkyle en (C₁-C₆), R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R¹O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R¹O(O)CO, (R¹)₂N(O)CO, (R¹)₂N, R¹(O)C(R¹)N, R²(O)₂S(R¹)N, R¹O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R¹O(O)C(R¹)N(O)₂S, (R¹)₂N(O)C(R¹)N(O)₂S, (R⁵O)₂(O)P, R¹(O)C-alkyle en (C₁-C₆), R¹O(O)C-alkyle en (C₁-C₆), (R¹)₂N(O)C-alkyle en (C₁-C₆), (R¹O)(R¹)N(O)C-alkyle en (C₁-C₆), (R¹)₂N(R¹)N(O)C-alkyle en (C₁-C₆), R¹(O)C(R¹)N(O)C-alkyle en (C₁-C₆), R¹O(O)C(R¹)N(O)C-alkyle en (C₁-C₆), (R¹)₂N(O)C(R¹)N(O)C-alkyle en (C₁-C₆), R²(O)₂S(R¹)N(O)C-alkyle en (C₁-C₆), R¹O(O)₂S(R¹)N(O)C-alkyle en (C₁-C₆), (R¹)₂N(O)₂S(R¹)N(O)C-alkyle en (C₁-C₆), NC-alkyle en (C₁-C₆), R¹O-alkyle en (C₁-C₆), R¹(O)CO-alkyle en (C₁-C₆), R²(O)₂SO-alkyle en (C₁-C₆), R¹O(O)CO-alkyle en (C₁-C₆), (R¹)₂N(O)CO-alkyle en (C₁-C₆), (R¹)₂N-alkyle en (C₁-C₆), R¹(O)C(R¹)N-alkyle en (C₁-C₆), R²(O)₂S(R¹)N-alkyle en (C₁-C₆), R¹O(O)C(R¹)N-alkyle en (C₁-C₆), (R¹)₂N(O)C(R¹) N-alkyle en (C₁-C₆), R¹O(O)₂S(R¹)N-alkyle en (C₁-C₆), (R¹)₂N(O)₂S(R¹)N-alkyle en (C₁-C₆), R²(O)ₙS-alkyle en (C₁-C₆), R¹O(O)₂S-alkyle en (C₁-C₆), (R¹)₂N(O)₂S-alkyle en (C₁-C₆), R¹(O)C(R¹)N(O)₂S-alkyle en (C₁-C₆), R¹O(O)C(R¹)N(O)₂S-alkyle en (C₁-C₆), (R¹)₂N(O)C(R¹)N(O)₂S-alkyle en (C₁-C₆), (R⁵O)₂(O)P-alkyle en (C₁-C₆), phényle, hétéroaryle, hétérocyclyle, phényl-alkyle en (C₁-C₆), hétéroaryl-alkyle en (C₁-C₆), hétérocyclyl-alkyle en (C₁-C₆), les six derniers radicaux cités étant chacun substitués par s radicaux du groupe constitué par nitro, halogène, cyano, rhodano, alkyle en (C₁-C₆), halogéno-alkyle en (C₁-C₆), cycloalkyle en (C₃-C₆), R¹O(O)C, (R¹)₂N(O)C, R¹O, (R¹)₂N, R²(O)ₙS, R¹O(O)₂S, (R¹)₂N(O)₂S et R¹O-alkyle en (C₁-C₆), et l'hétérocyclyle portant n groupes oxo,
n signifie 0, 1 ou 2,
s signifie 0, 1, 2 ou 3.

2. Cétoxime-benzoylamides substitués de formule (I) selon la revendication 1, dans lesquels les symboles et les indices ont les significations suivantes :
Q signifie un radical Q1, Q2, Q3 ou Q4, 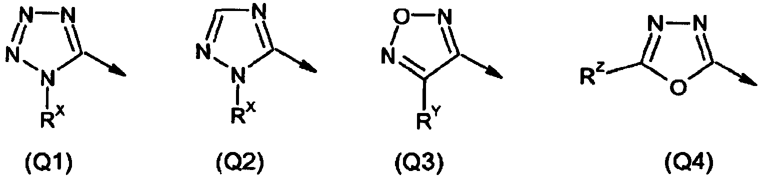
R signifie hydrogène, alkyle en (C₁-C₆), halogéno-alkyle en (C₁-C₆), R¹(O)C-alkyle en (C₁-C₆), R¹O(O)C-alkyle en (C₁-C₆), R¹O(O)C-O-alkyloxy en (C₁-C₆), R¹O-alkyle en (C₁-C₆), R¹(O)C, R¹O(O)C, R¹O, R²(O)₂S ; ou benzyle substitué par s radicaux du groupe constitué par méthyle, éthyle, méthoxy, nitro, trifluorométhyle et halogène,
R¹ signifie hydrogène, alkyle en (C₁-C₆), halogéno-alkyle en (C₁-C₆), alcényle en (C₂-C₆), alcynyle en (C₂-C₆), cycloalkyle en (C₃-C₆), cycloalcényle en (C₃-C₆), alkyle en (C₁-C₆)-O-alkyle en (C₁-C₆), phényle, hétéroaryle ou hétérocyclyle, les trois derniers radicaux cités étant chacun substitués par s radicaux du groupe constitué par nitro, halogène, cyano, rhodano, alkyle en (C₁-C₆), halogéno-alkyle en (C₁-C₆), cycloalkyle en (C₃-C₆), R³O(O)C, (R³)₂N(O)C, R³O, (R³)₂N, R⁴(O)ₙS, R³O(O)₂S, (R³)₂N(O)₂S et R³O-alkyle en (C₁-C₆), et l'hétérocyclyle portant n groupes oxo,
R² signifie alkyle en (C₁-C₆), halogéno-alkyle en (C₁-C₆), alcényle en (C₂-C₆), alcynyle en (C₂-C₆), cycloalkyle en (C₃-C₆), alkyle en (C₁-C₆)-O-alkyle en (C₁-C₆), phényle, hétéroaryle ou hétérocyclyle, les trois derniers radicaux cités étant chacun substitués par s radicaux du groupe constitué par nitro, halogène, cyano, rhodano, alkyle en (C₁-C₆), halogéno-alkyle en (C₁-C₆), cycloalkyle en (C₃-C₆), R³O(O)C, (R³)₂N(O)C, R³O, (R³)₂N, R⁴(O)ₙS, R³O(O)₂S, (R³)₂N(O)₂S et R³O-alkyle en (C₁-C₆), et l'hétérocyclyle portant n groupes oxo,
R³ signifie hydrogène, alkyle en (C₁-C₆), halogéno-alkyle en (C₁-C₆), cycloalkyle en (C₃-C₆) ou phényle,
R⁴ signifie alkyle en (C₁-C₆), halogéno-alkyle en (C₁-C₆), cycloalkyle en (C₃-C₆) ou phényle,
R⁵ signifie hydrogène ou alkyle en (C₁-C₄),
R⁶ signifie alkyle en (C₁-C₄),
R⁷ signifie acétoxy, benzoyloxy, méthoxycarbonyle, éthoxycarbonyle, méthylcarbonyle, trifluorométhylcarbonyle, méthylsulfényle, méthylsulfinyle, méthylsulfonyle, cycloalkyle en (C₃-C₆) ; ou hétéroaryle ou hétérocyclyle, chacun substitués par s radicaux du groupe constitué par méthyle, éthyle, méthoxy, trifluorométhyle et halogène,
R⁸ signifie alkyle en (C₁-C₆), halogéno-alkyle en (C₁-C₆), alkyle en (C₁-C₆)-O-alkyle en (C₁-C₆), alcényle en (C₂-C₆)ou alcynyle en (C₃-C₆),
R⁹ signifie alkyle en (C₁-C₆) ou halogéno-alkyle en (C₁-C₆),
R¹⁰ signifie hydrogène, alkyle en (C₁-C₆), halogéno-alkyle en (C₁-C₆) ou cycloalkyle en (C₃-C₆),
R¹¹ signifie alkyle en (C₁-C₆),
R¹' signifie alkyle en (C₁-C₆), cycloalkyle en (C₃-C₆), halogéno-alkyle en (C₁-C₆), OR⁸, SR⁸, NR⁸R⁹,
R²' signifie hydrogène, alkyle en (C₁-C₆), alcényle en (C₂-C₆), alcynyle en (C₂-C₆), cycloalkyle en (C₃-C₆), cycloalcényle en (C₃-C₆), cycloalkyle en (C₃-C₆) -alkyle en (C₁-C₆), les six derniers radicaux cités étant substitués par s radicaux du groupe constitué par cyano, halogène, OR¹⁰, S(O)ₙR¹¹, N(R¹⁰)₂, COR¹⁰, CO₂R¹⁰ et alcoxy en (C₁-C₄)-alcoxycarbonyle en (C₂-C₆),
ou R²' signifie phényle, hétéroaryle, hétérocyclyle, les trois derniers radicaux cités étant chacun substitués par s radicaux du groupe constitué par cyano, halogène, nitro, rhodano, alkyle en (C₁-C₆), halogéno-alkyle en (C₁-C₆), OR¹⁰, S(O)ₙR¹¹, N(R¹⁰)₂, NR¹⁰OR¹⁰, COR¹⁰, OCOR¹⁰, SCOR¹¹, NR¹⁰COR¹⁰, NR¹⁰SO₂R¹¹, CO₂R¹⁰, COSR¹¹, CON(R¹⁰)₂ et alcoxy en (C₁-C₄)-alcoxycarbonyle en (C₂-C₆), et l'hétérocyclyle portant n groupes oxo,
R^{X} signifie alkyle en (C₁-C₆), halogéno-alkyle en (C₁-C₆), alcényle en (C₂-C₆), halogéno-alcényle en (C₂-C₆), alcynyle en (C₂-C₆), les cinq radicaux cités précédemment étant chacun substitués par s radicaux du groupe constitué par cyano, (R⁶)₃Si, (R⁵O)₂(O)P, R²(O)ₙS, (R¹)₂N, R¹O, R¹(O)C, R¹O(O)C, R¹(O)CO, R²O(O)CO, cycloalkyle en (C₃-C₆), hétéroaryle, hétérocyclyle et phényle, les quatre derniers radicaux cités étant eux-mêmes substitués par s radicaux du groupe constitué par alkyle en (C₁-C₆), halogéno-alkyle en (C₁-C₆), alcoxy en (C₁-C₆), halogéno-alcoxy en (C₁-C₆) et halogène, et l'hétérocyclyle portant n groupes oxo,
ou R^{X} signifie cycloalkyle en (C₃-C₇), hétéroaryle, hétérocyclyle ou phényle, les quatre radicaux cités précédemment étant chacun substitués par s radicaux du groupe constitué par halogène, nitro, cyano, alkyle en (C₁-C₆), halogéno-alkyle en (C₁-C₆), cycloalkyle en (C₃-C₆), alkyle en (C₁-C₆)-S(O)ₙ, alcoxy en (C₁-C₆), halogéno-alcoxy en (C₁-C₆) et alcoxy en (C₁-C₆)-alkyle en (C₁-C₄),
R^{Y} signifie hydrogène, alkyle en (C₁-C₆), halogéno-alkyle en (C₁-C₆), alcényle en (C₂-C₆), alcynyle en (C₂-C₆), cycloalkyle en (C₃-C₇), alcoxy en (C₁-C₆), cyano, méthylsulfényle, méthylsulfinyle, méthylsulfonyle, acétylamino, méthoxycarbonyle, éthoxycarbonyle, méthoxycarbonylméthyle, éthoxycarbonylméthyle, benzoyle, méthylcarbonyle, trifluorométhylcarbonyle, halogène, amino, méthylaminocarbonyle, diméthylaminocarbonyle, méthoxyméthyle ; ou hétéroaryle, hétérocyclyle ou phényle, chacun substitués par s radicaux du groupe constitué par alkyle en (C₁-C₆), halogéno-alkyle en (C₁-C₆), alcoxy en (C₁-C₆), halogéno-alcoxy en (C₁-C₆) et halogène ; et l'hétérocyclyle portant n groupes oxo,
R^{Z} signifie hydrogène, alkyle en (C₁-C₆), R¹O-alkyle en (C₁-C₆), R⁷CH₂, cycloalkyle en (C₃-C₇), halogéno-alkyle en (C₁-C₆), alcényle en (C₂-C₆), halogéno-alcényle en (C₂-C₆), alcynyle en (C₂-C₆), R¹O, R¹(H)N, méthoxycarbonyle, éthoxycarbonyle, méthylcarbonyle, diméthylamino, trifluorométhylcarbonyle, acétylamino, méthylsulfényle, méthylsulfinyle, méthylsulfonyle ; ou hétéroaryle, hétérocyclyle, benzyle ou phényle, chacun substitués par s radicaux du groupe constitué par halogène, cyano, alkyle en (C₁-C₆), halogéno-alkyle en (C₁-C₆), cycloalkyle en (C₃-C₆), alkyle en (C₁-C₆)-S(O)ₙ, alcoxy en (C₁-C₆), halogéno-alcoxy en (C₁-C₆) et alcoxy en (C₁-C₆)-alkyle en (C₁-C₄) ; l'hétérocyclyle portant n groupes oxo,
W signifie hydrogène, halogène, alkyle en (C₁-C₆), halogéno-alkyle en (C₁-C₆), alcényle en (C₂-C₆), alcynyle en (C₂-C₆), cycloalkyle en (C₃-C₇), alcoxy en (C₁-C₆), halogéno-alcoxy en (C₁-C₆), alkyle en (C₁-C₆)-(O)ₙS-, alcoxy en (C₁-C₆)-alkyle en (C₁-C₄), alcoxy en (C₁-C₆)-halogénoalkyle en (C₁-C₄), R¹(O)C, (R¹)₂N, R¹(O)C(R¹)N ou R²(O)₂S(R¹)N,
X signifie nitro, halogène, cyano, alkyle en (C₁-C₆), halogéno-alkyle en (C₁-C₆), alcényle en (C₂-C₆), halogéno-alcényle en (C₂-C₆), alcynyle en (C₂-C₆), cycloalkyle en (C₃-C₆), halogéno-cycloalkyle en (C₃-C₆), cycloalkyle en (C₃-C₆) -alkyle en (C₁-C₆), halogéno-cycloalkyle en (C₃-C₆) -alkyle en (C₁-C₆), R¹(O)C, R¹(R¹ON=)C, R¹O(O)C, (R¹)₂N(O)C, R¹O, R¹(O)CO, R¹O(O)CO, (R¹)₂N, R¹(O)C(R¹)N, R²(O)₂S(R¹)N, R¹O(O)C(R¹)N, R²(O)ₙS, (R⁵O)₂(O)P, R¹(O)C-alkyle en (C₁-C₆), R¹O(O)C-alkyle en (C₁-C₆), (R¹)₂N(O)C-alkyle en (C₁-C₆), NC-alkyle en (C₁-C₆), R¹O-alkyle en (C₁-C₆), R¹(O)CO-alkyle en (C₁-C₆), R²(O)₂SO-alkyle en (C₁-C₆), R¹O(O)CO-alkyle en (C₁-C₆), (R¹)₂N(O)CO-alkyle en (C₁-C₆), (R¹)₂N-alkyle en (C₁-C₆), R¹(O)C(R¹)N-alkyle en (C₁-C₆), R²(O)₂S(R¹)N-alkyle en (C₁-C₆), R¹O(O)C(R¹)N-alkyle en (C₁-C₆), (R¹)₂N(O)C(R¹)N-alkyle en (C₁-C₆), R¹O(O)₂S(R¹)N-alkyle en (C₁-C₆), (R¹)₂N(O)₂S(R¹)N-alkyle en (C₁-C₆), R²(O)ₙS-alkyle en (C₁-C₆), (R⁵O)₂(O)P-alkyle en (C₁-C₆), phényle, hétéroaryle ou hétérocyclyle, les trois derniers radicaux cités étant chacun substitués par s radicaux du groupe constitué par nitro, halogène, cyano, rhodano, alkyle en (C₁-C₆), halogéno-alkyle en (C₁-C₆), cycloalkyle en (C₃-C₆), R¹O(O)C, (R¹)₂N(O)C, R¹O, (R¹)₂N, R²(O)ₙS, R¹O(O)₂S, (R¹)₂N(O)₂S et R¹O-alkyle en (C₁-C₆), et l'hétérocyclyle portant n groupes oxo,
Y signifie hydrogène, nitro, halogène, cyano, rhodano, alkyle en (C₁-C₆), halogéno-alkyle en (C₁-C₆), alcényle en (C₂-C₆), halogéno-alcényle en (C₂-C₆), alcynyle en (C₂-C₆), halogéno-alcynyle en (C₃-C₆), cycloalkyle en (C₃-C₆), halogéno-cycloalkyle en (C₃-C₆), cycloalkyle en (C₃-C₆)-alkyle en (C₁-C₆), halogéno-cycloalkyle en (C₃-C₆)-alkyle en (C₁-C₆), R¹(O)C, R¹(R¹ON=)C, R¹O(O)C, (R¹)₂N-(O)C, R¹O, R¹(O)CO, R²(O)₂SO, R¹O(O)CO, (R¹)₂N(O)CO, (R¹)₂N, R¹(O)C(R¹)N, R²(O)₂S(R¹)N, R¹O(O)C(R¹)N, R²(O)ₙS, (R⁵O)₂(O)P, R¹(O)C-alkyle en (C₁-C₆), R¹O(O)C-alkyle en (C₁-C₆), (R¹)₂N(O)C-alkyle en (C₁-C₆), NC-alkyle en (C₁-C₆), R¹O-alkyle en (C₁-C₆), R¹(O)CO-alkyle en (C₁-C₆), R²(O)₂SO-alkyle en (C₁-C₆), R¹O(O)CO-alkyle en (C₁-C₆), (R¹)₂N(O)CO-alkyle en (C₁-C₆), (R¹)₂N-alkyle en (C₁-C₆), R¹(O)C(R¹)N-alkyle en (C₁-C₆), R²(O)₂S(R¹)N-alkyle en (C₁-C₆), R¹O(O)C(R¹)N-alkyle en (C₁-C₆), (R¹)₂N(O)C(R¹)N-alkyle en (C₁-C₆), R¹O(O)₂S(R¹)N-alkyle en (C₁-C₆), (R¹)₂N(O)₂S(R¹)N-alkyle en (C₁-C₆), R²(O)ₙS-alkyle en (C₁-C₆), (R⁵O)₂(O)P-alkyle en (C₁-C₆), phényle, hétéroaryle, hétérocyclyle, les trois derniers radicaux cités étant chacun substitués par s radicaux du groupe constitué par nitro, halogène, cyano, rhodano, alkyle en (C₁-C₆), halogéno-alkyle en (C₁-C₆), cycloalkyle en (C₃-C₆), R¹O(O)C, (R¹)₂N(O)C, R¹O, (R¹)₂N, R²(O)ₙS, R¹O(O)₂S, (R¹)₂N(O)₂S et R¹O-alkyle en (C₁-C₆), et l'hétérocyclyle portant n groupes oxo,
n signifie 0, 1 ou 2,
s signifie 0, 1, 2 ou 3.

3. Cétoxime-benzoylamides substitués de formule (I) selon la revendication 1 ou 2, dans lesquels les symboles et les indices ont les significations suivantes :
Q signifie un radical Q1, Q2, Q3 ou Q4, 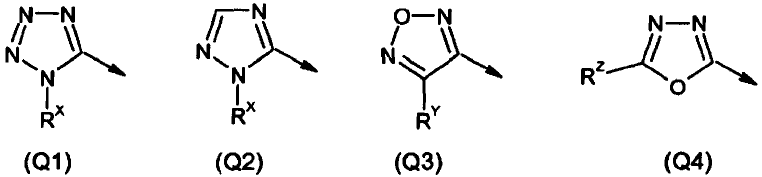
R signifie hydrogène, alkyle en (C₁-C₆), halogéno-alkyle en (C₁-C₆), R¹(O)C-alkyle en (C₁-C₆), R¹O(O)C-O-alkyloxy en (C₁-C₆), R¹(O)C, R¹O(O)C ou R²(O)₂S,
R¹ signifie hydrogène, alkyle en (C₁-C₆), halogéno-alkyle en (C₁-C₆), cycloalkyle en (C₃-C₆), alkyle en (C₁-C₆)-O-alkyle en (C₁-C₆), phényle, hétéroaryle ou hétérocyclyle, les trois derniers radicaux cités étant chacun substitués par s radicaux du groupe constitué par halogène, alkyle en (C₁-C₆), halogéno-alkyle en (C₁-C₆) et R³O(O)C, et l'hétérocyclyle portant n groupes oxo,
R² signifie alkyle en (C₁-C₆), halogéno-alkyle en (C₁-C₆), cycloalkyle en (C₃-C₆), alkyle en (C₁-C₆)-O-alkyle en (C₁-C₆), phényle, hétéroaryle ou hétérocyclyle, les trois derniers radicaux cités étant chacun substitués par s radicaux du groupe constitué par halogène, alkyle en (C₁-C₆), halogéno-alkyle en (C₁-C₆), R³O(O)C, R³O, R⁴(O)ₙS et R³O-alkyle en (C₁-C₆), et l'hétérocyclyle portant n groupes oxo,
R³ signifie hydrogène, alkyle en (C₁-C₆) ou halogéno-alkyle en (C₁-C₆),
R⁴ signifie alkyle en (C₁-C₆) ou halogéno-alkyle en (C₁-C₆),
R⁵ signifie hydrogène ou alkyle en (C₁-C₄),
R⁶ signifie alkyle en (C₁-C₄),
R⁷ signifie acétoxy, méthylsulfényle ou cycloalkyle en (C₃-C₆),
R⁸ signifie alkyle en (C₁-C₆) ou halogéno-alkyle en (C₁-C₆),
R⁹ signifie alkyle en (C₁-C₆),
R¹⁰ signifie hydrogène, alkyle en (C₁-C₆) ou halogéno-alkyle en (C₁-C₆),
R¹¹ signifie alkyle en (C₁-C₆),
R¹' signifie alkyle en (C₁-C₆), cycloalkyle en (C₃-C₆), halogéno-alkyle en (C₁-C₆), OR⁸, SR⁸, NR⁸R⁹,
R²' signifie hydrogène, alkyle en (C₁-C₆), cycloalkyle en (C₃-C₆), cycloalkyle en (C₃-C₆) -alkyle en (C₁-C₆), les trois derniers radicaux cités étant substitués par s radicaux du groupe constitué par cyano, halogène, OR¹⁰, S(O)ₙR¹¹, N(R¹⁰)₂, COR¹⁰, CO₂R¹⁰ et alcoxy en (C₁-C₄)-alcoxycarbonyle en (C₂-C₆),
ou R²' signifie phényle, hétéroaryle, hétérocyclyle, les trois derniers radicaux cités étant chacun substitués par s radicaux du groupe constitué par cyano, halogène, alkyle en (C₁-C₆), halogéno-alkyle en (C₁-C₆), OR¹⁰, S(O)ₙR¹¹, N(R¹⁰)₂, COR¹⁰ et CO₂R¹⁰, et l'hétérocyclyle portant n groupes oxo,
R^{X} signifie alkyle en (C₁-C₆), halogéno-alkyle en (C₁-C₆), alcényle en (C₂-C₆), alcynyle en (C₂-C₆), les quatre radicaux cités précédemment étant chacun substitués par s radicaux du groupe constitué par cyano, (R⁶)₃Si, (R⁵O)₂(O)P, R²(O)ₙS, (R¹)₂N, R¹O, R¹(O)C, R¹O(O)C, cycloalkyle en (C₃-C₆), hétéroaryle, hétérocyclyle et phényle, les quatre derniers radicaux cités étant eux-mêmes substitués par s radicaux du groupe constitué par alkyle en (C₁-C₆), halogéno-alkyle en (C₁-C₆), alcoxy en (C₁-C₆), halogéno-alcoxy en (C₁-C₆) et halogène, et l'hétérocyclyle portant n groupes oxo, ou R^{X} signifie cycloalkyle en (C₃-C₇), hétéroaryle, hétérocyclyle ou phényle, les quatre radicaux cités précédemment étant chacun substitués par s radicaux du groupe constitué par halogène, cyano, alkyle en (C₁-C₆), halogéno-alkyle en (C₁-C₆), cycloalkyle en (C₃-C₆), alkyle en (C₁-C₆)-S(O)ₙ, alcoxy en (C₁-C₆), halogéno-alcoxy en (C₁-C₆) et alcoxy en (C₁-C₆)-alkyle en (C₁-C₄),
R^{Y} signifie hydrogène, alkyle en (C₁-C₆), halogéno-alkyle en (C₁-C₆), alcényle en (C₂-C₆), alcynyle en (C₂-C₆), cycloalkyle en (C₃-C₇), alcoxy en (C₁-C₆), méthylsulfényle, méthylsulfinyle, méthylsulfonyle, acétylamino, méthoxycarbonyle, éthoxycarbonyle, méthoxycarbonylméthyle, éthoxycarbonylméthyle, benzoyle, méthylcarbonyle, trifluorométhylcarbonyle, halogène, méthoxyméthyle ; ou phényle substitué par s radicaux du groupe constitué par alkyle en (C₁-C₆), halogéno-alkyle en (C₁-C₆), alcoxy en (C₁-C₆), halogéno-alcoxy en (C₁-C₆) et halogène,
R^{Z} signifie hydrogène, alkyle en (C₁-C₆), R¹O-alkyle en (C₁-C₆), R⁷CH₂, cycloalkyle en (C₃-C₇), halogéno-alkyle en (C₁-C₆), alcényle en (C₂-C₆), halogéno-alcényle en (C₂-C₆), alcynyle en (C₂-C₆), R¹O, R¹(H)N, méthoxycarbonyle, éthoxycarbonyle, méthylcarbonyle, diméthylamino, trifluorométhylcarbonyle, acétylamino, méthylsulfényle, méthylsulfinyle, méthylsulfonyle ; ou phényle substitué par s radicaux du groupe constitué par halogène, cyano, alkyle en (C₁-C₆), halogéno-alkyle en (C₁-C₆), cycloalkyle en (C₃-C₆), alkyle en (C₁-C₆)-S(O)ₙ, alcoxy en (C₁-C₆), halogéno-alcoxy en (C₁-C₆),
W signifie hydrogène, halogène, alkyle en (C₁-C₆), halogéno-alkyle en (C₁-C₆), cycloalkyle en (C₃-C₇), alcoxy en (C₁-C₆), halogéno-alcoxy en (C₁-C₆), alkyle en (C₁-C₆)-S(O)ₙ ou alcoxy en (C₁-C₆)-alkyle en (C₁-C₄),
X signifie halogène, alkyle en (C₁-C₆), halogéno-alkyle en (C₁-C₆), cycloalkyle en (C₃-C₆), R¹(O)C, R¹O(O)C, (R¹)₂N(O)C, R¹O, (R¹)₂N, R²(O)ₙS, R¹(O)-alkyle en (C₁-C₆), (R¹)₂N-alkyle en (C₁-C₆), R²(O)ₙS-alkyle en (C₁-C₆), phényle, le dernier radical cité étant substitué par s radicaux du groupe constitué par halogène, alkyle en (C₁-C₆), halogéno-alkyle en (C₁-C₆), R¹O(O)C, R¹O, (R¹)₂N, R²(O)ₙS et R¹O-alkyle en (C₁-C₆),
Y signifie hydrogène, halogène, cyano, alkyle en (C₁-C₆), halogéno-alkyle en (C₁-C₆), alcényle en (C₂-C₆), alcynyle en (C₂-C₆), cycloalkyle en (C₃-C₆), halogéno-cycloalkyle en (C₃-C₆), cycloalkyle en (C₃-C₆)-alkyle en (C₁-C₆), R¹(O)C, R¹O(O)C, R¹O, (R¹)₂N, R²(O)ₙS, (R⁵O)₂(O)P, R¹(O)-alkyle en (C₁-C₆), (R¹)₂N-alkyle en (C₁-C₆), R²(O)ₙS-alkyle en (C₁-C₆), phényle, le dernier radical cité étant substitué par s radicaux du groupe constitué par halogène, alkyle en (C₁-C₆), halogéno-alkyle en (C₁-C₆), R¹O(O)C, R¹O, (R¹)₂N, R²(O)ₙS et R¹O-alkyle en (C₁-C₆),
n signifie 0, 1 ou 2,
s signifie 0, 1, 2 ou 3.

4. Cétoxime-benzoylamides substitués de formule (I) selon la revendication 1 ou 2, dans lesquels les symboles et les indices ont les significations suivantes :
Q signifie un radical Q1, Q2, Q3 ou Q4, 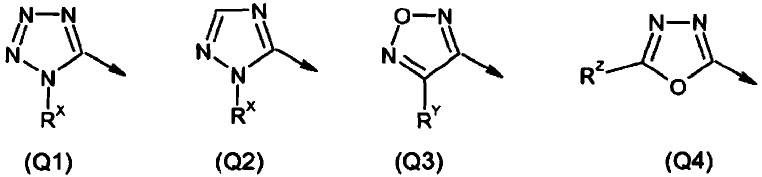
R signifie hydrogène,
R¹' signifie méthyle, éthyle, trifluorométhyle ou cyclopropyle,
R²' signifie méthyle, éthyle, 2,2,2-trifluoroéthyle, cyclo-propylméthyle ou trifluorométhyle,
R^{X} signifie méthyle, éthyle, propyle, méthoxyéthyle, 2-méthyoxy-2-méthyl-1-propyle ou phényle,
R^{Y} signifie chlore, méthyle ou éthyle,
R^{Z} signifie chlore, méthyle ou éthyle
X signifie fluor, chlore, brome, méthyle, méthoxy, méthylsulfonyle, méthoxyméthyle, méthylsulfényle, trifluorométhyle ou cyclopropyle,
Y signifie chlore, méthyle, éthyle, cyclopropyle, allyle, vinyle, trifluorométhyle, difluorométhyle, chlorodifluorométhyle, pentafluoroéthyle, méthoxy, méthylsulfényle, méthylsulfinyle, méthylsulfonyle ou éthylsulfonyle,
W signifie hydrogène, fluor, chlore, méthyle ou trifluorométhyle.

5. Agent herbicide, **caractérisé par** une teneur efficace du point de vue herbicide en au moins un cétoxime-benzoylamide de formule (I) selon l'une quelconque des revendication 1 à 4.

6. Agent herbicide selon la revendication 5, en mélange avec des adjuvants de formulation.

7. Agent herbicide selon la revendication 5 ou 6, contenant au moins une autre substance à activité pesticide du groupe constitué par les insecticides, les acaricides, les herbicides, les fongicides, les agents protecteurs et les régulateurs de croissance.

8. Agent herbicide selon la revendication 7, contenant un agent protecteur.

9. Agent herbicide selon l'une quelconque des revendications 7 à 8, contenant un herbicide supplémentaire.

10. Procédé de lutte contre des plantes indésirables, **caractérisé en ce qu'**une quantité efficace d'au moins un cétoxime-benzoylamide de formule (I) selon l'une quelconque des revendications 1 à 4 ou d'un agent herbicide selon l'une quelconque des revendications 5 à 9 est appliquée sur les plantes ou à l'emplacement de la végétation indésirable.

11. Utilisation de cétoxime-benzoylamides de formule (I) selon l'une quelconque des revendications 1 à 4 ou d'agents herbicides selon l'une quelconque des revendications 5 à 9 pour lutter contre des plantes indésirables.

12. Utilisation selon la revendication 11, **caractérisée en ce que** les cétoxime-benzoylamides de formule (I) sont utilisés pour lutter contre des plantes indésirables dans des cultures de plantes utiles.

13. Utilisation selon la revendication 12, **caractérisée en ce que** les plantes utiles sont des plantes utiles transgéniques.
